# EUROPEAN PATENT APPLICATION

(11) **EP 4 582 440 A2**
(43) Date of publication of application: **09.07.2025**
(21) Application number: 25163562.9
(22) Date of filing: 20.12.2019
(51) Int. Cl.: C07K 14/54

(54) **IL-10-CONTAINING VACCINES AND USES THEREOF**

(30) Priority: 21.12.2018 US 201862784039 P
(62) Divisional of application: 19899716.5
(71) Applicant: The Regents of the University of California, Oakland, CA 94607-5200 (US)
(72) Inventor: HARTIGAN-O'CONNOR, Dennis, California, 94607-5200 (US)
(74) Representative: J A Kemp LLP

(57) **Abstract**

Provided herein are systems, compositions, and methods for expressing an antigen and a protein having interleukin-10-like activity in a cell. The systems, compositions, and methods described herein can be used to induce an immune response against an antigen and are useful, for example, in the prevention and treatment of a number of infectious diseases and cancers.

## Description

### CROSS-REFERENCE TO RELATED APPLICATIONS

This application claims priority to U.S. Provisional Application No. 62/784,039, filed December 21, 2018, the contents of which are herein incorporated by reference in their entirety for all purposes.

### STATEMENT AS TO RIGHTS TO INVENTIONS MADE UNDER FEDERALLY SPONSORED RESEARCH AND DEVELOPMENT

This invention was made with Government support under Grant No. AI118451, awarded by the National Institutes of Health. The Government has certain rights in this invention.

### BACKGROUND OF THE INVENTION

Many vaccine candidates induce robust immune responses, but these immune responses are not preventative or therapeutic for the targeted disease because they are qualitatively different from therapeutic responses. Furthermore, some desired immune responses are not reliably induced by current vaccines. As one example, most known vaccines do not provoke responses among T cells that are restricted by MHC class Ib molecules (*e.g.,* HLA-E in humans, Mamu-E in macaques, or Qa-1 in mice). Current vaccines can sometimes elicit such responses, but are not able to do so at high levels in all recipients. In addition, tolerance responses of all kinds, as opposed to inflammatory responses, are not reliably elicited by known vaccines. Such responses are revealed by non-responsiveness or low responsiveness of some arm of the immune system after vaccination. This non-responsiveness or low responsiveness can be therapeutic for certain infectious diseases, cancers, or immune conditions. Accordingly, there is a need for improved vaccines that can more reliably provoke desired types of immune responses. The present invention satisfies this need, and provides related advantages as well.

### BRIEF SUMMARY OF THE INVENTION

In one aspect, provided herein is a system for expressing an antigen and a protein having interleukin-10 (IL-10)-like activity in a cell. In some embodiments, the system comprises a recombinant polynucleotide comprising a nucleic acid sequence encoding the antigen and a recombinant polynucleotide comprising a nucleic acid sequence encoding the protein having IL-10-like activity. In some embodiments, the antigen and the protein having IL-10-like activity are expressed in the same cell. In some embodiments, the cell is selected from the group consisting of an antigen-presenting cell, a muscle cell, a tumor cell, a fibroblast, an epithelial cell, an endothelial cell, and a combination thereof. In some embodiments, the antigen-presenting cell is a dendritic cell.

In some embodiments, the recombinant polynucleotide comprising the nucleic acid sequence encoding the antigen and/or the recombinant polynucleotide comprising the nucleic acid sequence encoding the protein having IL-10-like activity further comprise one or more nucleic acid sequences that encode a regulatory sequence. In some embodiments, the regulatory sequence controls expression of the antigen and/or the protein having IL-10-like activity.

In some embodiments, the regulatory sequence is selected from the group consisting of a promoter, an intronic sequence, an internal ribosome entry sequence (IRES), a polyadenylation signal, a Kozak consensus sequence, and a combination thereof. In some embodiments, the promoter is an EF1α promoter. In some instances, the EF1α promoter contains an intronic sequence (*e.g.,* an EF1α intron A sequence). In some embodiments, the polyadenylation signal is an SV40 virus polyadenylation signal. In some embodiments, the IRES is selected from the group consisting of an encephalomyocarditis (EMCV) IRES, a human VCIP IRES, a mouse Gtx IRES, a cricket paralysis virus intergenic region, a hepatitis C virus IRES, a poliovirus IRES, a human rhinovirus IRES, a dicistrovirus intergenic IRES, a hepatitis A virus IRES, an enterovirus IRES, an Aichivirus IRES, a cardiovirus IRES, an aphthorivus IRES, a porcine teschovirus IRES, a sapelovirus IRES, a simian virus IRES, an avian encephalomyelitis virus IRES, a foot and mouth disease virus IRES, a cold stress-induced mRNA (Rbm3) IRES, a human NF-kB repressing factor (NRF) IRES, a human apoptotic protease-activating factor-1 (Apaf-1) IRES, an immunoglobulin heavy chain binding protein (BIP) IRES, an aquaporin 4 (AQP-4) IRES, a c-myc33 IRES, and a rat cationic amino-acid transporter-1 (CAT-1) IRES.

In some embodiments, the recombinant polynucleotide comprising the nucleic acid sequence encoding the antigen and the recombinant polynucleotide comprising the nucleic acid sequence encoding the protein having IL-10-like activity are separate recombinant polynucleotides. In some embodiments, the recombinant polynucleotide comprising the nucleic acid sequence encoding the antigen and the recombinant polynucleotide comprising the nucleic acid sequence encoding the protein having IL-10-like activity are the same recombinant polynucleotide.

In some embodiments, the recombinant polynucleotide comprises: (a) the nucleic acid sequence encoding the antigen; (b) a nucleic acid sequence encoding an IRES; and (c) the nucleic acid sequence encoding the protein having IL-10-like activity. In some embodiments, the nucleic acid sequence encoding the antigen is located 5' of the nucleic acid sequence encoding the IRES and the nucleic acid sequence encoding the protein having IL-10-like activity is located 3' of the nucleic acid sequence encoding the IRES. In some embodiments, the recombinant polynucleotide further comprises: (d) a nucleic acid sequence encoding an EF1α promoter that is located 5' of the nucleic acid sequence encoding the antigen; (e) a nucleic acid sequence encoding an SV40 virus polyadenylation signal that is located 3' of the nucleic acid sequence encoding the protein having IL-10-like activity, and (f) a nucleic acid sequence encoding a Kozak consensus sequence that is located 3' of the nucleic acid sequence encoding the EF1α promoter and 5' of the nucleic acid sequence encoding the antigen.

In some embodiments, the antigen and the protein having IL-10-like activity are expressed under the control of two different promoters. In some embodiments, the antigen and the protein having IL-10-like activity are expressed as a single protein. In some embodiments, the single protein is a self-cleaving protein, and self-cleavage of the single protein yields the antigen and the protein having IL-10-like activity as separate proteins.

In some embodiments, the nucleic acid sequence encoding the antigen is codon optimized for expression in a host. In some embodiments, the antigen is an infectious disease antigen. In some embodiments, the infectious disease antigen is a bacterial, viral, fungal, protozoal, and/or helminthic infectious disease antigen. In some embodiments, the infectious disease antigen is a viral infectious disease antigen from simian immunodeficiency virus (SIV), human immunodeficiency virus (HIV), hepatitis C virus, herpes simplex virus 1, herpes simplex virus 2, varicella-zoster virus, human cytomegalovirus, human herpesvirus 6A, human herpesvirus 6B, human herpesvirus 7, human herpesvirus 8, Epstein-Barr virus, a hemorrhagic fever virus, or a combination thereof. In some embodiments, the infectious disease antigen comprises an HIV or SIV group-specific antigen (gag) protein. In some instances, the system comprises a recombinant polynucleotide comprising the nucleic acid sequence set forth in SEQ ID NO: 1. In some embodiments, the infectious disease antigen is a bacterial infectious disease antigen from *Mycobacterium tuberculosis.*

In some embodiments, the antigen is a tumor-associated antigen. In some embodiments, the tumor-associated antigen is selected from the group consisting of prostate-specific antigen, melanoma-associated antigen 4 (MAGEA4), melanoma-associated antigen 10 (MAGEA10), NY-ESO-1, and a combination thereof. In some instances, the system comprises a recombinant polynucleotide comprising the nucleic acid sequence set forth in SEQ ID NO:8 or 9. In some embodiments, the tumor-associated antigen is an antigen that newly arises in a subject's tumor.

In some embodiments, the protein having IL-10-like activity is a heterologous protein having IL-10-like activity and/or a viral protein having IL-10-like activity. In some embodiments, the heterologous protein having IL-10-like activity is an IL-10 protein from a host. In some embodiments, the IL-10 protein from the host is a human IL-10 protein or a rhesus macaque IL-10 protein. In some embodiments, the viral protein having IL-10-like activity is a cytomegalovirus (CMV) protein having IL-10-like activity. In some embodiments, the CMV protein having IL-10-like activity is human CMV IL-10 (HCMVIL-10) or rhesus macaque CMV IL-10 (RhCMVIL-10). In some embodiments, the heterologous protein having IL-10-like activity is STAT3 or a constitutively active form of STAT3. In some embodiments, the constitutively active form of STAT3 is STAT3C. In some embodiments, the STAT3 or constitutively active form of STAT3 (*e.g.*, STAT3C) is a human STAT3 or constitutively active form of STAT3 (*e.g.*, STAT3C), or a rhesus macaque STAT3 or constitutively active form of STAT3 (*e.g.*, STAT3C).

In some embodiments, the nucleic acid sequence encoding the antigen and/or the nucleic acid sequence encoding the protein having IL-10-like activity do not contain intronic sequences. In some embodiments, the nucleic acid sequence encoding the antigen and/or the nucleic acid sequence encoding the protein having IL-10-like activity contain intronic sequences. In some embodiments, the recombinant polynucleotide comprising the nucleic acid sequence encoding the antigen and/or the recombinant polynucleotide comprising the nucleic acid sequence encoding the protein having IL-10-like activity further comprise a nucleic acid sequence encoding a tag. In some embodiments, the tag is a FLAG tag. In some embodiments, the nucleic acid sequences encoding the antigen, the protein having IL-10-like activity, the one or more regulatory sequences, and/or the tag are located within the same open reading frame.

In some embodiments, the recombinant polynucleotide comprising the nucleic acid sequence encoding the antigen and/or the recombinant polynucleotide comprising the nucleic acid sequence encoding the protein having IL-10-like activity are present within a viral vector. In some embodiments, the viral vector is selected from the group consisting of a CMV vector, an adenoviral vector, an adeno-associated virus vector, a lentiviral vector, a herpes virus vector, an alphavirus vector, a retroviral vector, a poxvirus vector, and a vesicular stomatitis virus vector. In some embodiments, the viral vector is selected from the group consisting of an adenoviral vector, an adeno-associated virus vector, a lentiviral vector, an alphavirus vector, a retroviral vector, and a vesicular stomatitis virus vector.

In some embodiments, the viral vector contains a mutation that increases tropism for the cell. In some embodiments, the viral vector is a CMV vector and the mutation that increases tropism comprises a mutation that modifies a protein, or portion thereof, that may be positioned on the outside of the CMV virion.

In some embodiments, the mutation that increases tropism comprises an insertion of a nucleotide sequence encoding a cellular targeting ligand. In some embodiments, the cellular targeting ligand is selected from the group consisting of an antibody fragment that recognizes a target cell antigen, a ligand that is recognized by a target cell cognate receptor, a viral capsid protein that recognizes a target cell, and a combination thereof. In some embodiments, the cellular targeting ligand is CD154.

In some embodiments, the viral vector is a CMV vector, the CMV is a CMV capable of infecting rhesus macaque cells, and the mutation that increases tropism comprises a mutation within a gene selected from the group consisting of Rh13.1, Rh61/Rh60, Rh157.4, Rh157.5, Rh157.6, and a combination thereof. In some embodiments, the viral vector is a CMV vector, the CMV is a CMV capable of infecting human cells, and the mutation that increases tropism comprises a mutation within a gene selected from the group consisting of RL13, UL36, UL130, UL128, UL131, and a combination thereof.

In some embodiments, the protein having IL-10-like activity is one to which a host does not have pre-existing immunity. In some embodiments, the host is a human or a non-human primate. In some embodiments, the recombinant polynucleotide comprising the nucleic acid sequence encoding the antigen and/or the recombinant polynucleotide comprising the nucleic acid sequence encoding the protein having IL-10-like activity further comprise a nucleic acid sequence encoding a selectable marker. In some embodiments, the nucleic acid sequence encoding the selectable marker comprises a nucleic acid sequence encoding an antibiotic resistance gene and/or a fluorescent protein.

In another aspect, provided herein is a viral particle comprising a system (*i.e.,* for expressing an antigen and a protein having interleukin-10 (IL-10)-like activity in a cell) of the present invention. In yet another aspect, provided herein is an engineered cell comprising a system or a viral particle of the present invention.

In another aspect, provided herein is a pharmaceutical composition. In some embodiments, the pharmaceutical composition comprises (a) a system (*i.e.,* for expressing an antigen and a protein having interleukin-10 (IL-10)-like activity in a cell) of the present invention, a viral particle of the present invention, or an engineered cell of the present invention; and (b) a pharmaceutically acceptable carrier.

In another aspect, provided herein is a method for inducing an immune response against an antigen in a subject. In some embodiments, the method comprises administering to the subject a therapeutically effective amount of a pharmaceutical composition of the present invention. In some embodiments, the antigen is an infectious disease antigen or a tumor-associated antigen.

In some embodiments, the infectious disease antigen is a bacterial, viral, fungal, protozoal, and/or helminthic infectious disease antigen. In some embodiments, the infectious disease antigen is a viral infectious disease antigen from simian immunodeficiency virus (SIV), human immunodeficiency virus (HIV), hepatitis C virus, herpes simplex virus 1, herpes simplex virus 2, varicella-zoster virus, human cytomegalovirus, human herpesvirus 6A, human herpesvirus 6B, human herpesvirus 7, human herpesvirus 8, Epstein-Barr virus, a hemorrhagic fever virus, or a combination thereof. In some embodiments, the infectious disease antigen comprises an HIV or SIV group-specific antigen (gag) protein. In some embodiments, the infectious disease antigen is a bacterial infectious disease antigen from *Mycobacterium tuberculosis.*

In some embodiments, the tumor-associated antigen is selected from the group consisting of prostate-specific antigen, melanoma-associated antigen 4 (MAGEA4), melanoma-associated antigen 10 (MAGEA10), NY-ESO-1, and a combination thereof. In some embodiments, the tumor-associated antigen is an antigen that newly arises in the subject's tumor.

In some embodiments, inducing the immune response comprises generating antibodies that recognize the antigen. In some embodiments, the immune response that is induced in the subject comprises a lower CD8⁺ T cell response as compared to the CD8⁺ T cell response that is induced using a pharmaceutical composition that does not express the antigen and the protein having IL-10-like activity in the cell. In some embodiments, the immune response that is induced in the subject comprises a higher CD4⁺ T cell response as compared to the CD4⁺ T cell response that is induced using a pharmaceutical composition that does not express the antigen and the protein having IL-10-like activity in the cell. In some embodiments, the expression or activity of interleukin-8 (IL-8) is increased in the subject. In some embodiments, inducing the immune response comprises increasing the number or activation of MHC class Ib-restricted T cells in the subject.

In some embodiments, a sample is obtained from the subject. In some embodiments, the sample is selected from the group consisting of a blood sample, a tissue sample, a urine sample, a saliva sample, a cerebrospinal fluid (CSF) sample, and a combination thereof. In some embodiments, the level of one or more biomarkers is determined in the sample. In some embodiments, the one or more biomarkers is selected from the group consisting of IL-8, IL-10, IL-12, interferon-gamma, tumor necrosis factor-alpha, and a combination thereof.

In some embodiments, the level of the one or more biomarkers is compared to a reference sample. In some embodiments, the reference sample is obtained from the subject. In some embodiments, the reference sample is obtained from a different subject or a population of subjects.

In another aspect, provided herein is a method for preventing or treating a disease in a subject. In some embodiments, the method comprises administering to the subject a therapeutically effective amount of a pharmaceutical composition of the present invention. In some embodiments, the disease is an infectious disease or cancer.

In some embodiments, the infectious disease is a bacterial, viral, fungal, protozoal, and/or helminthic infectious disease. In some embodiments, the viral infectious disease is caused by a simian immunodeficiency virus (SIV), a human immunodeficiency virus (HIV), a hepatitis C virus, a herpes simplex virus 1, a herpes simplex virus 2, a varicella-zoster virus, a human cytomegalovirus, a human herpesvirus 6A, a human herpesvirus 6B, a human herpesvirus 7, a human herpesvirus 8, an Epstein-Barr virus, or a hemorrhagic fever virus. In some embodiments, the cancer is melanoma, ovarian cancer, lymphoma, leukemia, or prostate cancer.

In some embodiments, treating the subject comprises decreasing or eliminating one or more signs or symptoms of the disease. In some embodiments, the method further comprises administering an exogenous protein having IL-10-like activity to the subject.

Other objects, features, and advantages of the present invention will be apparent to one of skill in the art from the following detailed description and figures.

### BRIEF DESCRIPTION OF THE DRAWINGS

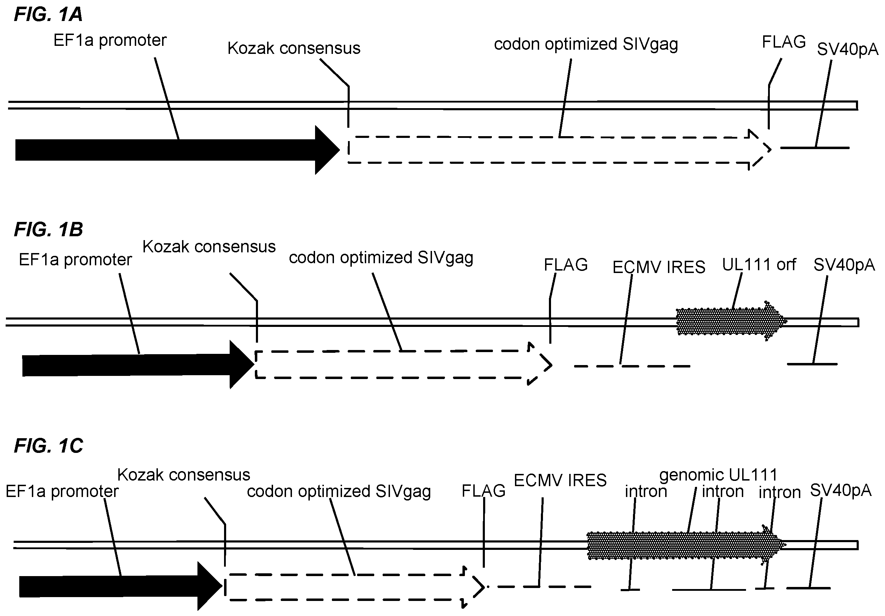

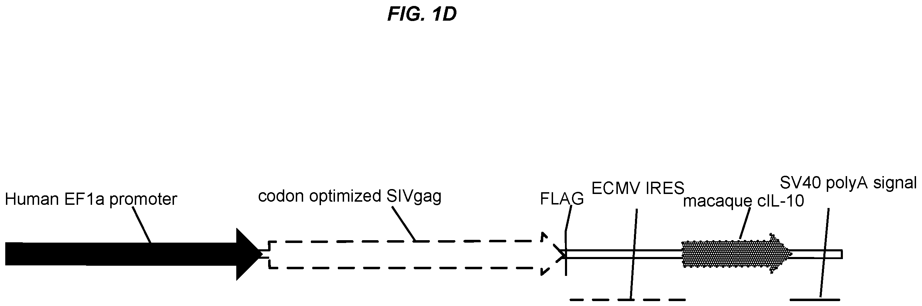

**FIGS. 1A-1D** show schematics of expression cassettes. **FIG. 1A** shows a control expression cassette. **FIG. 1B** shows an expression cassette containing a nucleic acid sequence encoding RhCMV UL111A in which the introns have been removed ("UL111 orf"). **FIG. 1C** shows an expression cassette containing a nucleic acid sequence encoding genomic RhCMV UL111A (*i.e.,* containing introns). **FIG. 1D** shows an expression cassette containing a nucleic acid encoding rhesus macaque IL-10.

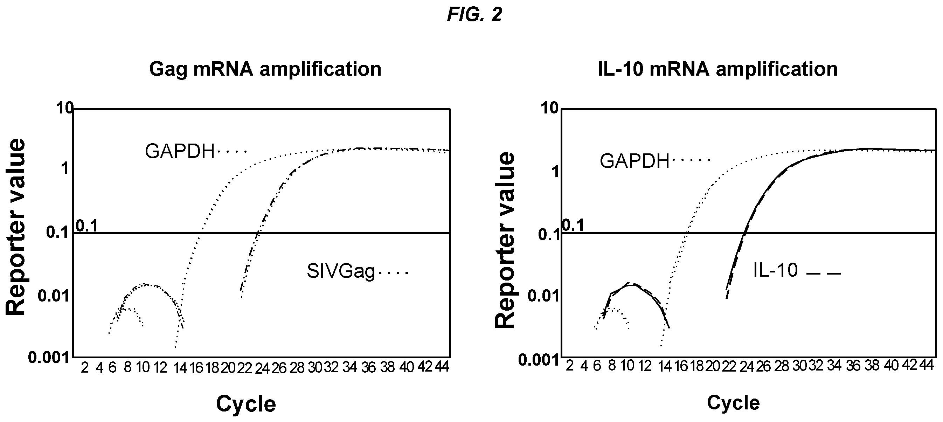

**FIG. 2** shows that AdGag-IRES-cIL-10 expresses both SIV Gag and rhesus macaque IL-10 mRNAs. Shown are real-time PCR amplification plots for amplicons within the SIV Gag (left) or IL-10 (right) genes. The plots demonstrate that the bicistronic message included in AdGag-IRES-cIL-10 contained coding regions for both proteins.

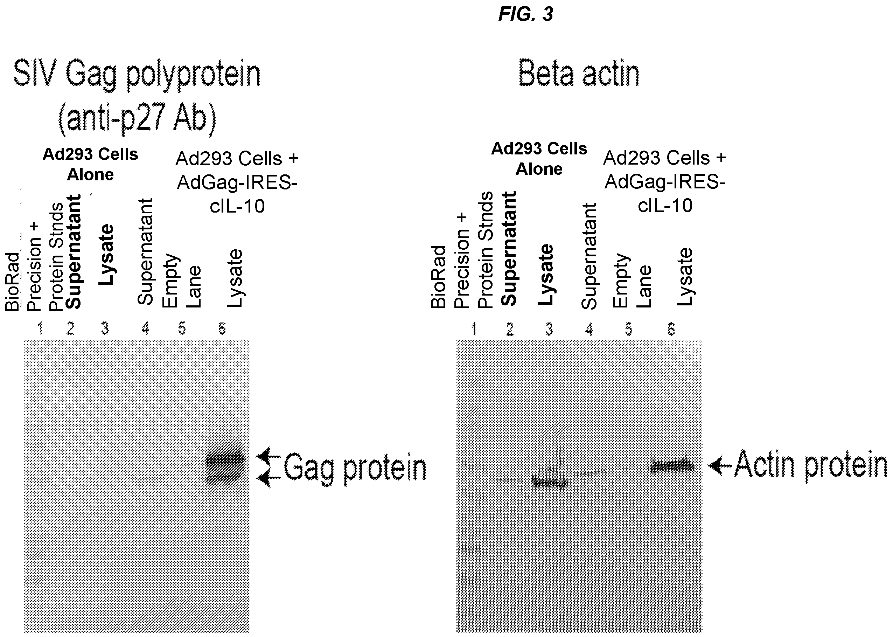

**FIG. 3** shows that AdGag-IRES-cIL-10 expressed SIV Gag protein. The left panel is a Western blot probed with an anti-SIV Gag antibody, demonstrating that only Ad Gag-IRES-cIL-10-transduced cells (or supernatants from such cells) contained SIV Gag (lanes 4 and 6). The right panel is a loading control, demonstrating that all experimental lanes contained sufficient cellular protein, as indicated by staining for beta-actin, for SIV Gag to be detected if present.

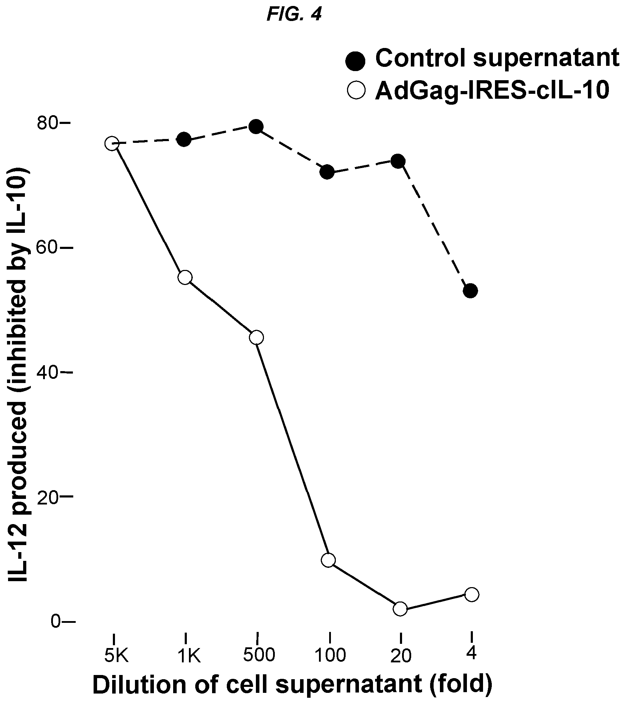

**FIG. 4** shows that AdGag-IRES-cIL-10 expressed functional cellular IL-10 (cIL-10) protein. This graph shows the amount of IL-12 produced by peripheral blood mononuclear cells in response to LPS challenge. IL-12 production under these conditions is inhibited by functional IL-10 protein. The upper line shows that IL-12 production was negligibly inhibited by a control supernatant from uninfected cells, which did not make IL-10. Transduction with AdGag-IRES-cIL-10, however, drove IL-10 production and secretion by the cells, which was detected by capacity of the supernatant to inhibit IL-12 production (lower line).

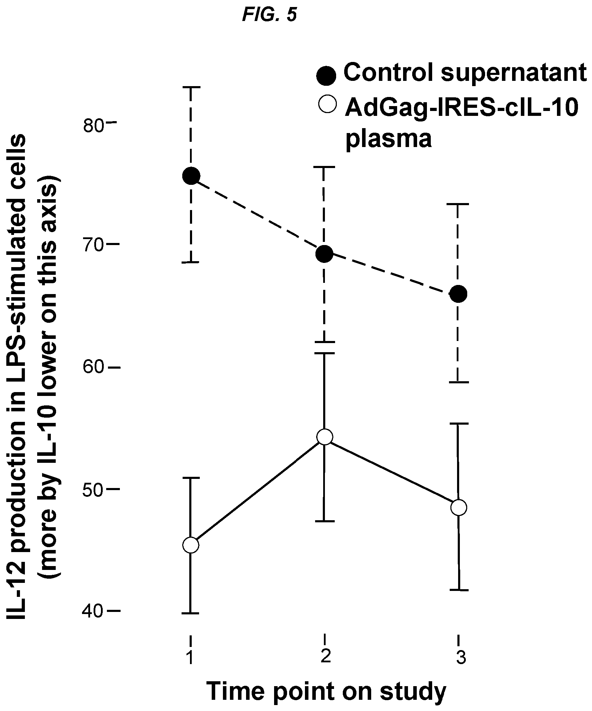

**FIG. 5** shows that AdGag-IRES-cIL-10 expressed functional cellular IL-10 (cIL-10) protein *in vivo.* This graph shows the amount of IL-12 produced by peripheral blood mononuclear cells in response to LPS challenge, *i.e.,* in the presence of plasma from control animals (upper line) or AdGag-IRES-cIL-10-injected animals (lower line). The presence of functional IL-10 protein was revealed by less production of IL-12, *i.e.,* plasma samples having more IL-10 activity were plotted lower on the y-axis. The data shows that animals receiving AdGag-IRES-cIL-10 had more circulating IL-10 functional activity than control animals.

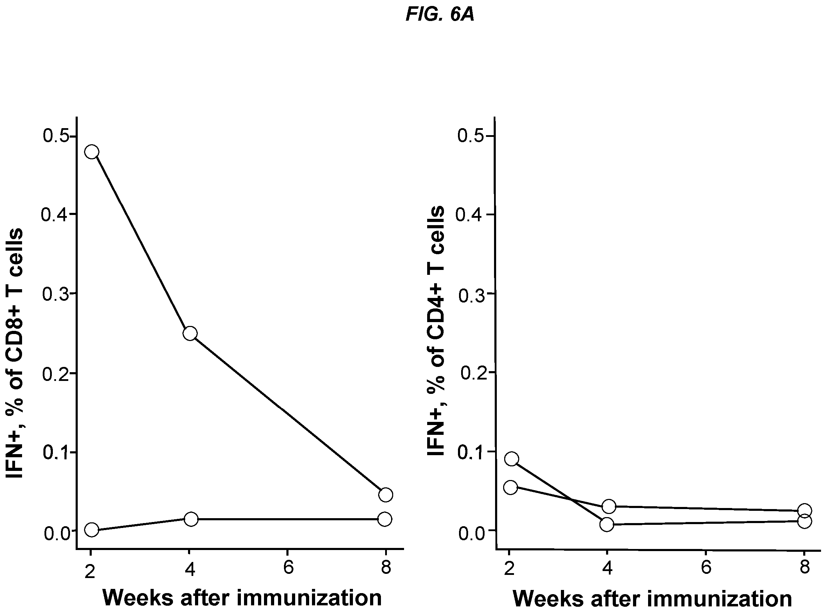

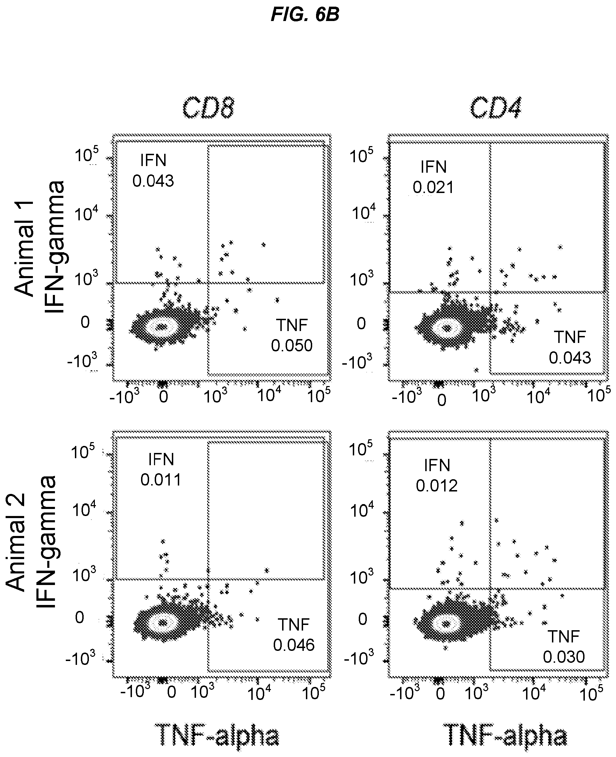

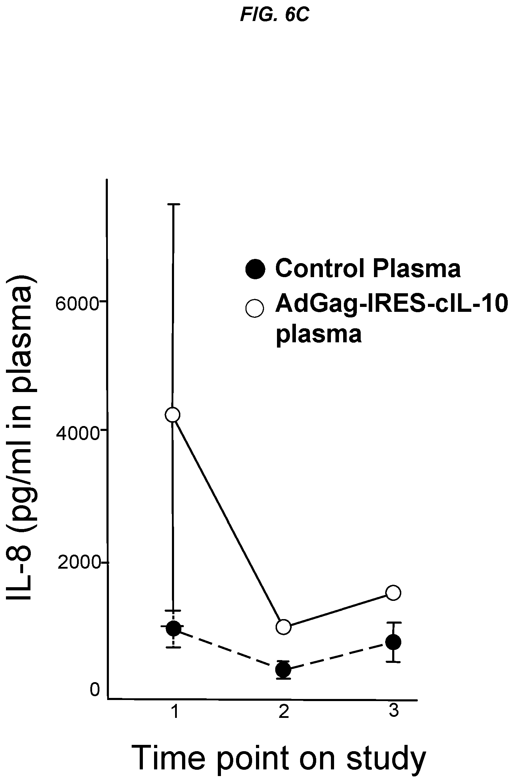

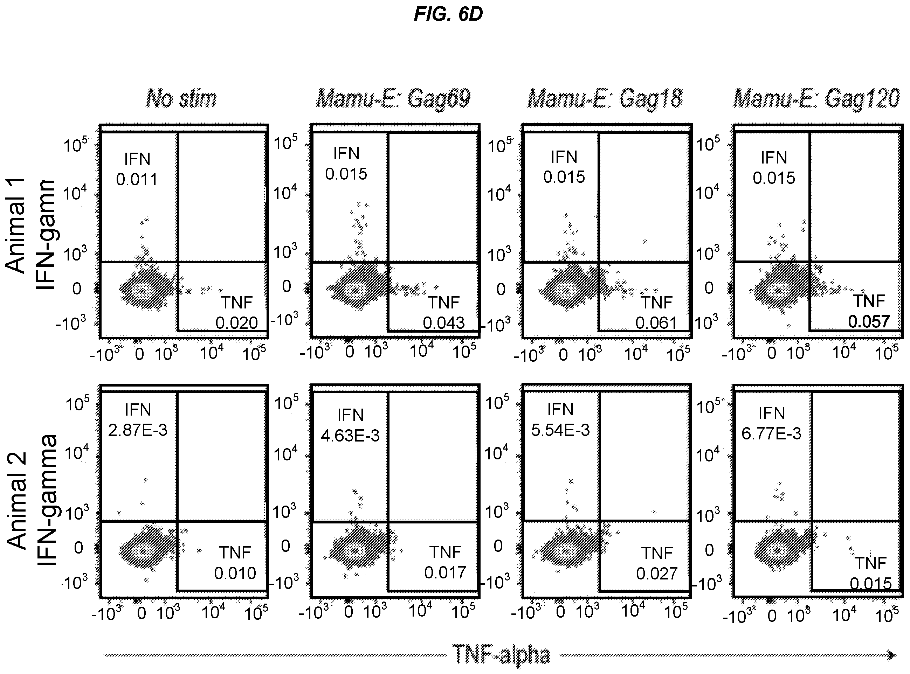

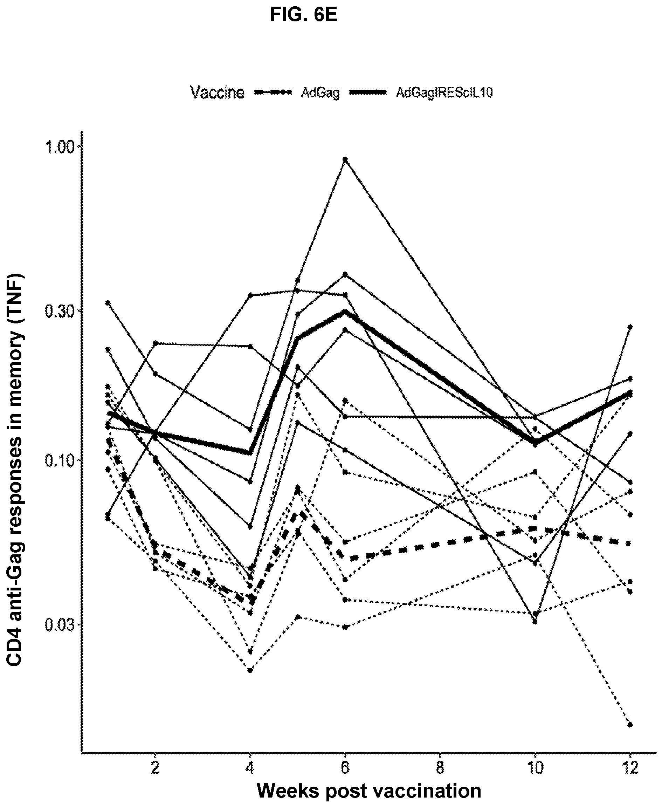

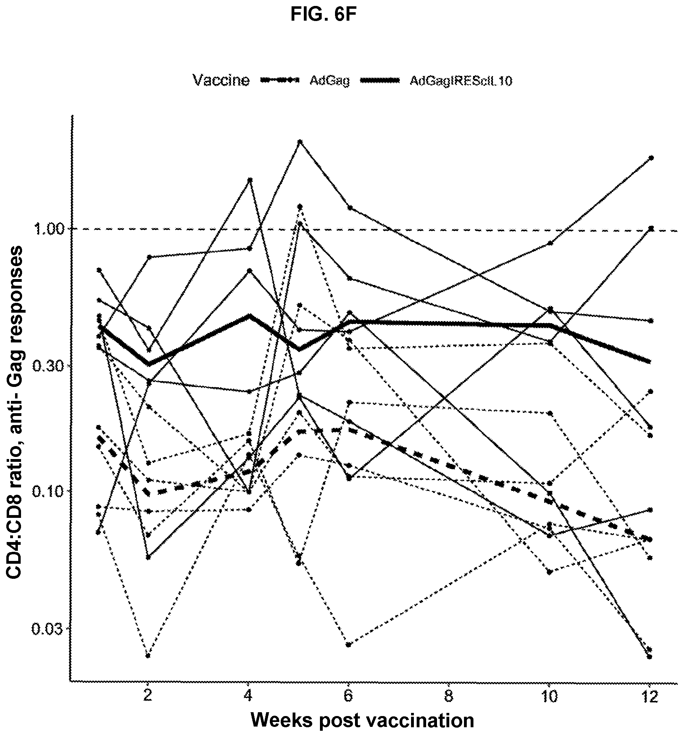

**FIGS. 6A-6F** show features of T cell responses among AdGag-IRES-cIL-10-vaccinated macaques. **FIG. 6A** shows that one of two animals initially mounted low CD8⁺ T cells responses to the vaccine antigen, while the other mounted virtually no response in CD8⁺ cells. The initial CD8⁺ T cell responses declined rapidly and nearly disappeared by week 8 after vaccination. The CD4⁺ T cell response (right) was low but clearly detectable. **FIG. 6B** shows CD8⁺ and CD4⁺ T cell responses 8 weeks after vaccination. The CD4⁺ T cell response (right panels) was low but clearly detectable. **FIG. 6C** shows that macaques vaccinated with AdGag-IRES-cIL-10 manifested a different cytokine environment in peripheral blood. Shown here is a higher concentration of circulating IL-8 in such animals. **FIG. 6D** shows Mamu-E-restricted T cell responses in animals receiving AdGag-IRES-cIL-10. The control responses observed in unstimulated cells are seen in the first column. Each of the subsequent columns shows immune responses to peptides that are known to be Mamu-E restricted: Gag69, Gag18, and Gag120. **FIG. 6E** shows CD4 anti-Gag immune responses over time post-vaccination. The responses in the CD4 compartment were strong and were substantially higher than those observed after administration of AdGag (solid line). **FIG. 6F** shows the ratio of CD4:CD8 anti-Gag responses over time post-vaccination. The ratio of CD4:CD8 responses was dramatically greater after administration of AdGagIREScIL10 vs. AdGag (solid line). In some cases immune responses to AdGagIREScIL10 provoked comparable CD4 and CD8 T cell responses, approaching the ratio of 1.0.

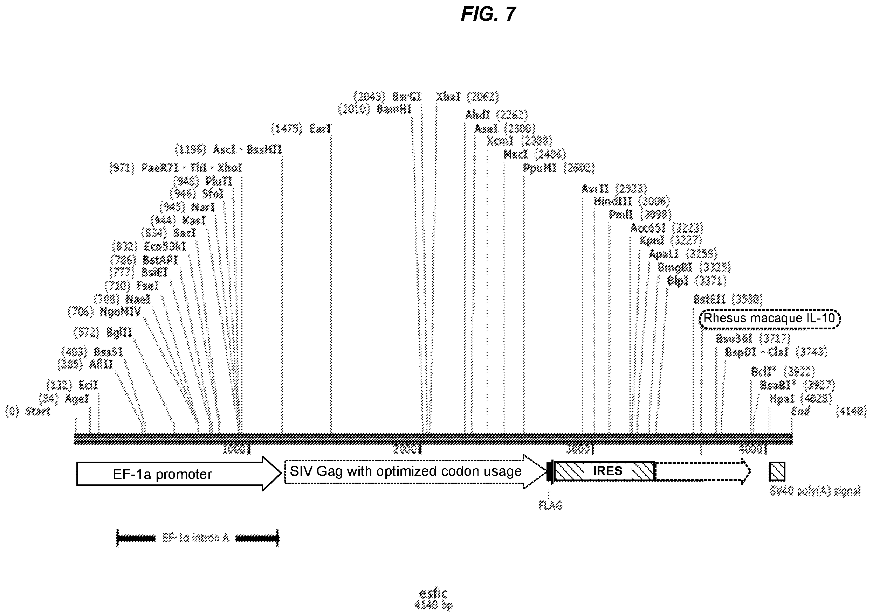

**FIG. 7** shows a schematic of an expression cassette encoding both the SIV gag antigen and rhesus macaques IL-10, with an intervening IRES sequence to allow expression of the IL-10 protein.

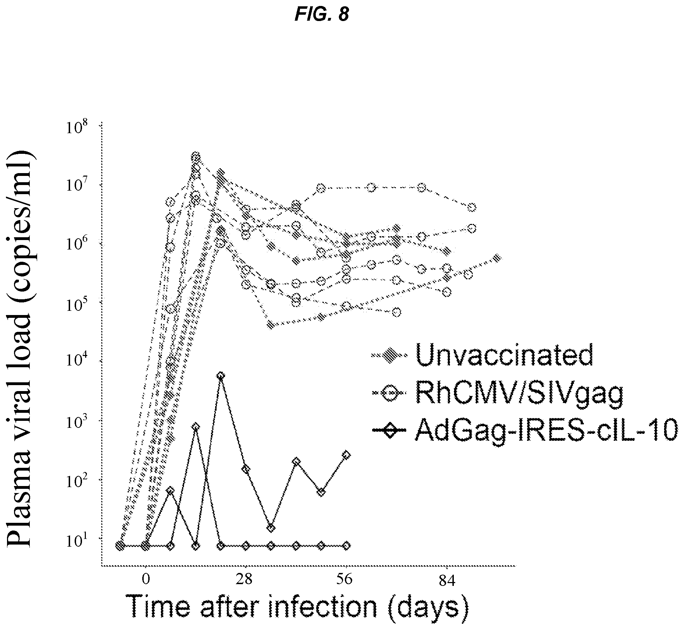

**FIG. 8** shows that AdGag-IRES-cIL-10 provides superior protection against SIV challenge. Macaques were vaccinated with AdGag-IRES-cIL-10 (solid lines, open diamonds), were vaccinated twice with a control rhesus CMV-based vaccine (dashed lines, open circles), or were left unvaccinated (dotted lines, filled diamonds).

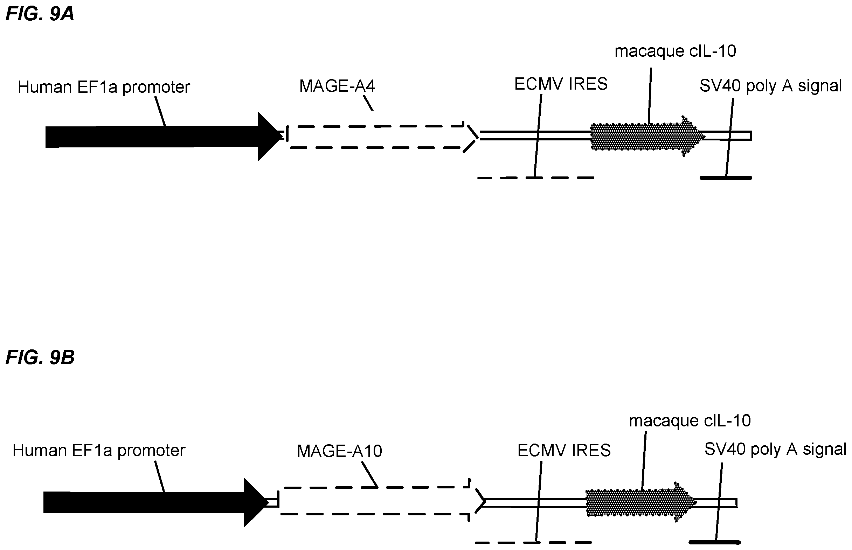

**FIGS. 9A** and **9B** show schematics of expression cassettes for melanoma-associated antigen (MAGE)-IL-10 systems of the present invention. **FIG. 9A** shows an expression cassette for a MAGEA4-IL-10 system. **FIG. 9B** shows an expression cassette for a MAGEA10-IL-10 system.

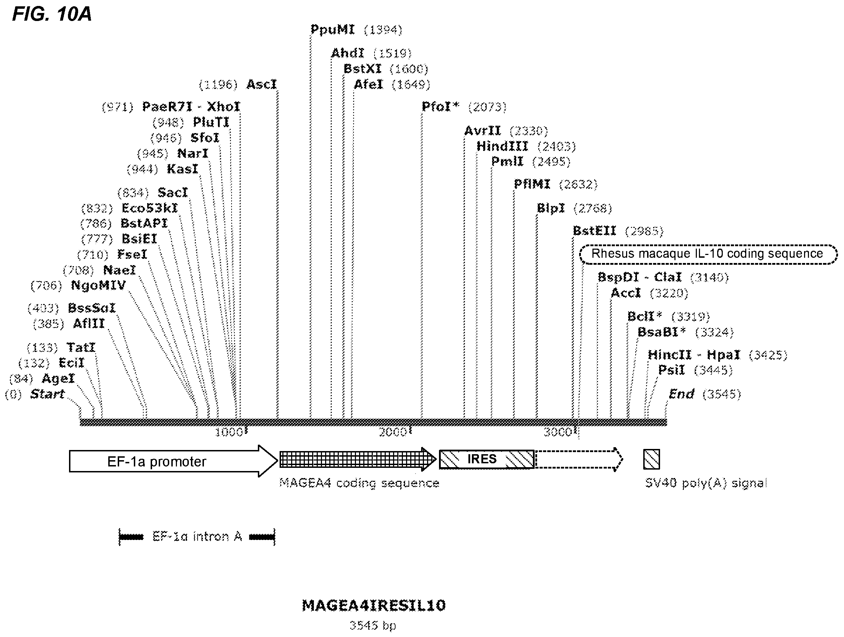

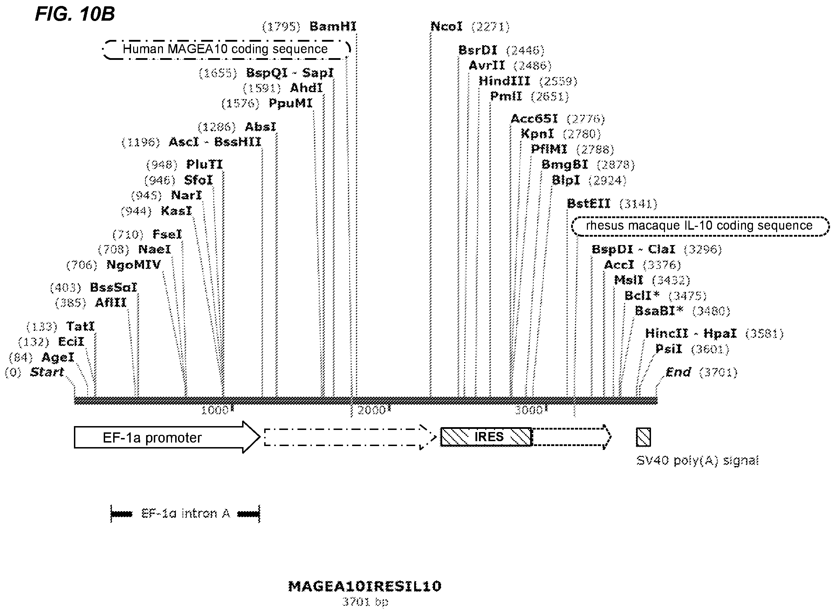

**FIGS. 10A** and **10B** show schematics of expression cassettes, including restriction endonuclease target sites, for melanoma-associated antigen (MAGE)-IL-10 systems of the present invention. **FIG. 10A** shows an expression cassette for a MAGEA4-IL-10 system. **FIG. 10B** shows an expression cassette for a MAGEA10-IL-10 system.

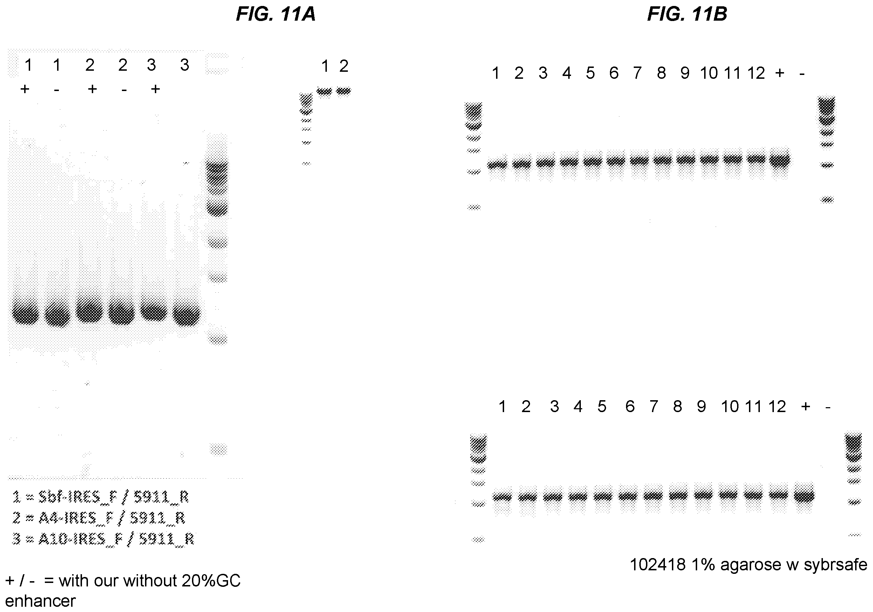

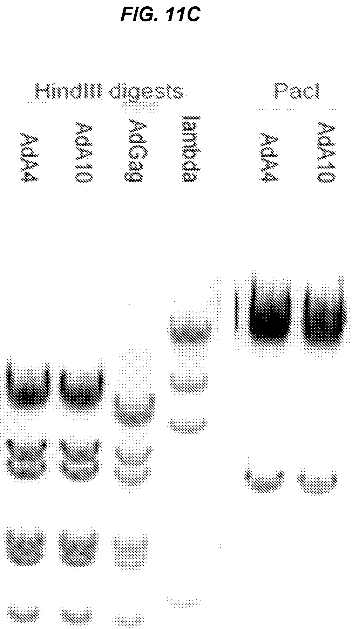

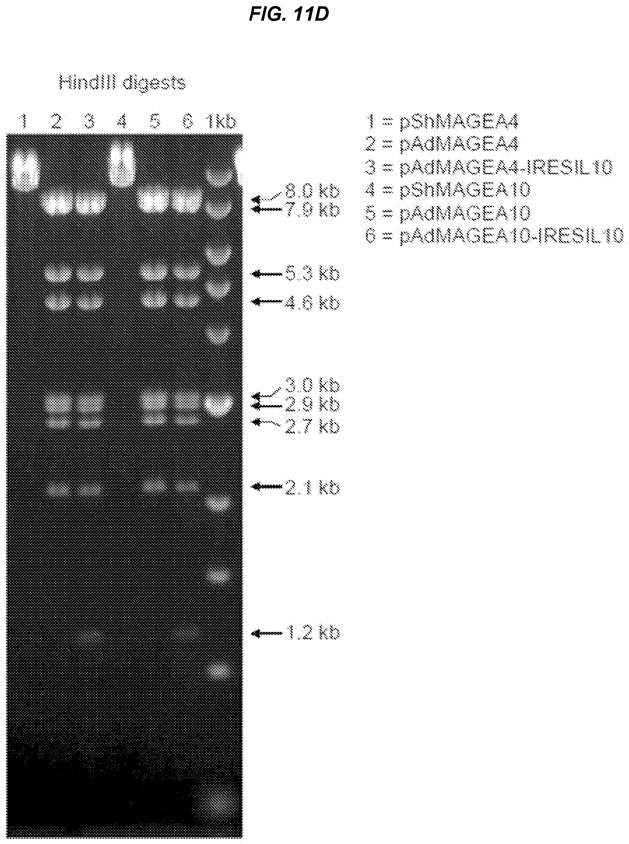

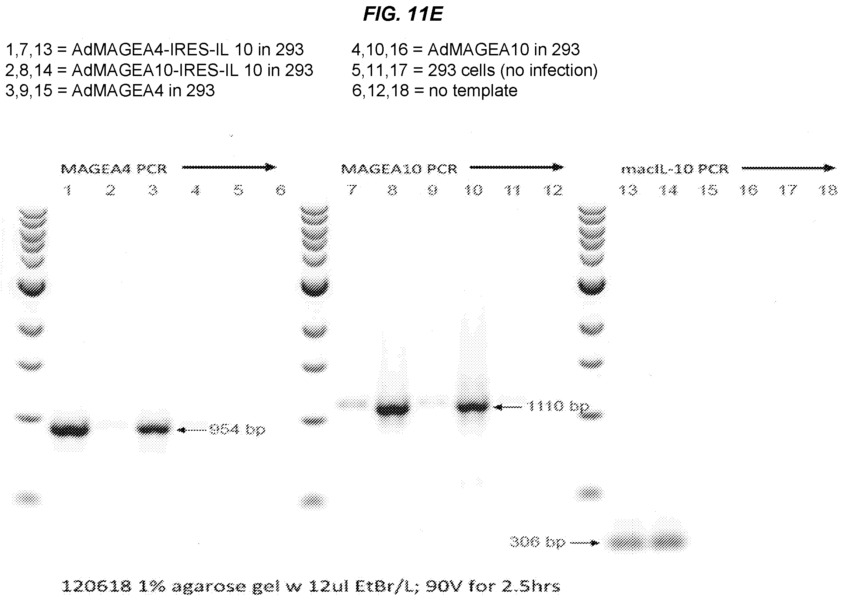

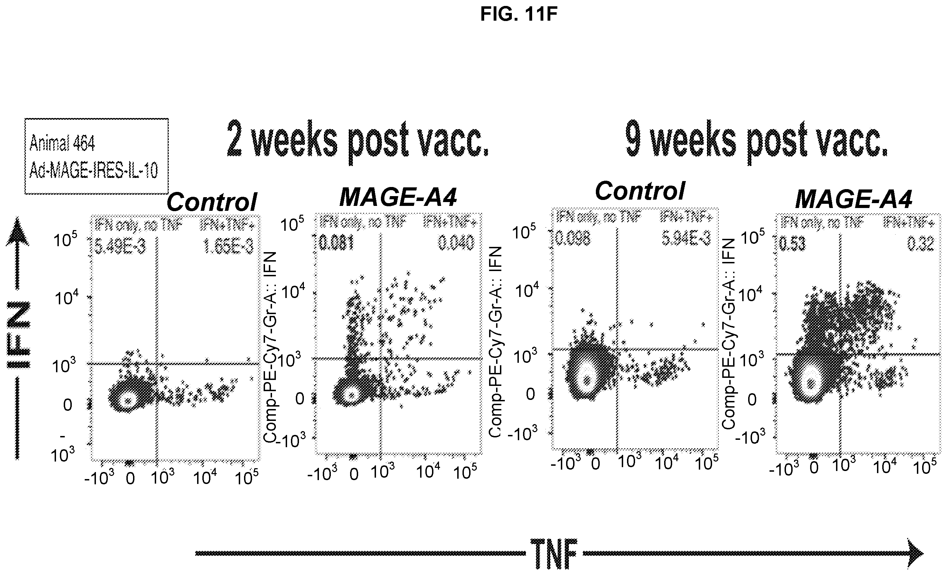

**FIGS. 11A-11F** show data pertaining to the construction of adenoviral vectors delivering MAGEA4 with IL-10 or MAGEA10 with IL-10. **FIG. 11A** shows preps for IRES-IL-10 and MAGE fragments. Left: IRES-cIL-10 fragments were amplified from plasmid 4pAdGagIRESIL10_7.1. Right: lanes 1 and 2 show *Sbf*I-digested pSh-MAGEA4 and pSh-MAGEA10 ready for overlap assembly with NEB 2xHiFiAssembly mix. **FIG. 11B** shows the results of pShMAGEA4-IRES-IL10 and pShMAGEA10-IRES-IL-10 colony screens (after assembly of IRES-cIL-10 fragments into pShMAGEA4 and pShMAGEA10). The Sbf-IRES_F and 5911_R oligos amplified a 1,162 bp band from insertion of IRES-cIL-10 at the *Sbj*I site, upstream of the polyadenylation signal. Clones 1-3 from top (pShMAGEA4-IRESIL10) and bottom (pShMAGEA10-IRESIL10) were chosen. **FIG. 11C** shows correct *Hind*III and *PacI* digests of complete plasmids containing MAGEA4-IRES-IL10 and MAGEA10-IRES-IL10 in adenoviral vector genomes, which resulted from recombination of the shuttle plasmids (**FIG. 11B**) with plasmids containing adenoviral vector genomes. **FIG. 11D** shows correct *Hind*III digests of selected Ad genomes with MAGEA4/10 and IL-10, demonstrating that select adenoviral vector genomes had the predicted structure. The 1.2-kb band, visible in lanes 3 and 6, was specific to vectors containing the IRES-IL10 fragment. **FIG. 11E** shows PCR confirmation of MAGEA4 or MAGEA10 expression with IL-10. cDNA templates were from DNase-treated total RNA (cDNA made with SuperscriptIV; 50 µg starting RNA template per PCR reaction). AdMAGEA4-IRES-IL10 produced both MAGEA4 (lane 1) and IL-10 (lane 13); AdMAGEA10-IRES-IL10 produced both MAGEA10 (lane 8) and IL-10 (lane 14). **FIG. 11F** shows immune responses following injection of a rhesus macaque serially with Ad5MAGEA4-IRES-IL10 and Ad26MAGEA4-IRES-IL10 (at 0 and 4 weeks).

### DETAILED DESCRIPTION OF THE INVENTION

### I. Introduction

Provided herein are vaccines that incorporate host and/or viral IL-10 such that an antigen and IL-10 are expressed in a cell to drive a therapeutic immune response. Also provided are vaccines that incorporate proteins with IL-10-like activity, *e.g.*, proteins that stimulate downstream IL-10 signaling, such that an antigen and the protein are expressed and IL-10-like activity is enhanced to drive a therapeutic immune response. Among other advantages, the IL-10-containing vaccines provided herein drive T cell responses that have unique qualities and enable improved therapeutic responses against infectious diseases and cancers.

### II. Definitions

As used herein, the following terms have the meanings ascribed to them unless specified otherwise.

The terms "a," "an," or "the" as used herein not only include aspects with one member, but also include aspects with more than one member. For instance, the singular forms "a," "an," and "the" include plural referents unless the context clearly dictates otherwise. Thus, for example, reference to "a cell" includes a plurality of such cells and reference to "the agent" includes reference to one or more agents known to those skilled in the art, and so forth.

The terms "about" and "approximately" as used herein shall generally mean an acceptable degree of error for the quantity measured given the nature or precision of the measurements. Typically, exemplary degrees of error are within 20 percent (%), preferably within 10%, and more preferably within 5% of a given value or range of values. Any reference to "about X" specifically indicates at least the values X, 0.8X, 0.81X, 0.82X, 0.83X, 0.84X, 0.85X, 0.86X, 0.87X, 0.88X, 0.89X, 0.9X, 0.91X, 0.92X, 0.93X, 0.94X, 0.95X, 0.96X, 0.97X, 0.98X, 0.99X, 1.01X, 1.02X, 1.03X, 1.04X, 1.05X, 1.06X, 1.07X, 1.08X, 1.09X, 1.1X, 1.11X, 1.12X, 1.13X, 1.14X, 1.15X, 1.16X, 1.17X, 1.18X, 1.19X, and 1.2X. Thus, "about X" is intended to teach and provide written description support for a claim limitation of, *e.g.,* "0.98X."

The term "antigen" refers to a molecule, or a portion thereof, that is capable of inducing an immune response (*e.g.,* in a subject). While in many instances an immune response involves the production of an antibody that targets or specifically binds to the antigen, as used herein the term "antigen" also refers to molecules that induce immune responses other than those that specifically involve the production of an antibody that targets the antigen, *e.g.,* a cell-mediated immune response involving expansion of T cells that target antigen-derived peptides presented on the surface of target cells. The antigen can originate from a foreign organism, such as a virus or microbe (*e.g.,* bacterial organism), or can originate from a foreign tissue. Alternatively, the antigen can originate from within a subject (*i.e.,* a subject in which the antigen induces an immune response). As a non-limiting example, an antigen can originate from a cell in a subject that has been injured, has been infected with a pathogen (*e.g.* a virus or microbe such as a bacterial organism), or is aberrant or damaged (*e.g.,* a cancer cell). The term also refers to molecules that do not necessarily induce immune responses by themselves but can be made to induce an immune response when presented in the right context, and/or in combination with other molecules that facilitate immune responses.

The term "interleukin" refers to a group of cytokines that play important roles in innate and adaptive immune system function. For example, some interleukins promote the development and differentiation of B lymphocytes, T lymphocytes, and hematopoietic cells. Most interleukins are produced by helper CD4 T lymphocytes, monocytes, macrophages, and endothelial cells. Interleukins can either enhance or inhibit immune function, depending on the particular interleukin.

Examples of interleukins (ILs) include IL-1 (which targets T helper cells, B cells, natural killer (NK) cells, macrophages, and endothelial cells, among others), IL-2 (which targets activated T cells and B cells, NK cells, macrophages, and oligodendrocytes), IL-3 (which targets hematopoietic stem cells and mast cells), IL-4 (which targets activated B cells, T cells, and endothelial cells), IL-5 (which targets B cells and eosinophils), IL-6 (which targets activated B cells, plasma cells, hematopoietic cells, and T cells, among others), IL-7 (which targets pre/pro-B and pre/pro-T cells, as well as NK cells), IL-8 (also known as CXCL8, which targets neutrophils, basophils, and lymphocytes), IL-9 (which targets T cells and B cells), IL-10 (which targets macrophages, B cells, mast cells, Th1 cells, and Th2 cells), IL-11 (which targets bone marrow stromal cells), IL-12 (which targets activated T cells and NK cells), IL-13 (which targets Th2 cells, B cells, and macrophages), IL-14 (which targets activated B cells), IL-15 (which targets T cells and activated B cells), IL-16 (which targets CD4⁺ T cells), IL-17 (which targets epithelial and endothelial cells, among others), IL-18 (which targets Th1 cells and NK cells), IL-19, IL-20, IL-21 (which targets dendritic cells and all lymphocytes), IL-22, IL-23, IL-24, IL-25, IL-26, IL-27, IL-28, IL-29, IL-30, IL-31, IL-32, IL-33, IL-34, IL-35, and IL-36.

"Interleukin-8" or "IL-8" is produced by macrophages, among other cell types including endothelial cells, epithelial cells and airway smooth muscle cells. Primary functions of IL-8 include the induction of chemotaxis in target cells such as neutrophils and other granulocytes, stimulation of phagocytosis by target cells, and the promotion of angiogenesis. Non-limiting examples of human IL-8 amino acid sequences are set forth under NCBI Reference Sequence numbers NP_000575 and NP_001341769.

"Interleukin-10" or "IL-10," also known as "cytokine synthesis inhibitory factor," is an anti-inflammatory cytokine that is encoded by the *IL10* gene in humans. IL-10, which is a homodimer having subunits that are each 178 amino acids in length, binds to a receptor complex that consists of two IL-10 receptor-1 proteins and two IL-10 receptor-2 proteins. Binding of IL-10 to the receptor complex induces STAT3 signaling, via JAK1 phosphorylation of the cytoplasmic tails of IL-10 receptor-1 and Tyk2 phosphorylation of the cytoplasmic tails of IL-10 receptor-2. IL-10 is produced by subsets of monocytes, Th2 cells, CD8⁺ T cells, mast cells, macrophages, and B cells. IL-10 has multiple effects, including but not limited to, downregulation of the expression of Th1 and macrophage cytokines (*e.g.,* interferon-gamma, IL-1-beta, IL-2, IL-6, IL-12, TNF-alpha, and GM-CSF), MHC class II antigens, and/or macrophage co-stimulatory molecules, blockade of NF-κB activity, and/or enhancement of B cell survival, proliferation, and/or antibody production. A non-limiting example of a human IL-10 amino acid sequence is set forth under NCBI Reference Sequence number NP_000563. Another non-limiting example of a human IL-10 amino acid sequence is shown as SEQ ID NO: 7.

"Interleukin-12" or "IL-12" is produced by dendritic cells, macrophages, neutrophils, and B-lymphoblastoid cells in response to antigenic stimulation. IL-12 is involved in the differentiation of naive T cells into Th1 cells, and also plays a role in the enhancement of the cytotoxic activity of NK cells and CD8⁺ T cells.

The term "protein that has interleukin-10-like activity" or "protein that has IL-10-like activity" refers to any protein that functions in a similar way to interleukin-10 (IL-10) or produces a similar effect (*e.g.,* has a similar immunomodulatory effect) to IL-10. The term includes, but is not limited to, mammalian IL-10 proteins (*e.g.,* human, non-human primate, or rodent IL-10 proteins) and proteins encoded by viral IL-10 genes or portions thereof (*e.g.,* CMV IL-10 genes) such as HCMVIL-10 in HCMV and RhCMVIL-10 in RhCMV, proteins that bind to an IL-10 receptor, proteins that stimulate downstream IL-10 receptor signaling, and functional portions thereof. The term also includes, but is not limited to, proteins encoded by corresponding viral IL-10 genes, or portions thereof, in SCCMV/AGMCMV, BaCMV, OMCMV, and SMCMV, as well as homologs thereof. A protein that has IL-10-like activity can, for example, downregulate the expression of Th1 and macrophage cytokines (*e.g.,* interferon-gamma, IL-1-beta, IL-2, IL-6, IL-12, TNF-alpha, and GM-CSF), MHC class II antigens, and/or macrophage co-stimulatory molecules, promote blockade of NF-κB activity, and/or enhance B cell survival, proliferation, and/or antibody production. Non-limiting examples of amino acid sequences or nucleic acid sequences that encode proteins having IL-10-like activity are set forth under SEQ ID NOS:4-7. A non-limiting example of a protein having IL-10-like activity that stimulates downstream IL-10 receptor signaling is STAT3 (*e.g.,* as shown in SEQ ID NOS: 10-11) or a constitutively active form thereof (*e.g.,* STAT3C) (*e.g.,* as shown in SEQ ID NOS: 12-13).

"STAT3" or "Signal Transducer and Activator of Transcription 3" is a member of the STAT protein family and is encoded by the *STAT3* gene. STAT3 signaling is induced by binding of IL-10 to the receptor complex, via JAK1 phosphorylation of the cytoplasmic tails of IL-10 receptor-1 and Tyk2 phosphorylation of the cytoplasmic tails of IL-10 receptor-2. Upon activation, STAT3 forms homo- or heterodimers and translocates to the nucleus to activate gene transcription. "STAT3C" is a constitutively active form of STAT3 in which two amino acids within the C-terminal loop (*e.g.,* residues 662 and 664 of the human STAT3 protein as shown in SEQ ID NO: 10) are replaced with cysteine residues, leading to spontaneous dimerization, DNA binding, and transcriptional activation. Non-limiting examples of human STAT3 proteins are set forth in, *e.g.,* NCBI Reference Sequence Nos. NP_001356448.1, NP_003141.2, NP_001356446.1, NP_001356443.1, NP_001356447.1, NP_001356442.1, NP_001356449.1, NP_001356445.1, NP_998827.1, NP_644805.1, XP_016880462.1, XP_024306664.1, and the sequence shown as SEQ ID NO: 10. Non-limiting examples of human STAT3C proteins are set forth as, *e.g.,* SEQ ID NO: 12, or any of NP_001356448.1, NP_003141.2, NP_001356446.1, NP_001356443.1, NP_001356447.1, NP_001356442.1, NP_001356449.1, NP_001356445.1, NP_998827.1, NP_644805.1, XP_016880462.1, XP_024306664.1 in which the amino acids corresponding to amino acids 662 and 664 of SEQ ID NO: 10 are replaced with cysteine residues.

The term "heterologous protein having IL-10-like activity" refers to a protein having IL-10-like activity that is expressed under the control of a heterologous regulatory sequence (*e.g.,* a heterologous regulatory sequence that is present within a system or recombinant polynucleotide of the present invention), *i.e.,* is not expressed under the sole control of all the endogenous regulatory sequences of the host or virus from which the protein having IL-10-like activity derives.

The term "exogenous protein having IL-10-like activity" refers to a protein having IL-10-like activity that is not encoded by or expressed from a nucleic acid sequence present within a system or recombinant polynucleotide of the present invention.

The term "nucleic acid sequence encoding a peptide" refers to a segment of DNA, which in some embodiments may be a gene or a portion thereof, that is involved in producing a peptide chain (*e.g.,* an antigen or a protein having IL-10-like activity). A gene will generally include regions preceding and following the coding region (leader and trailer) involved in the transcription/translation of the gene product and the regulation of the transcription/translation. A gene can also include intervening sequences (introns) between individual coding segments (exons). Leaders, trailers, and introns can include regulatory elements that are necessary during the transcription and the translation of a gene (*e.g.,* promoters, terminators, translational regulatory sequences such as ribosome binding sites and internal ribosome entry sites, enhancers, silencers, insulators, boundary elements, replication origins, matrix attachment sites and locus control regions, etc.). A "gene product" can refer to either the mRNA or protein expressed from a particular gene.

The terms "expression" and "expressed" refer to the production of a transcriptional and/or translational product, *e.g.,* of a nucleic acid sequence encoding a protein (*e.g.,* an antigen or a protein having IL-10-like activity). In some embodiments, the term refers to the production of a transcriptional and/or translational product encoded by a gene (*e.g.,* a gene encoding an antigen or a protein having IL-10-like activity) or a portion thereof. The level of expression of a DNA molecule in a cell may be assessed on the basis of either the amount of corresponding mRNA that is present within the cell or the amount of protein encoded by that DNA produced by the cell.

The term "recombinant" when used with reference, *e.g.,* to a polynucleotide, protein, vector, or cell, indicates that the polynucleotide, protein, vector, or cell has been modified by the introduction of a heterologous nucleic acid or protein or the alteration of a native nucleic acid or protein, or that the cell is derived from a cell so modified. For example, recombinant polynucleotides contain nucleic acid sequences that are not found within the native (non-recombinant) form of the polynucleotide.

The term "immune response" refers to any response that is induced (*e.g.,* in a subject) by an antigen, including the induction of immunity against pathogens (*e.g.,* viruses and microbes such as bacteria). Immune responses induced by systems, recombinant polynucleotides, compositions, and methods of the present invention are typically desired, intended, and/or protective immune responses. The term includes the production of antibodies against an antigen, as well as the development, maturation, differentiation, and activation of immune cells (*e.g.,* B cells and T cells). In some instances, an immune response comprises increasing the number or activation of MHC-E-restricted CD4⁺ and/or CD8⁺ T cells (*e.g.,* in a subject). The term also includes increasing or decreasing the expression or activity of cytokines that are involved in regulating immune function. As another non-limiting example, an immune response can comprise increasing the expression or activity of interferon-gamma and/or tumor necrosis factor-alpha (*e.g.,* in a subject).

Further examples of desired, intended, and/or protective immune responses that can be induced according to recombinant polynucleotides, compositions, and methods of the present invention include, but are not limited to, those involving class Ia-, class Ib-, or class II-restricted CD4⁺ T cells; class Ia-, class Ib-, or class II-restricted CD8⁺ T cells; cytokine-producing T cells (*e.g.,* T cells that produce IFN-gamma, TNF-alpha, IL-1-beta, IL-2, IL-4, IL-5, IL-10, IL-13, IL-17, IL-18, or IL-23); CCR7⁻CD8⁺ T cells (*e.g.*, effector-memory cells); CXCR5⁺ T cells (*i.e.,* those homing to B cell follicles); CD4⁺ regulatory T cells; CD8⁺ regulatory T cells; antigen-specific T follicular helper cells; antibody production; NK cells; NKG2C⁺ NK cells; CD57⁺ NK cells; FcR-gamma-negative NK cells; and NK-CTL cells, *i.e.*, CD8⁺ T cells expressing molecules typical of NK cells, such as NKG2A.

The term "cytomegalovirus" or "CMV" refers to viruses that include members of the *Cytomegalovirus* genus of viruses (within the order *Herpesvirales*, family *Herpesviridae*, subfamily *Betaherpesvirinae).* The term includes, but is not limited to, *Human cytomegalovirus* (HCMV; also known as *Human herpesvirus 5* (HHV-5)), *Simian cytomegalovirus* (SCCMV or AGMCMV), *Baboon cytomegalovirus* (BaCMV), *Owl monkey cytomegalovirus* (OMCMV), *Squirrel monkey cytomegalovirus* (SMCMV), and *Rhesus cytomegalovirus* (RhCMV) that infects macaques.

The term "antigen-presenting cell" or "APC" refers to a cell that displays or presents an antigen, or a portion thereof, on the surface of the cell. Typically, antigens are displayed or presented with a major histocompatibility complex (MHC) molecule. Almost all cell types can serve as APCs, and APCs are found in a large number of different tissue types. Professional APCs, such as dendritic cells, macrophages, and B cells, present antigens to T cells in a context that most efficiently leads to their activation and subsequent proliferation. Many cell types present antigens to cytotoxic T cells.

The term "infectious disease" refers to any disease or disorder caused by an organism, (*e.g.,* viruses, bacteria, fungi, protozoa, helminths, and parasitic organisms). The term includes diseases and disorders that are transmitted from one subject to another (*e.g.,* human to human, non-human animal to human, and human to non-human animal), as well as those caused by ingesting contaminated food or water or by exposure to pathogenic organisms (*e.g.,* in the environment).

An "infectious disease antigen" refers to any molecule originating from an infectious disease-causing organism that can induce an immune response (*e.g.,* in a subject). For example, an infectious disease antigen can originate from a virus, bacterium, fungus, protozoan, helminth, or parasite, and can be, for example, a bacterial wall protein, a viral capsid or structural protein (*e.g.,* a retroviral group-specific antigen (gag) protein, such as an HIV or SIV gag protein), or a portion thereof. In some embodiments, the infectious disease antigen is a bacterial infectious disease antigen from *Mycobacterium tuberculosis.*

The term "cancer" refers to any of various malignant neoplasms characterized by the proliferation of anaplastic cells that tend to invade surrounding tissue and metastasize to new body sites. Non-limiting examples of different types of cancer suitable for treatment using the methods and compositions of the present invention include colorectal cancer, colon cancer, anal cancer, liver cancer, ovarian cancer, breast cancer, lung cancer, bladder cancer, thyroid cancer, pleural cancer, pancreatic cancer, cervical cancer, prostate cancer, testicular cancer, bile duct cancer, gastrointestinal carcinoid tumors, esophageal cancer, gall bladder cancer, rectal cancer, appendix cancer, small intestine cancer, stomach (gastric) cancer, renal cancer (*e.g.,* renal cell carcinoma), cancer of the central nervous system, skin cancer, oral squamous cell carcinoma, choriocarcinomas, head and neck cancers, bone cancer, osteogenic sarcomas, fibrosarcoma, neuroblastoma, glioma, melanoma, leukemia (*e.g.,* acute lymphocytic leukemia, chronic lymphocytic leukemia, acute myelogenous leukemia, chronic myelogenous leukemia, or hairy cell leukemia), lymphoma (*e.g.,* non-Hodgkin's lymphoma, Hodgkin's lymphoma, B-cell lymphoma, or Burkitt's lymphoma), and multiple myeloma.

The term "tumor-associated antigen" or "TAA" refers to any antigen that is produced by a tumor cell (*i.e.,* any protein or molecule produced by a tumor cell that can induce an immune response, *e.g.,* in a subject). TAAs include, but are not limited to, products of mutated oncogenes and mutated tumor suppressor genes, overexpressed or aberrantly expressed cellular proteins, antigens that are produced by oncogenic viruses, oncofetal antigens, altered cell surface glycolipids and glycoproteins, and antigens that are cell type-specific. In some embodiments, the TAA is one that newly arises in a tumor (*e.g.,* in a subject's tumor).

Non-limiting examples of TAAs include the melanoma-associated antigens (MAGEs). MAGE proteins contain a conserved domain that is about 200 amino acids in length and is usually located near the C-terminal end of the protein, although the conserved domain is located closer to the central portion of some MAGE proteins. Human MAGE proteins include MAGEA1, MAGEA2, MAGEA2B, MAGEA3, MAGEA4, MAGEA5, MAGEA6, MAGEA7P, MAGEA8, MAGEA9, MAGEA9B, MAGEA10, MAGEA11, MAGEA12, MAGEA13P, MAGEB1, MAGEB2, MAGEB3, MAGEB4, MAGEB5, MAGEB6, MAGEB10, MAGEB16, MAGEB17, MAGEB18, MAGEC1, MAGEC2, MAGEC3, MAGED1, MAGED2, MAGED3 (also known as "trophin" or "TRO"), MAGED4, MAGED4B, MAGEE1, MAGEE2, MAGEF1, MAGEEG1 (also known as "NSMCE3"), MAGEH1, MAGEL2, and NDN.

The protein "melanoma-associated antigen 4" or "MAGEA4" is encoded by the *MAGEA4* gene in humans, located at chromosomal location Xq28. Non-limiting examples of human MAGEA4 amino acid sequences are set forth under NCBI Reference Sequence numbers NP_001011548, NP_001011549, NP_001011550, and NP_002353.

The protein "melanoma-associated antigen 10" or "MAGEA10" is encoded by the *MAGEA10* gene in humans, located at chromosomal location Xq28. Non-limiting examples of human MAGEA10 amino acid sequences are set forth under NCBI Reference Sequence numbers NP_001011543, NP_001238757, and NP_066386.

The term "prostate-specific antigen" or "PSA" refers to a glycoprotein encoded by the *KLK3* gene in humans, and is also known as "gamma-seminoprotein" and "kallikrein-3." PSA is present in small quantities in the serum of men with normal prostates, but is often elevated in the presence of prostate cancer or other disorders of the prostate. Non-limiting examples of human PSA amino acid sequences are set forth under NCBI Reference Sequence numbers NP_001025218, NP_001025219, NP_001639.

The term "NY-ESO-1" refers to the cancer/testis family tumor antigen that is also known as "cancer/testis antigen 1" and is encoded by the *CTAG1B* gene in humans. NY-ESO-1 is highly expressed in many poor-prognosis melanomas. A non-limiting example of a human NY-ESO-1 amino acid sequence is set forth under NCBI Reference Sequence number NP_001318.1.

The term "major histocompatibility complex" or "MHC" refers to a group of cell surface proteins that are essential for recognition of foreign molecules by the adaptive immune system. The primary function of MHC molecules is to bind to antigens or antigen-derived peptides that are derived from pathogens and to subsequently display the antigens on the surfaces of cells in order to facilitate recognition by T cells. MHC molecules also participate in interactions between leukocytes and other leukocytes, as well as between leukocytes and other cell types within the body. In humans, the MHC is also known as the "human leukocyte antigen complex" or "HLA complex."

Class I MHC molecules, which predominantly present peptides from inside the cell, are encoded by the *HLA-A, HLA-B, HLA-C, HLA-E, HLA-F,* and *HLA-G* genes in humans. *HLA-A, HLA-B*, and *HLA-C* genes are more polymorphic and thus considered *Class Ia* MHC molecules, while *HLA-E, HLA-F*, and *HLA-G* genes are less polymorphic and thus considered *Class Ib* MHC molecules. *HLA-K* and *HLA-L* are also known to exist as pseudogenes. In addition, beta-2-microglobulin is an MHC class I protein, encoded by the *B2M* gene. Non-limiting examples of *HLA-A* nucleotide sequences are set forth under NCBI Reference Sequence numbers NM_001242758 and NM_002116. A non-limiting example of an *HLA-B* nucleotide sequence is set forth under NCBI Reference Sequence number NM_005514. Non-limiting examples of *HLA-C* nucleotide sequences are set forth under NCBI Reference Sequence numbers NM_001243042 and NM_002117. A non-limiting example of an *HLA-E* nucleotide sequence is set forth under NCBI Reference Sequence number NM_005516. A non-limiting example of an *HLA-F* nucleotide sequence is set forth under NCBI Reference Sequence number NM_018950. A non-limiting example of an *HLA-G* nucleotide sequence is set forth under NCBI Reference Sequence number NM_002127. A non-limiting example of a *B2M* nucleotide sequence is set forth under NCBI Reference Sequence number NM_004048.

Class II MHC molecules, which predominantly present antigens from the outside of the cell to T lymphocytes, are encoded by the *HLA-DP, HLA-DM, HLA-DO, HLA-DQ*, and *HLA-DR* genes. *HLA-DM* genes include *HLA-DMA* and *HLA-DMB. HLA-DO* genes include *HLA-DOA* and *HLA-DOB. HLA-DP* genes include *HLA-DPA1* and *HLA-DPB1. HLA-DQ* genes include *HLA-DQA1, HLA-DQA2, HLA-DQB1,* and *HLA-DQB2. HLA-DR* genes include *HLA-DRA, HLA-DRB1, HLA-DRB3, HLA-DRB4,* and *HLA-DRB5.* Non-limiting examples of *HLA-DMA* and *HLA-DMB* nucleotide sequences are set forth under NCBI Reference Sequence numbers NM_006120 and NM_002118, respectively. Non-limiting examples of HLA-DRA, HLA-DRB1, HLA-DRB3, HLA-DRB4, and HLA-DRB5 nucleotide sequences are set forth in NCBI Reference Sequence numbers NM_01911, NM_002124, NM_022555, NM_021983, NM_002125, respectively.

The term "tumor necrosis factor-alpha" or "TNF-alpha" refers to the cytokine that is encoded by the *TNFA* gene in humans. TNF-alpha is produced by activated macrophages, T cells, NK cells, neutrophils, eosinophils, mast cells, and neurons. TNF-alpha is involved in processes such as the induction of fever, apoptosis, cachexia, and inflammation, as well as the inhibition of tumorigenesis and viral replication. TNF-alpha also functions in promoting responses to sepsis. A non-limiting example of a human TNF-alpha amino acid sequence is set forth under NCBI Reference Sequence number NP_000585.

The term "interferon-gamma," "IFN-γ," or "IFN-gamma" refers to a cytokine that is a member of the type II class of interferons and is encoded by the *IFNG* gene. IFN-γ plays important roles in innate and adaptive immunity against viral, bacterial, and protozoal infections. In particular, IFN-γ is a macrophage activator and induces expression of class II MHC molecules. IFN-γ is produced by natural killer cells, natural killer T cells, CD4⁺ Th1 cells, CD8⁺ cytotoxic T lymphocyte cells, and non-cytotoxic innate lymphoid cells. A non-limiting example of a human IFN-γ amino acid sequence is set forth under NCBI Reference Sequence number NP_000610.

As used herein, the term "tropism" refers to the ability of a composition of the present invention (*e.g.,* a system of the present invention, a recombinant polynucleotide of the present invention, or a viral particle comprising or encoded by a recombinant polynucleotide of the present invention) to enter, infect, or replicate in a particular cell or tissue type (*e.g.,* a target cell or tissue type found in a subject in whom an immune response against an antigen is being induced). As non-limiting examples, tropism can be broad (*i.e.,* a system or recombinant polynucleotide of the present invention can enter a large number of different cell or tissue types, or a virus comprising or encoded by a system or recombinant polynucleotide of the present invention can infect or replicate in a large number of different cell or tissue types) or can be narrow (*i.e.,* a system or recombinant polynucleotide of the present invention can enter only a small number of different cell or tissue types, or a virus comprising or encoded by a system or recombinant polynucleotide of the present invention can infect or replicate in only a small number of different cell or tissue types). Furthermore, as described further herein, systems and recombinant polynucleotides of the present invention can be modified such that they possess tropism for specific desired cell or tissue type(s) (*i.e.,* a system or recombinant polynucleotide can enter specific desired cell or tissue type(s), or a viral particle comprising or encoded by a system or recombinant polynucleotide of the present invention can enter specific desired cell or tissue type(s)). In some instances, tropism for a specific cell or tissue type is increased or imparted by the addition of a nucleic acid sequence that encodes a cellular targeting ligand.

The term "cellular targeting ligand" refers to any protein, molecule, or portion thereof that increases the ability of a composition of the present invention to enter, infect, or replicate in a specific cell or tissue type. As a non-limiting example, a cellular targeting ligand can increase the ability of a composition of the present invention (*e.g.,* a system or recombinant polynucleotide of the present invention, or a viral particle comprising or encoded by a system or recombinant polynucleotide of the present invention) to be recognized by a specific target cell or tissue type, or to recognize a specific target cell or tissue type. Cellular targeting ligands include, but are not limited to, antibody fragments that recognize a target cell antigen, ligands that are recognized by a target cell cognate receptor, and viral capsid proteins that recognize a target cell.

As used herein, the terms "polynucleotide," "nucleic acid," and "nucleotide," refer to deoxyribonucleic acids (DNA) or ribonucleic acids (RNA) and polymers thereof. The term includes, but is not limited to, single-, double-, or multi-stranded DNA or RNA, genomic DNA, cDNA, and DNA-RNA hybrids, as well as other polymers comprising purine and/or pyrimidine bases or other natural, chemically modified, biochemically modified, non-natural, synthetic, or derivatized nucleotide bases. Unless specifically limited, the term encompasses nucleic acids containing known analogs of natural nucleotides that have similar binding properties as the reference nucleic acid. Unless otherwise indicated, a particular nucleic acid sequence also implicitly encompasses conservatively modified variants thereof (*e.g.,* degenerate codon substitutions), homologs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

The terms "vector" and "expression vector" refer to a nucleic acid construct, generated recombinantly or synthetically, with a series of specified nucleic acid elements that permit transcription of a particular nucleic acid sequence (*e.g.,* encoding an antigen and/or a protein having IL-10-like activity) in a host cell or engineered cell. In some embodiments, a vector includes a polynucleotide to be transcribed, operably linked to a promoter. Other elements that may be present in a vector include those that enhance transcription (*e.g.*, enhancers), those that terminate transcription (*e.g.*, terminators), those that confer certain binding affinity or antigenicity to a protein (*e.g.,* recombinant protein) produced from the vector, and those that enable replication of the vector and its packaging (*e.g.,* into a viral particle). In some embodiments, the vector is a viral vector (*i.e.,* a viral genome or a portion thereof). A vector may contain nucleic acid sequences or mutations, for example, that increase tropism and/or modulate immune function.

The terms "polypeptide," "peptide," and "protein" are used interchangeably herein to refer to a polymer of amino acid residues. All three terms apply to amino acid polymers in which one or more amino acid residues are an artificial chemical mimetic of a corresponding naturally occurring amino acid, as well as to naturally occurring amino acid polymers and non-naturally occurring amino acid polymers. As used herein, the terms encompass amino acid chains of any length, including full-length proteins, wherein the amino acid residues are linked by covalent peptide bonds.

The terms "subject," "individual," and "patient" are used interchangeably herein to refer to a vertebrate, preferably a mammal, more preferably a human. Mammals include, but are not limited to, murines, mice, rats, simians, humans, farm animals, sport animals, and pets. Tissues, cells and their progeny of a biological entity obtained *in vivo* or cultured *in vitro* are also encompassed.

As used herein, the term "administering" includes oral administration, topical contact, administration as a suppository, intravenous, intraperitoneal, intramuscular, intralesional, intratumoral, intrathecal, intranasal, intraosseous, or subcutaneous administration to a subject. Administration is by any route, including parenteral and transmucosal (*e.g.,* buccal, sublingual, palatal, gingival, nasal, vaginal, rectal, or transdermal). Parenteral administration includes, *e.g.,* intravenous, intramuscular, intraarterial, intradermal, subcutaneous, intraperitoneal, intraventricular, intraosseous, and intracranial. Other modes of delivery include, but are not limited to, the use of liposomal formulations, intravenous infusion, transdermal patches, *etc.*

The term "treating" refers to an approach for obtaining beneficial or desired results including, but not limited to, a therapeutic benefit and/or a prophylactic benefit. "Therapeutic benefit" means any therapeutically relevant improvement in or effect on one or more diseases, conditions, or symptoms under treatment. Therapeutic benefit can also mean to effect a cure of one or more diseases, conditions, or symptoms under treatment. Furthermore, therapeutic benefit can also mean to increase survival. For prophylactic benefit, the compositions may be administered to a subject at risk of developing a particular disease, condition, or symptom, or to a subject reporting one or more of the physiological symptoms of a disease, even though the disease, condition, or symptom may not yet be present.

The term "therapeutically effective amount" or "sufficient amount" refers to the amount of a system, recombinant polynucleotide, or composition described herein that is sufficient to effect beneficial or desired results. The therapeutically effective amount may vary depending upon one or more of: the subject and disease condition being treated, the weight and age of the subject, the severity of the disease condition, the immune status of the subject, the manner of administration and the like, which can readily be determined by one of ordinary skill in the art. The specific amount may vary depending on one or more of: the particular agent chosen, the target cell type, the location of the target cell in the subject, the dosing regimen to be followed, whether it is administered in combination with other compounds, timing of administration, and the physical delivery system in which it is carried.

For the purposes herein an effective amount is determined by such considerations as may be known in the art. The amount must be effective to achieve the desired therapeutic effect in a subject suffering from a disease such as an infectious disease or cancer. The desired therapeutic effect may include, for example, amelioration of undesired symptoms associated with the disease, prevention of the manifestation of such symptoms before they occur, slowing down the progression of symptoms associated with the disease, slowing down or limiting any irreversible damage caused by the disease, lessening the severity of or curing the disease, or improving the survival rate or providing more rapid recovery from the disease. Further, in the context of prophylactic treatment the amount may also be effective to prevent the development of the disease.

The term "pharmaceutically acceptable carrier" refers to a substance that aids the administration of an active agent to a cell, an organism, or a subject. "Pharmaceutically acceptable carrier" also refers to a carrier or excipient that can be included in the compositions of the invention and that causes no significant adverse toxicological effect on the patient. Non-limiting examples of pharmaceutically acceptable carriers include water, sodium chloride (NaCl), normal saline solutions, lactated Ringer's, normal sucrose, normal glucose, binders, fillers, disintegrants, lubricants, coatings, sweeteners, flavors and colors, liposomes, dispersion media, microcapsules, cationic lipid carriers, isotonic and absorption delaying agents, and the like. The carrier may also comprise or consist of substances for providing the formulation with stability, sterility and isotonicity (*e.g.* antimicrobial preservatives, antioxidants, chelating agents and buffers), for preventing the action of microorganisms (*e.g*. antimicrobial and antifungal agents, such as parabens, chlorobutanol, phenol, sorbic acid and the like) or for providing the formulation with an edible flavor, *etc.* In some instances, the carrier is an agent that facilitates the delivery of a polypeptide, fusion protein, or polynucleotide to a target cell or tissue. One of skill in the art will recognize that other pharmaceutical carriers are useful in the present invention.

The term "vaccine" refers to a biological composition that, when administered to a subject, has the ability to produce an acquired immunity to a particular pathogen or disease in the subject. Typically, one or more antigens, fragments of antigens, or polynucleotides encoding antigens or fragments of antigens that are associated with the pathogen or disease of interest are administered to the subject. Vaccines can comprise, for example, inactivated or attenuated organisms (*e.g.,* bacteria or viruses), cells, proteins that are expressed from or on cells (*e.g.,* cell surface or other proteins produced by cells (*e.g.,* tumor cells)), proteins that are produced by organisms (*e.g.,* toxins), or portions of organisms (*e.g.,* viral envelope proteins or viral genes encoding various antigens). In some instances, cells are engineered to express proteins such that, when administered as a vaccine, they enhance the ability of a subject to acquire immunity to a particular cell type (*e.g.,* enhance the ability of a subject to acquire immunity to a cancer cell) or to an organism that causes an infectious disease such as a virus, a bacterium, a fungal organism, a protozoan, or a helminth. As used herein, the term "vaccine" includes, but is not limited to, systems and recombinant polynucleotides of the present invention, as well as viral particles, host cells, and pharmaceutical compositions that comprise systems or recombinant polynucleotides of the present invention.

The term "group-specific antigen" or "gag" refers to a protein encoded by a retroviral *gag* gene. *Gag* genes encode the core structural proteins of retroviruses. In human immunodeficiency virus (HIV), the *gag* gene encodes a gag polyprotein precursor (Pr55^{Gag}), which is subsequently proteolytically processed into the p17 matrix protein (MA), the p24 capsid protein (CA), the p7 nucleocapsid protein (NC), the SP1 and SP2 spacer peptides, and the p6 polypeptide that is located at the N-terminus of the gag polyprotein. In the closely related simian immunodeficiency viruses (SIV), the *gag* gene similarly encodes a gag polyprotein precursor, which is subsequently proteolytically processed into proteins having similar molecular weights to their HIV equivalents. Non-limiting examples of HIV and SIV gag protein sequences are set forth under UniProt reference numbers P04591 and P89153, respectively.

### III. Expression Systems and Recombinant Polynucleotides

In one aspect, provided herein is a system for expressing an antigen and a protein having interleukin-10 (IL-10)-like activity in a cell. In some embodiments, the antigen is a portion of an antigenic protein. In some embodiments, the protein having IL-10-like activity is a functional portion of such a protein. In some embodiments, the system comprises a recombinant polynucleotide comprising a nucleic acid sequence encoding the antigen and a recombinant polynucleotide comprising a nucleic acid sequence encoding the protein having IL-10-like activity. In some embodiments, the antigen and the protein having IL-10-like activity are expressed in the same cell.

Non-limiting examples of suitable cells for expressing the antigen and the protein having IL-10-like activity include antigen-presenting cells, muscle cells, tumor cells, fibroblasts, epithelial cells, endothelial cells, and combinations thereof. In some embodiments, the antigen-presenting cell is a dendritic cell.

In some embodiments, the recombinant polynucleotide comprising the nucleic acid sequence encoding the antigen and the recombinant polynucleotide comprising the nucleic acid sequence encoding the protein having IL-10-like activity are separate recombinant polynucleotides. It is understood that in systems of the present invention (*i.e.,* for expressing an antigen and a protein having IL-10-like activity in a cell), the recombinant polynucleotide comprising the nucleic acid sequence encoding the antigen and the recombinant polynucleotide comprising the nucleic acid sequence encoding the protein having IL-10-like activity can be present in the same composition or mixture (*i.e.,* even when present as separate recombinant polynucleotides), or can be present in separate compositions. In some embodiments, the recombinant polynucleotide comprising the nucleic acid sequence encoding the antigen and the recombinant polynucleotide comprising the nucleic acid sequence encoding the protein having IL-10-like activity are the same recombinant polynucleotide.

In some embodiments, the recombinant polynucleotide comprising the nucleic acid sequence encoding the antigen and/or the recombinant polynucleotide comprising the nucleic acid sequence encoding the protein having IL-10-like activity further comprise one or more nucleic acid sequences that encode a regulatory sequence. In some embodiments, the regulatory sequence(s) control the expression of the antigen and/or the protein having IL-10-like activity. In some instances, the regulatory sequence(s) control transcription of the nucleic acid sequence encoding the antigen and/or the nucleic acid sequence encoding the protein having IL-10-like activity. In some instances, the regulatory sequence(s) control translation of the antigen and/or the protein having IL-10-like activity.

Depending on the cell system used, the regulatory sequence(s) may comprise transcription and translation control elements, including promoters, transcription enhancers, transcription terminators, translation initiators, intronic sequences, and the like. Useful promoters can be derived from viruses or any other organism, *e.g.*, prokaryotic or eukaryotic organisms. Promoters may also be inducible (*i.e.,* capable of responding to environmental factors and/or external stimuli that can be artificially controlled). Non-limiting examples of promoters include unmodified and modified bacterial T7 promoters such as the EM7 promoter, the EF1α promoter, RNA polymerase II promoters (*e.g.,* pGAL7 and pTEF1), RNA polymerase III promoters (*e.g.,* RPR-tetO, SNR52, and tRNA-tyr), the SV40 early promoter, mouse mammary tumor virus long terminal repeat (LTR) promoter; adenovirus major late promoter (Ad MLP); a herpes simplex virus (HSV) promoter, a cytomegalovirus (CMV) promoter such as the CMV immediate early promoter region (CMVIE), a rous sarcoma virus (RSV) promoter, a human U6 small nuclear promoter (U6), an enhanced U6 promoter, a human H1 promoter (H1), *etc.* Promoters may contain intronic sequences (*e.g.,* an EF1α intron A sequence).

Suitable polyadenylation sequences and terminators include, but are not limited to, SV40, hGH, BGH, rbGlob SNR52, and RPR polyadenylation and terminator sequences.

Suitable translation initiators include, but are not limited to, Kozak consensus sequences and internal ribosomal entry site (IRES) sequences. Non-limiting examples of useful IRES sequences include an encephalomyocarditis (EMCV) IRES, a human VCIP IRES, a mouse Gtx IRES, a cricket paralysis virus intergenic region, a hepatitis C virus IRES, a poliovirus IRES, a human rhinovirus IRES, a dicistrovirus intergenic IRES, a hepatitis A virus IRES, an enterovirus IRES, an Aichivirus IRES, a cardiovirus IRES, an aphthorivus IRES, a porcine teschovirus IRES, a sapelovirus IRES, a simian virus IRES, an avian encephalomyelitis virus IRES, a foot and mouth disease virus IRES, a cold stress-induced mRNA (Rbm3) IRES, a human NF-kB repressing factor (NRF) IRES, a human apoptotic protease-activating factor-1 (Apaf-1) IRES, an immunoglobulin heavy chain binding protein (BIP) IRES, an aquaporin 4 (AQP-4) IRES, a c-myc33 IRES, and a rat cationic amino-acid transporter-1 (CAT-1) IRES.

Additionally, various primer binding sites may be incorporated into a vector to facilitate vector cloning, sequencing, genotyping, and the like. In some embodiments, a "CAG promoter" is used as the regulatory sequence, which comprises a CMV early enhancer, a chicken beta-actin gene promoter, a first exon of the chicken beta-actin gene, a first intron of the chicken beta-actin gene, and a splice acceptor of the rabbit beta-globin gene. Other suitable promoter, enhancer, terminator, and primer binding sequences will readily be known to one of skill in the art.

In some embodiments, a system of the present invention contains a recombinant polynucleotide that comprises a nucleic acid sequence encoding an antigen, a nucleic acid sequence encoding an IRES (*e.g.,* an EMCV IRES), and a nucleic acid sequence encoding a protein having IL-10-like activity. In some embodiments, the nucleic acid sequence encoding the antigen is located 5' of the nucleic acid sequence encoding the IRES and the nucleic acid sequence encoding the protein having IL-10-like activity is located 3' of the nucleic acid sequence encoding the IRES. Examples are shown in **FIGS. 1B, 1C****,** **1D****,** and 7 and discussed in Examples 1 and 2 below. It is understood that the IRES can be any suitable IRES, including but not limited to IRES sequences described elsewhere herein.

In some embodiments, the recombinant polynucleotide further comprises a nucleic acid sequence encoding a promoter (*e.g.,* an EF1α promoter) and a nucleic acid sequence encoding a polyadenylation signal (*e.g.,* an SV40 virus polyadenylation signal). In some embodiments, the nucleic acid sequence encoding the promoter is located 5' of the nucleic acid sequence encoding the antigen. In some embodiments, the nucleic acid sequence encoding the polyadenylation signal is located 3' of the nucleic acid sequence encoding the protein having IL-10-like activity. Examples are shown in **FIGS. 1B, 1C****,** **1D****,** and 7. It is understood that the promoter and/or polyadenylation signal can be any suitable promoter or polyadenylation signal, including but not limited to those described herein.

In some embodiments, the recombinant polynucleotide further comprises a nucleic acid sequence encoding a Kozak consensus sequence. In some embodiments, the Kozak consensus sequence is located 3' of the nucleic acid sequence encoding the promoter. In some embodiments, the Kozak consensus sequence is located 5' of the nucleic acid sequence encoding the antigen. Examples are shown in **FIGS. 1B** and **1C****.**

In some embodiments, the antigen and the protein having IL-10-like activity are expressed under the control of a single promoter. In some instances, the nucleic acid sequences encoding the antigen and the protein having IL-10-like activity are separated by a nucleic acid sequence encoding an IRES, such that a bicistronic transcript is produced. In some embodiments, the antigen and the protein having IL-10-like activity are expressed under the control of two different promoters.

Alternatively, rather than expressing the antigen and the protein having IL-10-like activity as separate proteins, in some embodiments, the antigen and the protein having IL-10-like activity are expressed as a single protein. In some embodiments, the single protein is a self-cleaving protein, such that cleavage yields the antigen and the protein having IL-10-like activity as separate proteins. As non-limiting examples, the self-cleaving protein can comprise a 2A self-cleaving peptide sequence from foot-and-mouth-disease virus, porcine teschovirus-1 (P2A), thosea asigna virus (T2A), equine rhinitis A virus (E2A), cytoplasmic polyhedrosis virus (BmCPV 2A), or flacherie virus of *B. mori* (BmIFV 2A).

The antigen encoded by a recombinant polynucleotide in a system of the present invention can be any antigen, so long as it produces an immune response against the desired cell type or pathogenic organism. In some embodiments, the antigen is an infectious disease antigen. In other embodiments, the antigen is a tumor-associated antigen (TAA).

In some embodiments, the infectious disease antigen is a bacterial infectious disease antigen. In some embodiments, the infectious disease antigen is a viral infectious disease antigen. In some embodiments, the infectious disease antigen is a fungal infectious disease antigen. In some embodiments, the infectious disease antigen is a protozoal infectious disease antigen. In some embodiments, the infectious disease antigen is a helminthic infectious disease antigen. In some embodiments, the infectious disease antigen is a bacterial, viral, fungal, protozoal, and/or helminthic infectious disease antigen. In some cases, the antigen is from a parasite. Non-limiting examples of suitable viral infectious disease antigens are those derived from simian immunodeficiency virus (SIV), human immunodeficiency virus (HIV), hepatitis C virus, herpes simplex virus 1, herpes simplex virus 2, varicella-zoster virus, human cytomegalovirus, human herpesvirus 6A, human herpesvirus 6B, human herpesvirus 7, human herpesvirus 8, Epstein-Barr virus, a hemorrhagic fever virus, and a combination thereof. As further non-limiting examples, the infectious disease antigen can comprise a retroviral group-specific antigen (gag) protein (*e.g.,* an HIV or SIV gag protein). In some embodiments, a system of the present invention comprises a recombinant polynucleotide comprising the nucleic acid sequence set forth in SEQ ID NO: 1. In some embodiments, the infectious disease antigen is a bacterial infectious disease antigen from *Mycobacterium tuberculosis.*

A tumor-associated antigen (TAA) can be derived from any cancer cell. TAAs include, but are not limited to, products of mutated oncogenes and mutated tumor suppressor genes, overexpressed or aberrantly expressed cellular proteins, antigens that are produced by oncogenic viruses, oncofetal antigens, altered cell surface glycolipids and glycoproteins, antigens that are aberrantly processed in tumor cells for presentation on MHC molecules, and antigens that are tumor cell type-specific.

A TAA can be derived from, for example, a colorectal cancer cell, a colon cancer cell, an anal cancer cell, a liver cancer cell, an ovarian cancer cell, a breast cancer cell, a lung cancer cell, a bladder cancer cell, a thyroid cancer cell, a pleural cancer cell, a pancreatic cancer cell, a cervical cancer cell, a prostate cancer cell, a testicular cancer cell, a bile duct cancer cell, a gastrointestinal carcinoid tumor cell, an esophageal cancer cell, a gall bladder cancer cell, a rectal cancer cell, an appendix cancer cell, a small intestine cancer cell, a stomach (gastric) cancer cell, a renal cancer (*e.g.,* renal cell carcinoma) cell, a central nervous system cancer cell, a skin cancer cell, an oral squamous cell carcinoma cell, a choriocarcinoma cell, a head and neck cancer cell, a bone cancer cell, an osteogenic sarcoma cell, a fibrosarcoma cell, a neuroblastoma cell, a glioma cell, a melanoma cell, a leukemia (*e.g.,* acute lymphocytic leukemia, chronic lymphocytic leukemia, acute myelogenous leukemia, chronic myelogenous leukemia, or hairy cell leukemia) cell, a lymphoma (*e.g.,* non-Hodgkin's lymphoma, Hodgkin's lymphoma, B-cell lymphoma, or Burkitt's lymphoma) cell, a multiple myeloma cell, or any combination thereof.

Non-limiting examples of TAAs that can be encoded by nucleic acid sequences in recombinant polynucleotides of the present invention include the melanoma-associated antigens (MAGEs). MAGE proteins contain a conserved domain that is about 200 amino acids in length and is usually located near the C-terminal end of the protein, although the conserved domain is located closer to the central portion of some MAGE proteins. Human MAGE proteins include MAGEA1, MAGEA2, MAGEA2B, MAGEA3, MAGEA4, MAGEA5, MAGEA6, MAGEA7P, MAGEA8, MAGEA9, MAGEA9B, MAGEA10, MAGEA11, MAGEA12, MAGEA13P, MAGEB1, MAGEB2, MAGEB3, MAGEB4, MAGEB5, MAGEB6, MAGEB10, MAGEB16, MAGEB17, MAGEB18, MAGEC1, MAGEC2, MAGEC3, MAGED1, MAGED2, MAGED3 (also known as "trophin" or "TRO"), MAGED4, MAGED4B, MAGEE1, MAGEE2, MAGEF1, MAGEEG1 (also known as "NSMCE3"), MAGEH1, MAGEL2, and NDN. Additional non-limiting examples of TAAs that are useful for the present invention include NY-ESO-1 and prostate-specific antigen (PSA). Non-limiting examples of MAGEA4-IL-10 systems of the present invention are depicted in **FIGS. 9A** and **10A****.** Non-limiting examples of MAGEA10-IL-10 systems of the present invention are depicted in **FIGS. 9B** and **10B****.** In some embodiments, a system of the present invention comprises a recombinant polynucleotide comprising the nucleic acid sequence set forth in SEQ ID NO:8. In some embodiments, a system of the present invention comprises a recombinant polynucleotide comprising the nucleic acid sequence set forth in SEQ ID NO:9.

In some embodiments, a TAA is one that newly arises in a tumor (*e.g.,* a subject's tumor). Such neoantigens can arise, for example, as a consequence of a tumor-specific mutation. In some embodiments, a TAA is a cell surface protein (*e.g.,* that is normally present on the surface of a cell), or a portion thereof, that is altered as a consequence of a mutation in a gene encoding the cell surface protein.

In some embodiments, the protein having IL-10-like activity is a heterologous protein having IL-10-like activity and/or a viral protein having IL-10-like activity. In some embodiments, the heterologous protein having IL-10-like activity is an IL-10 protein from a host (*e.g.,* a subject or patient who is administered a system of the present invention). In some embodiments, the heterologous protein having IL-10-like activity is a protein that stimulates downstream IL-10 receptor signaling, such as STAT3 or a constitutively active form of STAT3 such as STAT3C. In some instances, the host is a human, non-human primate (*e.g.,* rhesus macaque), or rodent (*e.g.,* mouse or rat). In some instances, the host IL-10 protein is a human IL-10 protein or a rhesus macaque IL-10 protein.

In some embodiments, the protein having IL-10-like activity is one to which a host (*e.g.,* a subject or patient who is administered a system of the present invention) does not have pre-existing immunity.

In some embodiments, the viral protein having IL-10-like activity is a cytomegalovirus (CMV) protein having IL-10-like activity. As non-limiting examples, the CMV protein having IL-10-like activity can be human CMV IL-10 (HCMVIL-10) or rhesus macaque CMV IL-10 (RhCMVIL-10). Additional non-limiting examples include other (*e.g.,* homologous) proteins, such as proteins having IL-10-like activity in SCCMV/AGMCMV, BaCMV, OMCMV, or SMCMV.

In some embodiments, nucleic acid sequences encoding the antigen and/or protein having IL-10-like activity contain intronic sequences. In some instances, the intronic sequences are removed prior to translation of the antigen and/or protein having IL-10-like activity. In some embodiments, the nucleic acid sequences encoding the antigen and/or protein having IL-10-like activity do not contain intronic sequences.

In some embodiments, the recombinant polynucleotide comprising the nucleic acid sequence encoding the antigen and/or the recombinant polynucleotide comprising the nucleic acid sequence encoding the protein having IL-10-like activity further comprise a nucleic acid sequence encoding a tag. Non-limiting examples of tags include StrepTag (StrepII) (8 a.a.); SBP (38 a.a.); biotin carboxyl carrier protein or BCCP (100 a.a.); epitope tags such as FLAG (8 a.a.), 3xFLAG (22 a.a.), and myc (22 a.a.); S-tag (Novagen) (15 a.a.); Xpress (Invitrogen) (25 a.a.); eXact (Bio-Rad) (75 a.a.); HA (9 a.a.); VSV-G (11 a.a.); Protein A/G (280 a.a.); HIS (6-10 a.a.); glutathione s-transferase or GST (218 a.a.); maltose binding protein or MBP (396 a.a.); CBP (28 a.a.); CYD (5 a.a.); HPC (12 a.a.); CBD intein-chitin binding domain (51 a.a.); Trx (Invitrogen) (109 a.a.); NorpA (5 a.a.); and NusA (495 a.a.).

Various nucleic acid sequences that are present in a recombinant polynucleotide used in a system of the present invention, including but not limited to those encoding an antigen, a protein having IL-10-like activity, a regulatory sequence, and/or tag, can all be located in the same open reading frame, different open reading frames, or a combination thereof. In some embodiments, nucleic acid sequences encoding an antigen, a protein having IL-10-like activity, one or more regulatory sequences, and/or a tag (*e.g.,* a FLAG tag) are all located within the same open reading frame.

In some embodiments, the nucleic acid sequence encoding the antigen and/or the nucleic acid sequence encoding the protein having IL-10-like activity are present within an expression cassette. Non-limiting examples of expression cassettes are described in Example 1 below. The nucleic acid sequences encoding the antigen and protein having IL-10-like activity can be present in the same expression cassette, or different expression cassettes. In some embodiments, the nucleic acid sequence encoding the antigen and/or the nucleic acid sequence encoding the protein having IL-10-like activity (*e.g.,* present within one or more expression cassettes) are present within a viral vector.

Non-limiting examples of suitable viral vectors include CMV vectors, adenoviral vectors, adeno-associated virus vectors, lentiviral vectors, herpes virus vectors, alphavirus vectors, retroviral vectors, poxvirus vectors, and vesicular stomatitis virus vectors. In some embodiments, the viral vector does not contain a nucleic acid sequence that encodes a viral protein having IL-10-like activity (*e.g.,* the viral genome or portion thereof which serves as the viral vector does not naturally contain a nucleic acid sequence encoding a viral protein having IL-10-like activity). Non-limiting examples include adenoviral vectors, adeno-associated virus vectors, lentiviral vectors, alphavirus vectors, retroviral vectors, and vesicular stomatitis virus vectors. In addition, certain CMV vectors can be used that do not contain a nucleic acid sequence that encodes a CMV protein having IL-10-like activity. Non-limiting examples include chimpanzee CMV, mouse cytomegalovirus, and Murid herpesvirus 2 (MuHV-2).

In some instances, it is useful to increase tropism for particular target cell or tissue type(s) (*i.e.,* in which the antigen and the protein having IL-10-like activity are expressed). This can be achieved, for example, by introducing mutation(s) into a recombinant polynucleotide (*i.e.,* that comprises a nucleic acid sequence encoding an antigen and/or a protein having IL-10-like activity according to systems of the present invention) that increase tropism for the desired cell or tissue type(s). In some embodiments, a mutation that increases or imparts tropism (*e.g.,* for a target cell or tissue) is introduced into a viral vector (*i.e.,* which contains one or more nucleic acid sequences or expression cassettes encoding the antigen and/or protein having IL-10-like activity). Non-limiting examples of suitable target cells are antigen-presenting cells, muscle cells, tumor cells, fibroblasts, epithelial cells, endothelial cells, and combinations thereof. Suitable antigen-presenting cells include, but are not limited to, dendritic cells, macrophages, and B cells. In particular embodiments, the antigen-presenting cell is a dendritic cell.

When a CMV vector is used, one approach for increasing or imparting target cell or tissue tropism is to introduce mutations into nucleic acid sequences (*i.e.,* within the CMV vector) that result in the modification of a protein, or portion thereof, that may be positioned on the outside of the CMV virion. As a non-limiting example, a CMV envelope protein can be modified by the addition of a blocking domain that decreases or prevents entry of the CMV into a cell, unless the blocking domain is cleaved, *e.g.,* by a protease expressed by a target cell. For example, proteases such as matrix metalloproteases that are expressed by tumor cells of interest can cleave off envelope protein blocking domains, thereby allowing CMV entry only into the tumor cells of interest and increasing tropism for those target cells.

Furthermore, tropism for a target cell or tissue type can be increased or imparted by introducing a nucleic acid sequence (*e.g.,* within the viral vector) that encodes a cellular targeting ligand. To serve as non-limiting examples, a cellular targeting ligand can be an antibody or fragment thereof that recognizes a target cell antigen, a ligand that is recognized by a target cell cognate receptor, a viral capsid protein that recognizes a target cell, or any combination thereof. Non-limiting examples of antibodies and fragments thereof that recognize target cell antigens include antibodies that recognize dendritic cell-specific intercellular adhesion molecule-3-grabbing non-integrin (DC-SIGN; also known as CD209), CD40, CD64, class II MHC molecules, and DEC205 (also known as CD205), all of which are expressed by dendritic cells.

Suitable ligands that are recognized by target cell cognate receptors include, but are not limited to, CD40L (which is also known as CD154 and binds to CD40, which is expressed, *e.g.,* by dendritic cells) and ICAM3 (which has high affinity for DC-SIGN that is expressed by, *e.g.,* APCs such as dendritic cells). In particular embodiments, the cellular targeting ligand is CD154.

Non-limiting examples of viral capsid proteins that are recognized by target cells include Ad16, Ad35, or Ad37 virus fiber proteins (*i.e.,* for targeting dendritic cells) and Sindbis virus envelope glycoproteins (which can also be used for targeting dendritic cells, via DC-SIGN).

Additional mutations that can be introduced into a recombinant polynucleotide (*e.g.,* to increase target tropism) include mutations (*e.g.,* deletions) within the Rh13.1, Rh61/Rh60, Rh157.4, Rh157.5, and/or Rh157.6 genes of RhCMV, or homologs thereof. Human CMV orthologs of Rh13.1, Rh61/Rh60, Rh157.4, Rh157.5, and Rh157.6 include, but are not limited to, RL13, UL36 (also known as viral inhibitor of caspase-8-induced apoptosis (vICA)), UL130, UL128, and UL131, respectively. Rh13.1 and RL13 are involved in, for example, inhibiting fibroblast growth. Rh157.4, Rh157.5, Rh157.6, UL130, UL128, and UL131 encode three components of an alternative entry receptor for non-fibroblast cells (*e.g.,* endothelial and epithelial cells).

In some embodiments, a recombinant polynucleotide in a system of the present invention contains a nucleic acid sequence that encodes a selectable marker. A selectable marker is useful, for example, when a polynucleotide of the present invention is being recombinantly modified, especially when it is desirable to screen a population of modified polynucleotides (*e.g.,* using bacterial, yeast, plant, or animal cells) for those that have incorporated the desired modification(s). Whether the polynucleotide is recombinantly modified within a cell (*e.g.,* a bacterial cell, for example, using Red/ET recombination) or is recombinantly modified and subsequently introduced into a cell (*e.g.,* bacterial, yeast, plant, or animal cell) for screening, the selectable marker can be used to identify which cells contain polynucleotides that have incorporated a modification of interest. Taking antibiotic resistance genes as an example of a selectable marker, treating the cells that contain the recombinant polynucleotides with the antibiotic will identify which cells contain recombinant polynucleotides that have incorporated the antibiotic resistance gene (*i.e.,* the cells that survive after antibiotic treatment must have incorporated the antibiotic resistance gene). If desired, the recombinant polynucleotides can be further screened (*e.g.,* purified from the cells, amplified, and sequenced), in order to verify that the desired modification has been recombinantly introduced into the polynucleotide at the correct position.

When the selectable marker is an antibiotic resistance gene, the gene can confer resistance to Zeocin, ampicillin, tetracycline, or another appropriate antibiotic that will be known to one of skill in the art. In some embodiments, a selectable marker is used that produces a visible phenotype, such as the color of an organism or population of organisms. As a non-limiting example, the phenotype can be examined by growing the organisms (*e.g.,* cells or other organisms that contain the recombinant polynucleotide) and/or their progeny under conditions that result in a phenotype, wherein the phenotype may not be visible under ordinary growth conditions.

In some embodiments, the selectable marker used for identifying cells that contain a polynucleotide containing a modification of interest is a fluorescently tagged protein, a chemical stain, a chemical indicator, or a combination thereof. In other embodiments, the selectable marker responds to a stimulus, a biochemical, or a change in environmental conditions. In some instances, the selectable marker responds to the concentration of a metabolic product, a protein product, a drug, a cellular phenotype of interest, a cellular product of interest, or a combination thereof.

The size of a recombinant polynucleotide in a system of the present invention will depend on the particular antigen(s) and protein(s) having IL-10-like activity that are being encoded, the presence and choice of regulatory sequences and/or expression vectors (*e.g.,* viral vectors), *etc.* Additionally, the size of a recombinant polynucleotide will depend on whether the nucleic acid sequences encoding the antigen and the protein having IL-10-like activity are present within the same recombinant polynucleotide or separate recombinant polynucleotides.

In some embodiments, a recombinant polynucleotide is between about 1 kilobase and about 300 kilobases (*e.g.,* about 1, 1.1, 1.2, 1.3, 1.4, 1.5, 1.6, 1.7. 1.8, 1.9, 2, 3, 4, 5, 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, or 300 kilobases) in length. In some embodiments, the recombinant polynucleotide is greater than about 300 kilobases in length.

In some embodiments, a recombinant polynucleotide present in a system of the present invention is about 1 kilobase to about 300 kilobases, about 1 kilobase to about 250 kilobases, about 1 kilobase to about 200 kilobases, about 1 kilobase to about 150 kilobases, about 1 kilobase to about 100 kilobases, about 1 kilobase to about 50 kilobases, about 1 kilobase to about 40 kilobases, about 1 kilobase to about 30 kilobases, about 1 kilobase to about 20 kilobases, about 1 kilobase to about 10 kilobases, about 50 kilobases to about 300 kilobases, about 50 kilobases to about 250 kilobases, about 50 kilobases to about 200 kilobases, about 50 kilobases to about 150 kilobases, about 50 kilobases to about 100 kilobases, about 100 kilobases to about 300 kilobases, about 100 kilobases to about 250 kilobases, about 100 kilobases to about 200 kilobases, about 100 kilobases to about 150 kilobases, about 150 kilobases to about 300 kilobases, about 150 kilobases to about 250 kilobases, about 150 kilobases to about 200 kilobases, about 200 kilobases to about 300 kilobases, or about 200 kilobases to about 250 kilobases in length.

In another aspect, viral particles are provided. In some embodiments, the viral particle comprises a system (*i.e.,* for expressing an antigen and a protein having interleukin-10 (IL-10)-like activity in a cell) of the present invention. In some embodiments, the viral particle is one that replicates in and/or is released from an infected, transfected, or transformed cell.

In yet another aspect, engineered cells are provided. In some embodiments, the engineered cell comprises a system (*i.e.,* for expressing an antigen and a protein having interleukin-10 (IL-10)-like activity in a cell) of the present invention. In other embodiments, the engineered cell comprises a viral particle of the present invention (*e.g.,* a viral particle comprising a system of the present invention, *i.e.,* for expressing an antigen and a protein having interleukin-10 (IL-10)-like activity in a cell). In some embodiments, the engineered cell has been transfected or transformed (*e.g.,* by one or more recombinant polynucleotides of a system of the present invention). In some embodiments, the engineered cell has been infected (*e.g.,* by a viral particle of the present invention). In some embodiments, a viral particle of the present invention is replicating inside the engineered cell.

The engineered cell may be any cell of interest. The cell can be a cell from any organism, *e.g.*, a bacterial cell, a cell of a single-cell eukaryotic organism, the cell of a multicellular eukaryotic organism, a plant cell (*e.g.,* a rice cell, a wheat cell, a tomato cell, an *Arabidopsis thaliana* cell, a *Zea mays* cell and the like), an animal cell, a cell from an invertebrate animal (*e.g.,* fruit fly, cnidarian, echinoderm, nematode, *etc.*), a cell from a vertebrate animal (*e.g.,* fish, amphibian, reptile, bird, mammal, *etc.*), a cell from a mammal, a cell from a human, a cell from a healthy human, a cell from a human patient, a cell from a cancer patient, *etc.* In some cases, the engineered cell can be transplanted to a subject (*e.g.,* patient). For instance, the cell can be derived from the subject to be treated (*e.g.*, patient).

Furthermore, the cell can be a stem cell (*e.g.*, embryonic stem cell, induced pluripotent stem cell, adult stem cell, mesenchymal stem cell, neural stem cell, hematopoietic stem cell, organ stem cell), a progenitor cell, a somatic cell (*e.g.,* fibroblast, epithelial cell, endothelial cell, heart cell, liver cell, pancreatic cell, muscle cell, skin cell, blood cell, neural cell, immune cell [*e.g.,* antigen-presenting cell such as a dendritic cell]), or any other cell of the body, *e.g.*, human body. The cell can be a primary cell or a primary cell culture derived from a subject, *e.g.,* an animal subject or a human subject, and allowed to grow *in vitro* for a limited number of passages. The cell can be a healthy cell or a diseased cell. In some embodiments, the engineered cell is a fibroblast (*e.g.,* telomerized fibroblast). In particular embodiments, a system of the present invention or viral particle of the present invention is purified from the engineered cell.

### General Recombinant Technology

Basic texts disclosing general methods and techniques in the field of recombinant genetics include Sambrook and Russell, Molecular Cloning, A Laboratory Manual (3rd ed. 2001); Kriegler, Gene Transfer and Expression: A Laboratory Manual (1990); and Ausubel et al., eds., Current Protocols in Molecular Biology (1994)*.*

For nucleic acids, sizes are given in either kilobases (kb) or base pairs (bp). In some instances, these are estimates derived from agarose or acrylamide gel electrophoresis, from sequenced nucleic acids, or from published DNA sequences. For proteins, sizes are given in kilodaltons (kDa) or amino acid residue numbers. In some instances, protein sizes are estimated from gel electrophoresis, from sequenced proteins, from derived amino acid sequences, or from published protein sequences.

Oligonucleotides that are not commercially available can be chemically synthesized, *e.g.*, according to the solid phase phosphoramidite triester method first described by Beaucage & Caruthers, Tetrahedron Lett. 22: 1859-1862 (1981), using an automated synthesizer, as described in Van Devanter et. al., Nucleic Acids Res. 12: 6159-6168 (1984). Purification of oligonucleotides is performed using any art-recognized strategy, *e.g.*, native acrylamide gel electrophoresis or anion-exchange HPLC as described in Pearson & Reanier, J. Chrom. 255: 137-149 (1983).

The sequence of a protein domain or gene of interest can be verified after cloning or subcloning using, *e.g.,* the chain termination method for sequencing double-stranded templates of Wallace et al., Gene 16: 21-26 (1981).

### Coding Sequence for a Protein of Interest

The present invention provides systems comprising recombinant polynucleotides (*e.g.,* isolated recombinant polynucleotides) that comprise nucleic acid sequences encoding antigens and proteins having IL-10-like activity of interest. Rapid progress in the studies of various genomes (*e.g.,* the human genome) has made possible a cloning approach where a human or other model organism DNA sequence database can be searched for any gene segment that has a certain percentage of sequence homology to a known nucleotide sequence, such as one encoding an antigen, protein having IL-10-like activity, *etc.* Any DNA sequence so identified can be subsequently obtained by chemical synthesis and/or a polymerase chain reaction (PCR) technique such as the overlap extension method. For a short sequence, completely *de novo* synthesis may be sufficient; whereas further isolation of full length coding sequence from a human or other model organism cDNA or genomic library using a synthetic probe may be necessary to obtain a larger gene.

Alternatively, a nucleic acid sequence can be isolated from a cDNA or genomic DNA library (*e.g.,* human or rodent cDNA or human, rodent, bacterial, or viral genomic DNA library) using standard cloning techniques such as polymerase chain reaction (PCR), where homology-based primers can often be derived from a known nucleic acid sequence. Commonly used techniques for this purpose are described in standard texts, *e.g.*, Sambrook and Russell, *supra.*

cDNA libraries may be commercially available or can be constructed. The general methods of isolating mRNA, making cDNA by reverse transcription, ligating cDNA into a recombinant vector, transfecting into a recombinant host for propagation, screening, and cloning are well known (*see*, *e.g.*, Gubler and Hoffman, Gene, 25: 263-269 (1983); Ausubel *et al.*, *supra*)*.* Upon obtaining an amplified segment of nucleotide sequence by PCR, the segment can be further used as a probe to isolate the full-length polynucleotide sequence encoding the protein of interest from the cDNA library. A general description of appropriate procedures can be found in Sambrook and Russell, *supra.*

A similar procedure can be followed to obtain a full-length sequence encoding a protein of interest from a human or other model organism genomic library. Genomic libraries are commercially available or can be constructed according to various art-recognized methods. As a non-limiting example, to construct a genomic library, the DNA is first extracted from a tissue of the organism. The DNA is then either mechanically sheared or enzymatically digested to yield fragments of about 12-20 kb in length. The fragments are subsequently separated by gradient centrifugation from polynucleotide fragments of undesired sizes and are inserted in bacteriophage λ vectors. These vectors and phages are packaged *in vitro.* Recombinant phages are analyzed by plaque hybridization as described in Benton and Davis, Science, 196: 180-182 (1977). Colony hybridization is carried out as described by Grunstein et al., Proc. Natl. Acad. Sci. USA, 72: 3961-3965 (1975).

Based on sequence homology, degenerate oligonucleotides can be designed as primer sets and PCR can be performed under suitable conditions (*see*, *e.g.*, White et al., PCR Protocols: Current Methods and Applications, 1993; Griffin and Griffin, PCR Technology, CRC Press Inc. 1994) to amplify a segment of nucleotide sequence from a cDNA or genomic library. Using the amplified segment as a probe, the full-length nucleic acid encoding a protein of interest is obtained.

Upon acquiring a nucleic acid sequence encoding a protein of interest, the coding sequence can be further modified by a number of well-known techniques such as restriction endonuclease digestion, PCR, and PCR-related methods to generate coding sequences, including mutants and variants derived from the wild-type protein. The polynucleotide sequence encoding the desired polypeptide can then be subcloned into a vector, for instance, an expression vector, so that a recombinant polypeptide can be produced from the resulting construct. Further modifications to the coding sequence, *e.g.*, nucleotide substitutions, may be subsequently made to alter the characteristics of the polypeptide.

A variety of mutation-generating protocols are established and described in the art, and can be readily used to modify a polynucleotide sequence encoding a protein of interest. *See, e.g.*, Zhang et al., Proc. Natl. Acad. Sci. USA, 94: 4504-4509 (1997); and Stemmer, Nature, 370: 389-391 (1994). The procedures can be used separately or in combination to produce variants of a set of nucleic acids, and hence variants of encoded polypeptides. Kits for mutagenesis, library construction, and other diversity-generating methods are commercially available.

Mutational methods of generating diversity include, for example, site-directed mutagenesis (Botstein and Shortle, Science, 229: 1193-1201 (1985)), mutagenesis using uracil-containing templates (Kunkel, Proc. Natl. Acad. Sci. USA, 82: 488-492 (1985)), oligonucleotide-directed mutagenesis (Zoller and Smith, Nucl. Acids Res., 10: 6487-6500 (1982)), phosphorothioate-modified DNA mutagenesis (Taylor et al., Nucl. Acids Res., 13: 8749-8787 (1985)), and mutagenesis using gapped duplex DNA (Kramer et al., Nucl. Acids Res., 12: 9441-9456 (1984)).

Other possible methods for generating mutations include point mismatch repair (Kramer et al., Cell, 38: 879-887 (1984)), mutagenesis using repair-deficient host strains (Carter et al., Nucl. Acids Res., 13: 4431-4443 (1985)), deletion mutagenesis (Eghtedarzadeh and Henikoff, Nucl. Acids Res., 14: 5115 (1986)), restriction-selection and restriction-purification (Wells et al., Phil. Trans. R. Soc. Lond. A, 317: 415-423 (1986)), mutagenesis by total gene synthesis (Nambiar et al., Science, 223: 1299-1301 (1984)), double-strand break repair (Mandecki, Proc. Natl. Acad. Sci. USA, 83: 7177-7181 (1986)), mutagenesis by polynucleotide chain termination methods (U.S. Patent No. 5,965,408), and error-prone PCR (Leung et al., Biotechniques, 1: 11-15 (1989)).

In some embodiments, a nucleic acid sequence encoding a protein of interest (*e.g.,* an antigen or a protein having IL-10-like activity) is codon optimized. The term "codon optimization" refers to altering a nucleic acid sequence, without changing the encoded amino acid sequence, in such a way that codon bias (*i.e.,* the preferential use of particular codons that can vary between species) is reduced or rebalanced. In some embodiments, codon optimization increases translational efficiency (e*.g.,* of an antigen or protein having IL-10-like activity). As a non-limiting example, leucine is encoded by six different codons, some of which are rarely used. By rebalancing codon usage (*e.g.,* within a reading frame), preferred leucine codons can be selected over rarely used codons. The nucleic acid sequence encoding the protein (*e.g.,* antigen or protein having IL-10-like activity) of interest is altered such that the rarely used codons are converted to preferred codons.

Rare codons can be defined, for example, by using a codon usage table derived from the sequenced genome of a host species, *i.e.,* the species in which the protein (*e.g.,* an antigen or protein having IL-10-like activity) will be expressed. See, *e.g.,* the codon usage table obtained from Kazusa DNA Research Institute, Japan (www.kazusa.or.jp/codon/) used in conjunction with software, *e.g.,* "Gene Designer 2.0" software, from DNA 2.0 (www.dna20.com/) at a cut-off threshold of 15%.

Codon optimization may also be employed to modulate GC content, *e.g.,* to increase mRNA stability or reduce secondary structure; or otherwise minimize codons that may result in stretches of sequence that impair expression of the protein of interest (*e.g.,* an antigen or protein having IL-10-like activity).

### IV. Methods for Inducing an Immune Response and Treating Disease

In another aspect, pharmaceutical compositions are provided herein. In some embodiments, the pharmaceutical composition comprises: (a) a system (*i.e.,* for expressing an antigen and a protein having interleukin-10 (IL-10)-like activity in a cell) of the present invention, a viral particle of the present invention (*e.g.,* a viral particle comprising a system of the present invention), and/or an engineered cell of the present invention (*e.g.,* an engineered cell comprising a system of the present invention and/or a viral particle of the present invention); and (b) a pharmaceutically acceptable carrier.

In some embodiments, a recombinant polynucleotide comprising a nucleic acid sequence encoding an antigen and a recombinant polynucleotide comprising a nucleic acid sequence encoding a protein having IL-10-like activity are contained in separate pharmaceutical compositions. In some embodiments, a recombinant polynucleotide comprising a nucleic acid sequence encoding an antigen and a recombinant polynucleotide comprising a nucleic acid sequence encoding a protein having IL-10-like activity are contained in the same pharmaceutical composition, but the two recombinant polynucleotides are separate polynucleotides. In some embodiments, the recombinant polynucleotide comprising the nucleic acid sequence encoding an antigen and the recombinant polynucleotide comprising the nucleic acid sequence encoding a protein having IL-10-like activity are present within the same recombinant polynucleotide.

Similarly, when the pharmaceutical composition comprises a viral particle of the present invention, the recombinant polynucleotide comprising the nucleic acid sequence encoding an antigen and the recombinant polynucleotide comprising the nucleic acid sequence encoding a protein having IL-10-like activity can be present within separate viral particles, which can in turn be present in separate pharmaceutical compositions or the same pharmaceutical composition, or can be present within the same viral particle. Also, when the pharmaceutical composition comprises an engineered cell of the present invention, the recombinant polynucleotide comprising the nucleic acid sequence encoding an antigen and the recombinant polynucleotide comprising the nucleic acid sequence encoding a protein having IL-10-like activity can be present within separate engineered cells, which can in turn be present in separate pharmaceutical compositions or the same pharmaceutical composition, or can be present within the same engineered cell.

In another aspect, methods for inducing an immune response against an antigen (*e.g.,* in a subject) are provided herein. In some embodiments, the method comprises administering a system (*i.e.,* for expressing an antigen and a protein having interleukin-10 (IL-10)-like activity in a cell) of the present invention (*e.g.,* a therapeutically effective amount thereof) to a subject (*e.g.,* a subject in need thereof). In some embodiments, the method comprises administering a viral particle (*e.g.,* a therapeutically effective amount thereof) of the present invention to a subject. In some embodiments, the method comprises administering an engineered cell (*e.g.,* a therapeutically effective amount thereof) of the present invention to a subject. In some embodiments, the method comprises administering a pharmaceutical composition (*e.g.,* a therapeutically effective amount thereof) of the present invention to a subject.

The antigen against which an immune response is generated (*e.g.,* in a subject) will depend on the particular disease(s) for which prophylactic and/or therapeutic benefit is sought. In some embodiments, the antigen is an infectious disease antigen. In other embodiments, the antigen is a tumor-associated antigen. In some embodiments, the antigen is both an infectious disease and a tumor-associated antigen. In particular embodiments, inducing an immune response against an infectious disease antigen prevents or treats another disease that is caused or exacerbated by the infectious disease. As a non-limiting example, inducing an immune response against an infectious disease antigen can prevent and/or treat a cancer that is caused or exacerbated by the particular infectious disease associated with that antigen. As another non-limiting example, inducing an immune response against an infectious disease that causes immunodeficiency (*e.g.,* in a subject), such as HIV or SIV, can prevent and/or treat diseases that result from the immunodeficiency.

In some embodiments, an immune response (*e.g.,* a desired, intended, or protective immune response, *e.g.,* in a subject) is induced against a bacterial antigen (*e.g.,* a bacterial infectious disease antigen). In some embodiments, an immune response is induced against a viral antigen (*e.g.,* a viral infectious disease antigen). In some embodiments, an immune response is induced against a fungal antigen (*e.g.,* a fungal infectious disease antigen). In some embodiments, an immune response is induced against a protozoal antigen (*e.g.,* a protozoal infectious disease antigen). In some embodiments, an immune response is induced against a helminthic antigen (*e.g.,* a helminthic infectious disease antigen). In some embodiments, the antigen is a bacterial, viral, fungal, protozoal, and/or helminthic antigen. In particular embodiments, the antigen is derived from a parasite.

In some instances, the antigen (*e.g.,* infectious disease antigen) is from simian immunodeficiency virus (SIV), human immunodeficiency virus (HIV), hepatitis C virus, herpes simplex virus 1, herpes simplex virus 2, varicella-zoster virus, human cytomegalovirus, human herpesvirus 6A, human herpesvirus 6B, human herpesvirus 7, human herpesvirus 8, Epstein-Barr virus, a hemorrhagic fever virus, *Mycobacterium tuberculosis,* or a combination thereof. Non-limiting examples of suitable infectious disease antigens include SIV and HIV gag proteins.

Compositions and methods of the present invention are useful for inducing a response (*e.g.,* a desired, intended, or protective immune response) against any number of tumor-associated antigens (TAAs). The TAA can be derived from, for example, a colorectal cancer cell, a colon cancer cell, an anal cancer cell, a liver cancer cell, an ovarian cancer cell, a breast cancer cell, a lung cancer cell, a bladder cancer cell, a thyroid cancer cell, a pleural cancer cell, a pancreatic cancer cell, a cervical cancer cell, a prostate cancer cell, a testicular cancer cell, a bile duct cancer cell, a gastrointestinal carcinoid tumor cell, an esophageal cancer cell, a gall bladder cancer cell, a rectal cancer cell, an appendix cancer cell, a small intestine cancer cell, a stomach (gastric) cancer cell, a renal cancer (*e.g.,* renal cell carcinoma) cell, a central nervous system cancer cell, a skin cancer cell, an oral squamous cell carcinoma cell, a choriocarcinoma cell, a head and neck cancer cell, a bone cancer cell, an osteogenic sarcoma cell, a fibrosarcoma cell, a neuroblastoma cell, a glioma cell, a melanoma cell, a leukemia (*e.g.,* acute lymphocytic leukemia, chronic lymphocytic leukemia, acute myelogenous leukemia, chronic myelogenous leukemia, or hairy cell leukemia) cell, a lymphoma (*e.g.,* non-Hodgkin's lymphoma, Hodgkin's lymphoma, B-cell lymphoma, or Burkitt's lymphoma) cell, a multiple myeloma cell, or any combination thereof. In particular embodiments, the TAA is derived from an ovarian cancer cell, a melanoma cell, a prostate cancer cell, or a combination thereof.

Non-limiting examples of TAAs to which an immune response (*e.g.,* a desired, intended, or protective immune response) can be induced (*e.g.,* using compositions and methods of the present invention) include the melanoma-associated antigens (MAGEs). MAGE proteins contain a conserved domain that is about 200 amino acids in length and is usually located near the C-terminal end of the protein, although the conserved domain is located closer to the central portion of some MAGE proteins. Human MAGE proteins include MAGEA1, MAGEA2, MAGEA2B, MAGEA3, MAGEA4, MAGEA5, MAGEA6, MAGEA7P, MAGEA8, MAGEA9, MAGEA9B, MAGEA10, MAGEA11, MAGEA12, MAGEA13P, MAGEB1, MAGEB2, MAGEB3, MAGEB4, MAGEB5, MAGEB6, MAGEB10, MAGEB16, MAGEB17, MAGEB18, MAGEC1, MAGEC2, MAGEC3, MAGED1, MAGED2, MAGED3 (also known as "trophin" or "TRO"), MAGED4, MAGED4B, MAGEE1, MAGEE2, MAGEF1, MAGEEG1 (also known as "NSMCE3"), MAGEH1, MAGEL2, and NDN. Additional non-limiting examples of TAAs that are useful for the present invention include NY-ESO-1 and prostate-specific antigen (PSA).

In some embodiments, an immune response (e*.g.,* a desired, intended, or protective immune response) is induced against an antigen selected from the group consisting of MAGEA4, MAGEA10, NY-ESO-1, PSA, and a combination thereof.

In some embodiments, a TAA is one that newly arises in a tumor (*e.g.,* a subject's tumor). Such neoantigens can arise, for example, as a consequence of a tumor-specific mutation. In some embodiments, a TAA is a cell surface protein (*e.g.,* that is normally present on the surface of a cell), or a portion thereof, that is altered as a consequence of a mutation in a gene encoding the cell surface protein.

In some embodiments, the immune response (*e.g.,* a desired, intended, or protective immune response) that is induced (*e.g.,* in a subject) using a system, viral particle, engineered cell, or pharmaceutical composition of the present invention is greater than the immune response that is induced using a system, viral particle, engineered cell, or pharmaceutical composition that does not express the antigen and the protein having IL-10-like activity in a cell (*e.g.,* the same cell). In some embodiments, the desired, intended, or protective immune response is equivalent or reduced but an undesirable or unintended response is less than the response that is induced using a system, viral particle, engineered cell, or pharmaceutical composition that does not express the antigen and the protein having IL-10-like activity in a cell (*e.g.,* the same cell). In some embodiments, systems, compositions, and methods of the present invention generate a collection of immune responses that is more therapeutically effective due to reduction in undesirable and/or unintended immune responses.

In some embodiments, inducing an immune response (*e.g.,* desired, intended, or protective immune response, *e.g.*, in a subject) comprises generating antibodies that recognize an antigen (*e.g.,* an antigen encoded by a recombinant polynucleotide present as part of a system of the present invention). In some instances, antibodies are generated that recognize an infectious disease antigen. In some instances, antibodies are generated that recognize a TAA. In some instances, antibodies are generated that recognize an infectious disease antigen and/or a TAA.

In some embodiments, inducing an immune response comprises increasing the expression and/or activity of an immunostimulatory protein (*e.g.,* in a subject). In some embodiments, inducing an immune response comprises increasing the expression and/or activity of a cytokine (*e.g.*, MIP-1alpha, MIP-1beta). In some instances, inducing an immune response comprises increasing the expression and/or activity of an interleukin (*e.g.,* IL-1beta, IL-4, IL-6, IL-8, IL-10, IL-13, IL-17, IL-21) and/or TNF-alpha.

In some embodiments, inducing an immune response comprises increasing the number and/or activation of one or more T cells (*e.g.,* in a subject), as compared to when a system, viral particle, engineered cell, or pharmaceutical composition that does not express the antigen and the protein having IL-10-like activity in a cell (*e.g.,* the same cell) is used. In some embodiments, the number and/or activation of MHC class Ib-restricted T cells is increased. In some embodiments, the MHC class Ib molecule is a human HLA-E, HLA-F, and/or HLA-G molecule. In some embodiments, the MHC class Ib molecule is a macaque Mamu-E, Mamu-F, and/or Mamu-AG molecule. In some embodiments, the MHC class Ib molecule is a mouse Qa-1 molecule.

In some embodiments, the immune response that is induced (*e.g.,* in a subject) comprises a lower CD8⁺ T cell response as compared to the CD8⁺ T cell response that is induced using a system, viral particle, engineered cell, or pharmaceutical composition that does not express the antigen and the protein having IL-10-like activity in a cell (*e.g.,* the same cell).

In some embodiments, the immune response that is induced (*e.g.,* in a subject) comprises a higher CD4⁺ T cell response as compared to the CD4⁺ T cell response that is induced using a system, viral particle, engineered cell, or pharmaceutical composition that does not express the antigen and the protein having IL-10-like activity in a cell (*e.g.,* the same cell).

In some embodiments, the protective immune response that is induced (*e.g.,* in a subject) comprises reduced or limited expression and/or activity of a cytokine (*e.g.*, IP-10, MCP-1) that promotes harmful inflammation as compared to the immune response that is induced using a system, viral particle, engineered cell, or pharmaceutical composition that does not express the antigen and the protein having IL-10-like activity in a cell (*e.g.,* the same cell).

In yet another aspect, methods for preventing and/or treating diseases (*e.g.,* in a subject) are provided herein. In some embodiments, the method comprises administering a system (*i.e.,* for expressing an antigen and a protein having interleukin-10 (IL-10)-like activity in a cell) of the present invention (*e.g.,* a therapeutically effective amount thereof) to a subject (*e.g.,* a subject in need thereof). In some embodiments, the method comprises administering a viral particle (*e.g.,* a therapeutically effective amount thereof) of the present invention to a subject. In some embodiments, the method comprises administering an engineered cell (*e.g.,* a therapeutically effective amount thereof) of the present invention to a subject. In some embodiments, the method comprises administering a pharmaceutical composition (*e.g.,* a therapeutically effective amount thereof) of the present invention to a subject.

Any number of diseases can be prevented and/or treated using compositions and/or methods of the present invention. In some embodiments, an infectious disease is prevented and/or treated. In other embodiments, cancer is prevented and/or treated. In some embodiments, an infectious disease and/or cancer are treated.

In some embodiments, a bacterial infectious disease is prevented and/or treated. In some embodiments, a viral infectious disease is prevented and/or treated. In some embodiments, a fungal infectious disease is prevented and/or treated. In some embodiments, a protozoal infectious disease is prevented and/or treated. In some embodiments, a helminthic infectious disease is prevented and/or treated. In some embodiments, a bacterial, viral, fungal, protozoal, and/or helminthic infectious disease is prevented and/or treated. In particular embodiments, the infectious disease is caused by a parasite. Non-limiting examples of viral infectious diseases that can be prevented and/or treated by the compositions and methods of the present invention include those caused by simian immunodeficiency virus (SIV), human immunodeficiency virus (HIV), hepatitis C virus, herpes simplex virus 1, herpes simplex virus 2, varicella-zoster virus, human cytomegalovirus, human herpesvirus 6A, human herpesvirus 6B, human herpesvirus 7, human herpesvirus 8, Epstein-Barr virus, and hemorrhagic fever virus.

Non-limiting examples of cancers that can be prevented and/or treated using compositions and methods of the present invention include colorectal cancer, colon cancer, anal cancer, liver cancer, ovarian cancer, breast cancer, lung cancer, bladder cancer, thyroid cancer, pleural cancer, pancreatic cancer, cervical cancer, prostate cancer, testicular cancer, bile duct cancer, gastrointestinal carcinoid tumors, esophageal cancer, gall bladder cancer, rectal cancer, appendix cancer, small intestine cancer, stomach (gastric) cancer, renal cancer (*e.g.,* renal cell carcinoma), cancer of the central nervous system, skin cancer, oral squamous cell carcinoma, choriocarcinomas, head and neck cancers, bone cancer, osteogenic sarcomas, fibrosarcoma, neuroblastoma, glioma, melanoma, leukemia (*e.g.,* acute lymphocytic leukemia, chronic lymphocytic leukemia, acute myelogenous leukemia, chronic myelogenous leukemia, or hairy cell leukemia), lymphoma (*e.g.,* non-Hodgkin's lymphoma, Hodgkin's lymphoma, B-cell lymphoma, or Burkitt's lymphoma), and multiple myeloma. In some embodiments, the cancer is melanoma, ovarian cancer, or prostate cancer.

Compositions and methods of the present invention can be used to treat cancer at any stage. In some embodiments, the cancer is an advanced cancer. In some embodiments, the cancer is a metastatic cancer. In some embodiments, the cancer is a drug-resistant cancer.

In some embodiments, the subject is treated (*e.g.,* an immune response against an antigen is induced) before any symptoms or sequelae of the disease (*e.g.,* infectious disease, or cancer) develop. In other embodiments, the subject has signs, symptoms, or sequelae of the disease. In some instances, treatment results in a reduction or elimination of the signs, symptoms, or sequelae of the disease.

In some embodiments, prevention and/or treatment includes administering compositions of the present invention directly to a subject. As a non-limiting example, pharmaceutical compositions of the present invention (*e.g.,* comprising a system, viral particle, and/or engineered cell of the present invention and a pharmaceutically acceptable carrier) can be delivered directly to a subject (*e.g.,* by local injection or systemic administration). In some instances, intratumoral injection is used. In other embodiments, the compositions of the present invention are delivered to an engineered cell or population of engineered cells, and then the engineered cell or population of engineered cells is administered or transplanted into the subject. The engineered cell or population of engineered cells can be administered or transplanted with a pharmaceutically acceptable carrier. In certain instances, progeny of the engineered cell or population of engineered cells are transplanted into the subject. Procedures for transplantation and administration will be known to one of skill in the art.

Compositions of the present invention may be administered as a single dose or as multiple doses, for example two doses administered at an interval of about one month, about two months, about three months, about six months, or about 12 months. Other suitable dosage schedules can be determined by a medical practitioner.

In some embodiments, a dose comprises between about 10⁴ and about 10¹³ plaque-forming units (pfu) (*e.g.,* about 10⁴, 10⁵, 10⁶, 10⁷, 10⁸, 10⁹, 10¹⁰, 10¹¹, 10¹², or 10¹³ pfu). In some embodiments, a dose comprises about 10⁴ pfu to about 10⁵ pfu, about 10⁴ pfu to about 10⁶ pfu, about 10⁴ pfu to about 10⁷ pfu, about 10⁴ pfu to about 10⁸ pfu, about 10⁴ pfu to about 10⁹ pfu, about 10⁴ pfu to about 10¹⁰ pfu, about 10⁴ pfu to about 10¹¹ pfu, about 10⁴ pfu to about 10¹² pfu, about 10⁴ pfu to about 10¹³ pfu, about 10⁵ pfu to about 10⁶ pfu, about 10⁵ pfu to about 10⁷ pfu, about 10⁵ pfu to about 10⁸ pfu, about 10⁵ pfu to about 10⁹ pfu, about 10⁵ pfu to about 10¹⁰ pfu, about 10⁵ pfu to about 10¹¹ pfu, about 10⁵ pfu to about 10¹² pfu, about 10⁵ pfu to about 10¹³ pfu, about 10⁶ pfu to about 10⁷ pfu, about 10⁶ pfu to about 10⁸ pfu, about 10⁶ pfu to about 10⁹ pfu, about 10⁶ pfu to about 10¹⁰ pfu, about 10⁶ pfu to about 10¹¹ pfu, about 10⁶ pfu to about 10¹² pfu, about 10⁶ pfu to about 10¹³ pfu, about 10⁷ pfu to about 10⁸ pfu, about 10⁷ pfu to about 10⁹ pfu, about 10⁷ pfu to about 10¹⁰ pfu, about 10⁷ pfu to about 10¹¹ pfu, about 10⁷ pfu to about 10¹² pfu, about 10⁷ pfu to about 10¹³ pfu, about 10⁸ pfu to about 10⁹ pfu, about 10⁸ pfu to about 10¹⁰ pfu, about 10⁸ pfu to about 10¹¹ pfu, about 10⁸ pfu to about 10¹² pfu, about 10⁸ pfu to about 10¹³ pfu, about 10⁹ pfu to about 10¹⁰ pfu, about 10⁹ pfu to about 10¹¹ pfu, about 10⁹ pfu to about 10¹² pfu, about 10⁹ pfu to about 10¹³ pfu, about 10¹⁰ pfu to about 10¹¹ pfu, about 10¹⁰ pfu to about 10¹² pfu, about 10¹⁰ pfu to about 10¹³ pfu, about 10¹¹ pfu to about 10¹² pfu, about 10¹¹ pfu to about 10¹³ pfu, or about 10¹² pfu to about 10¹³ pfu. In particular embodiments, a dose comprises between about 10⁴ and about 2 x 10¹¹ pfu. The dose will vary depending on factors such as the particular antigen to which an immune response is being induced, characteristics of the recombinant polynucleotide encoding the antigen, immune status of the subject, age of the subject, weight of the subject, concomitant medical conditions, route of administration, *etc.*

In some embodiments, additional compounds or medications can be co-administered to the subject. Such compounds or medications can be co-administered for the purpose of alleviating signs or symptoms of the disease being treated, reducing side effects caused by induction of the immune response, *etc.*

In some embodiments, an exogenous protein having IL-10-like activity is administered to the subject. In some embodiments, the exogenous protein having IL-10-like activity is a recombinant protein (*e.g.,* a recombinant human IL-10 protein or recombinant STAT3/STAT3C protein) that is administered to the subject. The exogenous protein having IL-10-like activity can be administered before, at the same time as, or after administration of a system, viral particle, or engineered cell of the present invention,

In some embodiments, a sample (*e.g.,* a test sample or a reference sample) is obtained from a subject (*e.g.,* a subject in whom an immune response against an antigen is to be induced or a subject in whom a disease is to be prevented and/or treated). In particular embodiments, the sample is obtained for the purposes of determining the presence or level of one or biomarkers. Determining the presence or level of biomarkers(s) can be used to, as non-limiting examples, determine response to treatment or to select an appropriate composition or method for the prevention or treatment of a disease.

In some embodiments, a test sample is obtained from the subject. The test sample can be obtained before and/or after a composition of the present invention is administered to the subject. Non-limiting examples of suitable samples include blood, serum, plasma, cerebrospinal fluid (CSF), tissue, saliva, urine, and combinations thereof. In some instances, the sample comprises normal tissue. In other instances, the sample comprises abnormal tissue (*e.g.,* cancer tissue). The sample can also be made up of a combination of normal and abnormal cells (*e.g.,* cancer cells). In some instances, the sample is obtained as a biopsy sample or fine needle aspirate (FNA) sample. In some embodiments, the tissue comprises one or more types of immune cells.

In some embodiments, a reference sample is obtained. The reference sample can be obtained, for example, from the subject (*i.e.,* the subject being treated or in whom an immune response is being induced). The reference sample can be also be obtained from a different subject and/or a population of subjects. In some instances, the reference sample is either obtained from the subject, a different subject, or a population of subjects before and/or after a composition of the present invention is administered to the subject, and comprises normal tissue. In other instances, the reference sample comprises abnormal tissue and is obtained from the subject and/or from a different subject or a population of subjects.

In some embodiments, the level of one or more biomarkers is determined in the test sample and/or reference sample. Non-limiting examples of suitable biomarkers include antigens, antibodies against antigens, immune cell numbers and/or activation levels, capacity for immune cell responses to an antigen after *in vitro* stimulation, immunostimulatory proteins, cytokines, interleukins, tumor necrosis factors, interferons, and other molecules that play roles in modulating immune responses. Further non-limiting examples of suitable biomarkers include C-reactive protein, interferon-gamma, IL-1beta, IL-4, IL-5, IL-6, IL-8, IL-10, IP-10, IL-12, IL-15, IL-13, IL-17, IL-21, MCP-1, MIP-lalpha, MIP-1beta, transforming growth factor-beta, tumor necrosis factor-alpha, and combinations thereof.

Typically, the level of a biomarker in a sample (*e.g.,* test sample) is compared to the level of the biomarker in a reference sample. Depending on the biomarker, an increase or a decrease relative to a normal control or reference sample can be indicative of the presence of a disease, or response to treatment for a disease. In some embodiments, an increased level of a biomarker in a sample (*e.g.,* test sample), and hence the presence of a disease (*e.g.,* an infectious disease or cancer), increased risk of the disease, or response to treatment is determined when the biomarker levels are at least, *e.g.*, about 1.1-fold, 1.2-fold, 1.3-fold, 1.4-fold, 1.5-fold, 1.6-fold, 1.7-fold, 1.8-fold, 1.9-fold, 2-fold, 3-fold, 4-fold, 5-fold, 6-fold, 7-fold, 8-fold, 9-fold, 10-fold, 11-fold, 12-fold, 13-fold, 14-fold, 15-fold, 16-fold, 17-fold, 18-fold, 19-fold, or 20-fold higher in comparison to a negative control. In other embodiments, a decreased level of a biomarker in the test sample, and hence the presence of the disease, increased risk of the disease, or response to treatment is determined when the biomarker levels are at least, *e.g.*, about 1.1-fold, 1.2-fold, 1.3-fold, 1.4-fold, 1.5-fold, 1.6-fold, 1.7-fold, 1.8-fold, 1.9-fold, 2-fold, 3-fold, 4-fold, 5-fold, 6-fold, 7-fold, 8-fold, 9-fold, 10-fold, 11-fold, 12-fold, 13-fold, 14-fold, 15-fold, 16-fold, 17-fold, 18-fold, 19-fold, or 20-fold lower in comparison to a negative control.

The biomarker levels can be detected using any method known in the art, including the use of antibodies specific for the biomarkers. Exemplary methods include, without limitation, PCR, Western Blot, dot blot, enzyme-linked immunosorbent assay (ELISA), radioimmunoassay (RIA), immunoprecipitation, immunofluorescence, FACS analysis, electrochemiluminescence, and multiplex bead assays (e.g., using Luminex or fluorescent microbeads). In some instances, nucleic acid sequencing is employed.

In certain embodiments, the presence of decreased or increased levels of one or more biomarkers is indicated by a detectable signal *(e.g.,* a blot, fluorescence, chemiluminescence, color, radioactivity) in an immunoassay or PCR reaction (e.g., quantitative PCR). This detectable signal can be compared to the signal from a reference sample or to a threshold value.

In some embodiments, the results of the biomarker level determinations are recorded in a tangible medium. For example, the results of diagnostic assays (e.g., the observation of the presence or decreased or increased presence of one or more biomarkers) and the diagnosis of whether or not there is an increased risk or the presence of a disease *(e.g.,* an infectious disease or cancer) or whether or not a subject is responding to treatment can be recorded, *e.g.,* on paper or on electronic media *(e.g.,* audio tape, a computer disk, a CD, a flash drive, *etc.).*

In other embodiments, the methods further comprise the step of providing the diagnosis to the patient (*i.e.,* the subject) and/or the results of treatment.

### V. Kits

In another aspect, kits are provided herein. In some embodiments, the kit comprises a system *(i.e.,* for expressing an antigen and a protein having interleukin-10 (IL-10)-like activity in a cell) of the present invention, a viral particle of the present invention (e.g., a viral particle comprising a system of the present invention), an engineered cell of the present invention (e.g., an engineered cell comprising a system of the present invention and/or a viral particle of the present invention) and/or a pharmaceutical composition of the present invention (e.g., comprising a system of the present invention, viral particle of the present invention, and/or an engineered cell of the present invention and a pharmaceutically acceptable carrier). In some embodiments, the kit is for inducing an immune response against an antigen *(e.g.,* in a subject). In other embodiments, the kit is for preventing or treating a disease. In particular embodiments, the kit is for preventing or treating an infectious disease described herein and/or a cancer described herein.

Kits of the present invention can be packaged in a way that allows for safe or convenient storage or use *(e.g.,* in a box or other container having a lid). Typically, kits of the present invention include one or more containers, each container storing a particular kit component such as a reagent, a control sample, and so on. The choice of container will depend on the particular form of its contents, *e.g.,* a kit component that is in liquid form, powder form, *etc.* Furthermore, containers can be made of materials that are designed to maximize the shelf-life of the kit components. As a non-limiting example, kit components that are light-sensitive can be stored in containers that are opaque.

In some embodiments, the kit contains one or more reagents. In some instances, the reagents are useful for transfecting or transforming an engineered cell with a recombinant polynucleotide that is part of a system of the present invention. The kit may also comprise one or more reagents useful for delivering recombinant polynucleotides (i.e., that are found in systems of the present invention) or viral particles of the present invention into a cell and/or for administering a pharmaceutical composition of the present invention to a subject. In yet other embodiments, the kit further comprises instructions for use.

### VI. Examples

The present invention will be described in greater detail by way of specific examples. The following examples are offered for illustrative purposes only, and are not intended to limit the invention in any manner. Those of skill in the art will readily recognize a variety of noncritical parameters which can be changed or modified to yield essentially the same results.

### Example 1. IL-10-Containing Vaccine Construction

This example describes the construction of one control vaccine and three IL-10-containing vaccine vectors for immunization against a model antigen, SIV Gag.

The SIV Gag codon-optimized open reading frame was juxtaposed, in-frame and upstream, to a FLAG tag sequence to allow easy monitoring of protein expression. This open reading frame (ORF) was placed downstream of a mammalian EF1α promoter and upstream of an SV40 virus polyadenylation signal. This promoter-ORF-polyadenylation signal combination was the control expression cassette (FIG. 1A) used in the control vaccine. Three additional IL-10-containing versions of the construct were then created by insertion between the ORF and polyadenylation signal of an internal ribosomal entry sequence (IRES) and one of three sequences: (i) the rhesus cytomegalovirus (RhCMV) UL111A open reading frame encoding RhCMV viral IL-10 **(****FIG. 1B****),** (ii) the RhCMV UL111A genomic region, which encodes an intron that is removed in mRNA processing to allow RhCMV viral IL-10 expression **(****FIG. 1C****),** and (iii) the rhesus macaque genomic open reading frame encoding rhesus macaque IL-10 **(****FIG. 1D** and **FIG. 7****;** SEQ ID NO:1).

These expression cassettes were then inserted into adenoviral vectors using the pAdEasy kit from Agilent. Briefly, the expression cassettes were cloned into the multicloning site of pShuttle, a plasmid from Agilent containing DNA sequences from adenovirus type 5. The resulting shuttle vectors containing transgene sequences were then transformed into recombination-competent bacteria along with the plasmid pAdEasy-1, which contains the genome of an E1/E3-deficient serotype-5 adenovirus. The resulting recombinant plasmids contained complete vaccine-vector genomes flanked by *Pac*I restriction enzyme sites. The vaccine-vector genomes were released by *Pac*I digestion and recovered as replicating adenovectors by transfection into C7 cells. The resulting adenovectors were expanded by serial passage on 293 cells. Purification and concentration of the vectors was accomplished using a CsCl equilibrium gradient.

Using the same methods described above, additional vectors were made containing only rhesus macaque IL-10 or only rhesus cytomegalovirus IL-10, which can be used for coadministration with vaccines that do not have the capability to express IL-10.

### Example 2. Vaccination with AdGag-IRES-cIL-10

The AdGag-IRES-cIL-10 vaccine used in this example expressed both the SIV gag gene (*i.e.,* as a vaccine antigen) and rhesus macaque IL-10 under control of the EF1α promoter **(****FIG. 1D** **and** **FIG. 7****;** SEQ ID NO:1). The two coding regions were expressed as a single bicistronic message with the SIV gag gene located near the 5' end of the message, followed by an internal ribosomal entry site (IRES) and the rhesus macaque IL-10 coding sequence. The amino acid sequences of the antigen and the rhesus macaque IL-10 protein are set forth under SEQ ID NOS:2 and 4, respectively. A FLAG tag was also expressed, the amino acid sequence of which is set forth under SEQ ID NO:3. The virus was created by preparation of an expression cassette, transfer to the AdEasy shuttle plasmid, recombination in BJ5183 bacterial cells supplied with the kit (1), and rescue of the virus in C7 cells (1,2). Expression of the proper proteins was confirmed by mRNA analysis (both SIV gag and IL-10; **FIG. 2****),** Western blot for SIV gag protein **(****FIG. 3****),** and bioassay for functional IL-10 protein **(****FIG. 4****).**

The vector was then grown to high titer, purified by CsCl equilibrium gradient centrifugation, dialyzed against buffered 5% sucrose, and titered. Two wtRhCMV-seronegative macaques were then injected intramuscularly with 3 x 10¹¹ particles. Increased circulating IL-10 was confirmed in the treated animals by bioassay for functional IL-10 protein **(****FIG. 5****).**

Samples from these monkeys were collected before vaccination or 3, 14, 28, and 56 days later. These samples were then assessed for (i) T cell responses to the vaccine antigen and (ii) host IL-10 bioactivity and a variety of other biomarkers (by Luminex), to determine if the T cell responses occurred in an unusual immunologic context. Three unusual features were noted. First, while adenoviral vectors normally provoke very strong CD8⁺ T cell responses, in this experiment minimal CD8⁺ T cell responses were elicited and these responses declined rapidly in the first eight weeks after vaccination **(****FIGS. 6A** and **6B****).** IL-10 expression might therefore be useful whenever CD8⁺ T cell responses are thought undesirable, as previously proposed in the case of HIV vaccines. Second, IL-10 expression was associated with a different cytokine environment. In addition to the differences in IL-10 activity shown above, monkeys receiving AdGag-IRES-cIL-10 demonstrated greater expression of IL-8 **(****FIG. 6C****).** Finally and most surprisingly, monkeys vaccinated with AdGag-IRES-cIL-10 developed immune responses to Mamu-E-restricted peptides, which is an atypical immune response not usually associated with adenoviral vectors **(****FIG. 6D****).** Such responses might be useful whenever target cells are known to downregulate class Ia MHC alleles but to maintain expression of HLA-E. To cite two examples, both HIV-infected cells and tumor cells have been shown to downregulate class Ia MHC expression and therefore may be more vulnerable to T cells that are reactive to peptides bound on HLA-E molecules.

To better characterize the unique immune responses incorporating IL-10 expression, we vaccinated two groups of animals (n=6 animals in each group) with either (i) two administrations of a conventional adenoviral vector expressing the SIV Gag gene (Ad5 prime and Ad26 boost at 0 and 4 weeks, respectively) or (ii) two administrations of adenoviral vector expressing the SIV Gag gene with IL-10, via inclusion of an IRES-IL-10 cassette (Ad5GagIREScIL10 prime and Ad5GagIREScIL10 boost at 0 and 4 weeks, respectively). We then followed immune responses longitudinally by cytokine flow cytometry. Two extraordinary characteristics of the immune response to AdGagIREScIL10 vaccination were noted. First, immune responses in the CD4 compartment were extraordinary, easily outstripping those observed after administration of AdGag **(****FIG. 6E****,** solid line). Second, the ratio of CD4:CD8 responses was dramatically greater after administration of AdGagIREScIL10 vs. AdGag **(****FIG. 6F****,** solid line). Indeed, in some cases immune responses to AdGagIREScIL10 provoked comparable CD4 and CD8 T cell responses, approaching the ratio of 1.0 in **FIG. 6F****.** Comparable responses such as these are rarely or never seen after administration of AdGag.

In addition, **FIG. 8** shows that AdGag-IRES-cIL-10 provided superior protection against SIV challenge. Macaques were vaccinated with AdGag-IRES-cIL-10 (solid lines, open diamonds), were vaccinated twice with a control rhesus CMV-based vaccine (dashed lines, open circles), or were left unvaccinated (dotted lines, filled diamonds). The animals were subsequently challenged with serial low-dose oral SIVmac251, with challenges discontinued following confirmed infection. Shown in **FIG. 8** are viral loads in peripheral blood, synchronized to the day of presumed infection. Both AdGag-IRES-cIL-10-vaccinated animals controlled infection, with 1/2 maintaining viral load below the level of detection. No unvaccinated animals, nor those receiving the control RhCMV-based vaccine, controlled infection. For the data shown in **FIG. 8****,** the macaques vaccinated with AdGag-IRES-cIL-10 are from the same population as discussed earlier in this example, whereas the macaques vaccinated with the control vaccine and the unvaccinated macaques were appropriate agematched controls from another study.

### References

1. Hartigan-O'Connor, D., A. Amalfitano, and J.S. Chamberlain. J. Virol. (1999) 73(9):7835-41.
2. Amalfitano, A., M.A. Hauser, H. Hu, D. Serra, C.R. Begy, and J.S. Chamberlain. J. Virol. (1998) 72(2):926-33.

### Example 3. Construction of adenoviral vectors delivering MAGEA4 with IL-10 or MAGEA10 with IL-10

Systems for expressing melanoma-associated antigen 4 (MAGEA4) or melanoma-associated antigen 10 (MAGEA10) with IL-10 in a cell were constructed. Schematics of expression cassettes are shown in **FIGS. 10A** and **10B****,** and sequences are set forth under SEQ ID NOS:8 and 9. Briefly, vectors were constructed by assembly of fragments (FIG. 11A) into a shuttle vector **(****FIG.** 11B; containing essential elements for transferring/shuttling the complete expression cassettes into adenoviral (Ad) genomes in plasmid form), followed by recombination of the shuttle vector with the Ad genome in plasmid form **(****FIGS. 11C** and **11D****),** and finally by confirmation that the vectors delivered both the MAGE genes and IL-10 into a cell line **(****FIG. 11E****;** 293 cells). As shown in **FIG. 11A****,** MAGEA4, MAGEA10, and IRES-IL10 fragments were prepared by PCR amplification. The PCR primers had overlapping extensions that allowed their assembly into a shuttle plasmid by Gibson cloning. Subsequently, colony screening for the shuttle vectors pShMAGEA4-IRES-IL10 and pShMAGEA10-IRES-IL10 was performed **(****FIG. 11B****).** The primers that were used confirmed insertion of the IRES-IL-10 fragment at the *Sbj*I site of the shuttle vector. **FIG.** 11C shows restriction digests of complete plasmids containing MAGEA4-IRES-IL10 and MAGEA10-IRES-IL10 in adenoviral vector genomes, which resulted from recombination of the shuttle plasmids **(****FIG. 11B****)** with plasmids containing adenoviral vector genomes. **FIG. 11D** provides greater detail, demonstrating by *HindIII* digest that select adenoviral vector genomes had the predicted structure. The 1.2-kb band, visible in lanes 3 and 6, was specific to vectors containing the IRES-IL10 fragment. **FIG. 11E** shows that adenoviral vectors produced from the constructed genomes delivered MAGEA4 with IL-10 or MAGEA10 with IL-10. AdMAGEA4-IRES-IL10 produced both MAGEA4 (lane 1) and IL-10 (lane 13), whereas AdMAGEA10-IRES-IL10 produced both MAGEA10 (lane 8) and IL-10 (lane 14).

To confirm that AdMAGEA4-IRES-IL10 vaccines could provoke a strong immune response of unusual character, we injected a rhesus macaque serially with Ad5MAGEA4-IRES-IL10 and Ad26MAGEA4-IRES-IL10 (at 0 and 4 weeks). We then followed immune responses **(****FIG. 11F****).** Two features were noted. First, the immune responses were robust and sustained, totaling 0.75% of circulating CD8+ T cells by nine weeks. Second, the responding cells exhibited an usual balance of cytokine production (as compared to conventional adenoviral vectors), with greater representation of cells expressing interferon (IFN) only (0.53% at nine weeks in **FIG. 11F****)** compared to those co-expressing IFN and TNF (0.32%). Conventional adenoviral vectors instead elicit primarily cells that co-express IFN and TNF, reflecting the greater inflammatory character of those responses.

### Example 4. IL-10-independent methods

As another method of achieving increased IL-10 signaling, but without production and secretion of IL-10 (which might potentially have unwanted systemic effects), a vaccine antigen can be expressed together with STAT3C instead of IL-10. STAT3C is a constitutively active form of STAT3, which normally transduces the IL-10 signal from outside the cell to inside; thus, expression of STAT3C produces similar effects as IL-10 expression without a need to express the cytokine. SEQ ID NOS: 10-11 provide exemplary amino acid sequences of human and rhesus STAT3 proteins, in which two amino acid mutations (A->C and N->C, corresponding to amino acids 662 and 664 of SEQ ID NO: 10) convert the protein into a constitutively active form, *i.e.,* into STAT3C (SEQ ID NOS: 12-13).

### VII. Exemplary Embodiments

Exemplary embodiments provided in accordance with the presently disclosed subject matter include, but are not limited to, the claims and the following embodiments:
1. A system for expressing an antigen and a protein having interleukin-10 (IL-10)-like activity in a cell, the system comprising a recombinant polynucleotide comprising a nucleic acid sequence encoding the antigen and a recombinant polynucleotide comprising a nucleic acid sequence encoding the protein having IL-10-like activity.
2. The system of embodiment 1, wherein the antigen and the protein having IL-10-like activity are expressed in the same cell.
3. The system of embodiment 1 or 2, wherein the cell is selected from the group consisting of an antigen-presenting cell, a muscle cell, a tumor cell, a fibroblast, an epithelial cell, an endothelial cell, and a combination thereof.
4. The system of embodiment 3, wherein the antigen-presenting cell is a dendritic cell.
5. The system of any one of embodiments 1 to 4, wherein the recombinant polynucleotide comprising the nucleic acid sequence encoding the antigen and/or the recombinant polynucleotide comprising the nucleic acid sequence encoding the protein having IL-10-like activity further comprise one or more nucleic acid sequences that encode a regulatory sequence.
6. The system of embodiment 5, wherein the regulatory sequence controls expression of the antigen and/or the protein having IL-10-like activity.
7. The system of embodiment 5 or 6, wherein the regulatory sequence is selected from the group consisting of a promoter, an intronic sequence, an internal ribosome entry sequence (IRES), a polyadenylation signal, a Kozak consensus sequence, and a combination thereof.
8. The system of embodiment 7, wherein the promoter is an EF1α promoter.
9. The system of embodiment 8, wherein the EF1α promoter contains an intronic sequence.
10. The system of embodiment 7, wherein the polyadenylation signal is an SV40 virus polyadenylation signal.
11. The system of embodiment 7, wherein the IRES is selected from the group consisting of an encephalomyocarditis (EMCV) IRES, a human VCIP IRES, a mouse Gtx IRES, a cricket paralysis virus intergenic region, a hepatitis C virus IRES, a poliovirus IRES, a human rhinovirus IRES, a dicistrovirus intergenic IRES, a hepatitis A virus IRES, an enterovirus IRES, an Aichivirus IRES, a cardiovirus IRES, an aphthorivus IRES, a porcine teschovirus IRES, a sapelovirus IRES, a simian virus IRES, an avian encephalomyelitis virus IRES, a foot and mouth disease virus IRES, a cold stress-induced mRNA (Rbm3) IRES, a human NF-kB repressing factor (NRF) IRES, a human apoptotic protease-activating factor-1 (Apaf-1) IRES, an immunoglobulin heavy chain binding protein (BIP) IRES, an aquaporin 4 (AQP-4) IRES, a c-myc33 IRES, and a rat cationic amino-acid transporter-1 (CAT-1) IRES.
12. The system of any one of embodiments 1 to 11, wherein the recombinant polynucleotide comprising the nucleic acid sequence encoding the antigen and the recombinant polynucleotide comprising the nucleic acid sequence encoding the protein having IL-10-like activity are separate recombinant polynucleotides.
13. The system of any one of embodiments 1 to 11, wherein the recombinant polynucleotide comprising the nucleic acid sequence encoding the antigen and the recombinant polynucleotide comprising the nucleic acid sequence encoding the protein having IL-10-like activity are the same recombinant polynucleotide.
14. The system of embodiment 13, wherein the recombinant polynucleotide comprises:
   (a) the nucleic acid sequence encoding the antigen;
   (b) a nucleic acid sequence encoding an IRES; and
   (c) the nucleic acid sequence encoding the protein having IL-10-like activity.
15. The system of embodiment 14, wherein the nucleic acid sequence encoding the antigen is located 5' of the nucleic acid sequence encoding the IRES and the nucleic acid sequence encoding the protein having IL-10-like activity is located 3' of the nucleic acid sequence encoding the IRES.
16. The system of embodiment 15, wherein the recombinant polynucleotide further comprises:
   (d) a nucleic acid sequence encoding an EF1α promoter that is located 5' of the nucleic acid sequence encoding the antigen;
   (e) a nucleic acid sequence encoding an SV40 virus polyadenylation signal that is located 3' of the nucleic acid sequence encoding the protein having IL-10-like activity, and
   (f) a nucleic acid sequence encoding a Kozak consensus sequence that is located 3' of the nucleic acid sequence encoding the EF1α promoter and 5' of the nucleic acid sequence encoding the antigen.
17. The system of embodiment 13, wherein the antigen and the protein having IL-10-like activity are expressed under the control of two different promoters.
18. The system of embodiment 13, wherein the antigen and the protein having IL-10-like activity are expressed as a single protein.
19. The system of embodiment 18, wherein the single protein is a self-cleaving protein, and wherein self-cleavage of the single protein yields the antigen and the protein having IL-10-like activity as separate proteins.
20. The system of any one of embodiments 1 to 19, wherein the nucleic acid sequence encoding the antigen is codon-optimized for expression in a host.
21. The system of any one of embodiments 1 to 20, wherein the antigen is an infectious disease antigen.
22. The system of embodiment 21, wherein the infectious disease antigen is a bacterial, viral, fungal, protozoal, and/or helminthic infectious disease antigen.
23. The system of embodiment 21 or 22, wherein the infectious disease antigen is a viral infectious disease antigen from simian immunodeficiency virus (SIV), human immunodeficiency virus (HIV), hepatitis C virus, herpes simplex virus 1, herpes simplex virus 2, varicella-zoster virus, human cytomegalovirus, human herpesvirus 6A, human herpesvirus 6B, human herpesvirus 7, human herpesvirus 8, Epstein-Barr virus, a hemorrhagic fever virus, or a combination thereof.
24. The system of any one of embodiments 21 to 23, wherein the infectious disease antigen comprises an HIV or SIV group-specific antigen (gag) protein.
25. The system of embodiment 24, wherein the system comprises a recombinant polynucleotide comprising the nucleic acid sequence set forth in SEQ ID NO:1.
26. The system of embodiment 21 or 22, wherein the infectious disease antigen is a bacterial infectious disease antigen from Mycobacterium tuberculosis.
27. The system of any one of embodiments 1 to 20, wherein the antigen is a tumor-associated antigen.
28. The system of embodiment 27, wherein the tumor-associated antigen is selected from the group consisting of prostate-specific antigen, melanoma-associated antigen 4 (MAGEA4), melanoma-associated antigen 10 (MAGEA10), NY-ESO-1, and a combination thereof.
29. The system of embodiment 28, wherein the system comprises a recombinant polynucleotide comprising the nucleic acid sequence set forth in SEQ ID NO:8 or 9.
30. The system of embodiment 27, wherein the tumor-associated antigen is an antigen that newly arises in a subject's tumor.
31. The system of any one of embodiments 1 to 30, wherein the protein having IL-10-like activity is a heterologous protein having IL-10-like activity and/or a viral protein having IL-10-like activity.
32. The system of embodiment 31, wherein the heterologous protein having IL-10-like activity is an IL-10 protein from a host.
33. The system of embodiment 32, wherein the IL-10 protein from the host is a human IL-10 protein or a rhesus macaque IL-10 protein.
34. The system of embodiment 31, wherein the viral protein having IL-10-like activity is a cytomegalovirus (CMV) protein having IL-10-like activity.
35. The system of embodiment 34, wherein the CMV protein having IL-10-like activity is human CMV IL-10 (HCMVIL-10) or rhesus macaque CMV IL-10 (RhCMVIL-10).
36. The system of embodiment 31, wherein the heterologous protein having IL-10-like activity is STAT3 or a constitutively active form of STAT3.
37. The system of embodiment 36, wherein the constitutively active form of STAT3 is STAT3C.
38. The system of embodiment 36 or 37, wherein the STAT3 or constitutively active form of STAT3 is a human STAT3, or a rhesus macaque STAT3, or a constitutively active form thereof.
39. The system of any one of embodiments 1 to 38, wherein the nucleic acid sequence encoding the antigen and/or the nucleic acid sequence encoding the protein having IL-10-like activity do not contain intronic sequences.
40. The system of any one of embodiments 1 to 38, wherein the nucleic acid sequence encoding the antigen and/or the nucleic acid sequence encoding the protein having IL-10-like activity contain intronic sequences.
41. The system of any one of embodiments 1 to 40, wherein the recombinant polynucleotide comprising the nucleic acid sequence encoding the antigen and/or the recombinant polynucleotide comprising the nucleic acid sequence encoding the protein having IL-10-like activity further comprise a nucleic acid sequence encoding a tag.
42. The system of embodiment 41, wherein the tag is a FLAG tag.
43. The system of any one of embodiments 1 to 42, wherein the nucleic acid sequences encoding the antigen, the protein having IL-10-like activity, the one or more regulatory sequences, and/or the tag are located within the same open reading frame.
44. The system of any one of embodiments 1 to 43, wherein the recombinant polynucleotide comprising the nucleic acid sequence encoding the antigen and/or the recombinant polynucleotide comprising the nucleic acid sequence encoding the protein having IL-10-like activity are present within a viral vector.
45. The system of embodiment 44, wherein the viral vector is selected from the group consisting of a CMV vector, an adenoviral vector, an adeno-associated virus vector, a lentiviral vector, a herpes virus vector, an alphavirus vector, a retroviral vector, a poxvirus vector, and a vesicular stomatitis virus vector.
46. The system of embodiment 44 or 45, wherein the viral vector is selected from the group consisting of an adenoviral vector, an adeno-associated virus vector, a lentiviral vector, an alphavirus vector, a retroviral vector, and a vesicular stomatitis virus vector.
47. The system of any one of embodiments 44 to 46, wherein the viral vector contains a mutation that increases tropism for the cell.
48. The system of embodiment 47, wherein the viral vector is a CMV vector and the mutation that increases tropism comprises a mutation that modifies a protein, or portion thereof, that may be positioned on the outside of the CMV virion.
49. The system of embodiment 47 or 48, wherein the mutation that increases tropism comprises an insertion of a nucleotide sequence encoding a cellular targeting ligand.
50. The system of embodiment 49, wherein the cellular targeting ligand is selected from the group consisting of an antibody fragment that recognizes a target cell antigen, a ligand that is recognized by a target cell cognate receptor, a viral capsid protein that recognizes a target cell, and a combination thereof.
51. The system of embodiment 49 or 50, wherein the cellular targeting ligand is CD154.
52. The system of any one of embodiments 47 to 50, wherein the viral vector is a CMV vector, wherein the CMV is a CMV capable of infecting rhesus macaque cells, and wherein the mutation that increases tropism comprises a mutation within a gene selected from the group consisting of Rh13.1, Rh61/Rh60, Rh157.4, Rh157.5, Rh157.6, and a combination thereof.
53. The system of any one of embodiments 47 to 50, wherein the viral vector is a CMV vector, wherein the CMV is a CMV capable of infecting human cells, and wherein the mutation that increases tropism comprises a mutation within a gene selected from the group consisting of RL13, UL36, UL130, UL128, UL131, and a combination thereof.
54. The system of any one of embodiments 1 to 53, wherein the protein having IL-10-like activity is one to which a host does not have pre-existing immunity.
55. The system of any one of embodiments 20 to 54, wherein the host is a human or a non-human primate.
56. The system of any one of embodiments 1 to 55, wherein the recombinant polynucleotide comprising the nucleic acid sequence encoding the antigen and/or the recombinant polynucleotide comprising the nucleic acid sequence encoding the protein having IL-10-like activity further comprise a nucleic acid sequence encoding a selectable marker.
57. The system of embodiment 56, wherein the nucleic acid sequence encoding the selectable marker comprises a nucleic acid sequence encoding an antibiotic resistance gene and/or a fluorescent protein.
58. A viral particle comprising the system of any one of embodiments 1 to 57.
59. An engineered cell comprising the system of any one of embodiments 1 to 57 or the viral particle of embodiment 58.
60. A pharmaceutical composition comprising:
   (a) the system of any one of embodiments 1 to 57, the viral particle of embodiment 58, or the engineered cell of embodiment 59; and
   (b) a pharmaceutically acceptable carrier.
61. A method for inducing an immune response against an antigen in a subject, the method comprising administering to the subject a therapeutically effective amount of the pharmaceutical composition of embodiment 60.
62. The method of embodiment 61, wherein the antigen is an infectious disease antigen or a tumor-associated antigen.
63. The method of embodiment 62, wherein the infectious disease antigen is a bacterial, viral, fungal, protozoal, and/or helminthic infectious disease antigen.
64. The method of embodiment 62 or 63, wherein the infectious disease antigen is a viral infectious disease antigen from simian immunodeficiency virus (SIV), human immunodeficiency virus (HIV), hepatitis C virus, herpes simplex virus 1, herpes simplex virus 2, varicella-zoster virus, human cytomegalovirus, human herpesvirus 6A, human herpesvirus 6B, human herpesvirus 7, human herpesvirus 8, Epstein-Barr virus, a hemorrhagic fever virus, or a combination thereof.
65. The method of any one of embodiments 62 to 64, wherein the infectious disease antigen comprises an HIV or SIV group-specific antigen (gag) protein.
66. The method of any one of embodiments 62 or 63, wherein the infectious disease antigen is a bacterial infectious disease antigen from Mycobacterium tuberculosis.
67. The method of embodiment 62, wherein the tumor-associated antigen is selected from the group consisting of prostate-specific antigen, melanoma-associated antigen 4 (MAGEA4), melanoma-associated antigen 10 (MAGEA10), NY-ESO-1, and a combination thereof.
68. The method of embodiment 62, wherein the tumor-associated antigen is an antigen that newly arises in the subject's tumor.
69. The method of any one of embodiments 61 to 68, wherein inducing the immune response comprises generating antibodies that recognize the antigen.
70. The method of any one of embodiments 61 to 69, wherein the immune response that is induced in the subject comprises a lower CD8+ T cell response as compared to the CD8+ T cell response that is induced using a pharmaceutical composition that does not express the antigen and the protein having IL-10-like activity in the cell.
71. The method of any one of embodiments 61 to 70, wherein the immune response that is induced in the subject comprises a higher CD4+ T cell response as compared to the CD4+ T cell response that is induced using a pharmaceutical composition that does not express the antigen and the protein having IL-10-like activity in the cell.
72. The method of any one of embodiments 61 to 71, wherein the expression or activity of interleukin-8 (IL-8) is increased in the subject.
73. The method of any one of embodiments 61 to 72, wherein inducing the immune response comprises increasing the number or activation of MHC class Ib-restricted T cells in the subject.
74. The method of any one of embodiments 61 to 73, wherein a sample is obtained from the subject.
75. The method of embodiment 74, wherein the sample is selected from the group consisting of a blood sample, a tissue sample, a urine sample, a saliva sample, a cerebrospinal fluid (CSF) sample, and a combination thereof.
76. The method of embodiment 74 or 75, wherein the level of one or more biomarkers is determined in the sample.
77. The method of embodiment 76, wherein the one or more biomarkers is selected from the group consisting of IL-8, IL-10, IL-12, interferon-gamma, tumor necrosis factor-alpha, and a combination thereof.
78. The method of embodiment 76 or 77, wherein the level of the one or more biomarkers is compared to a reference sample.
79. The method of embodiment 78, wherein the reference sample is obtained from the subject.
80. The method of embodiment 78, wherein the reference sample is obtained from a different subject or a population of subjects.
81. A method for preventing or treating a disease in a subject, the method comprising administering to the subject a therapeutically effective amount of the pharmaceutical composition of embodiment 60.
82. The method of embodiment 81, wherein the disease is an infectious disease or cancer.
83. The method of embodiment 82, wherein the infectious disease is a bacterial, viral, fungal, protozoal, and/or helminthic infectious disease.
84. The method of embodiment 83, wherein the viral infectious disease is caused by a simian immunodeficiency virus (SIV), a human immunodeficiency virus (HIV), a hepatitis C virus, a herpes simplex virus 1, a herpes simplex virus 2, a varicella-zoster virus, a human cytomegalovirus, a human herpesvirus 6A, a human herpesvirus 6B, a human herpesvirus 7, a human herpesvirus 8, an Epstein-Barr virus, or a hemorrhagic fever virus.
85. The method of embodiment 82, wherein the cancer is melanoma, ovarian cancer, lymphoma, leukemia, or prostate cancer.
86. The method of any one of embodiments 81 to 85, wherein treating the subject comprises decreasing or eliminating one or more signs or symptoms of the disease.
87. The method of any one of embodiments 61 to 86, further comprising administering an exogenous protein having IL-10-like activity to the subject.

It is understood that the examples and embodiments described herein are for illustrative purposes only and that various modifications or changes in light thereof will be suggested to persons skilled in the art and are to be included within the spirit and purview of this application and scope of the appended claims. All publications, patents, patent applications, and sequence reference numbers cited herein are hereby incorporated by reference in their entirety for all purposes.

**Table 1. Informal Sequence Listing**

| **SEQ ID NO:** | **Sequence** | **Description** |
|---|---|---|
| 1 | 5’- CGTGAGGCTCCGGTGCCCGTCAGTGGGCAGAGCGCACATCGCCCACAGTCCCC GAGAAGTTGGGGGGAGGGGTCGGCAATTGAACCGGTGCCTAGAGAAGGTGGC GCGGGGTAAACTGGGAAAGTGATGTCGTGTACTGGCTCCGCCTTTTTCCCGAG GGTGGGGGAGAACCGTATATAAGTGCAGTAGTCGCCGTGAACGTTCTTTTTCG CAACGGGTTTGCCGCCAGAACACAGGTAAGTGCCGTGTGTGGTTCCCGCGGGC CTGGCCTCTTTACGGGTTATGGCCCTTGCGTGCCTTGAATTACTTCCACCTGGC TGCAGTACGTGATTCTTGATCCCGAGCTTCGGGTTGGAAGTGGGTGGGAGAGT TCGAGGCCTTGCGCTTAAGGAGCCCCTTCGCCTCGTGCTTGAGTTGAGGCCTG GCCTGGGCGCTGGGGCCGCCGCGTGCGAATCTGGTGGCACCTTCGCGCCTGTC TCGCTGCTTTCGATAAGTCTCTAGCCATTTAAAATTTTTGATGACCTGCTGCGA CGCTTTTTTTCTGGCAAGATAGTCTTGTAAATGCGGGCCAAGATCTGCACACT GGTATTTCGGTTTTTGGGGCCGCGGGCGGCGACGGGGCCCGTGCGTCCCAGCG CACATGTTCGGCGAGGCGGGGCCTGCGAGCGCGGCCACCGAGAATCGGACGG GGGTAGTCTCAAGCTGGCCGGCCTGCTCTGGTGCCTGGCCTCGCGCCGCCGTG TATCGCCCCGCCCTGGGCGGCAAGGCTGGCCCGGTCGGCACCAGTTGCGTGAG CGGAAAGATGGCCGCTTCCCGGCCCTGCTGCAGGGAGCTCAAAATGGAGGAC GCGGCGCTCGGGAGAGCGGGCGGGTGAGTCACCCACACAAAGGAAAAGGGC CTTTCCGTCCTCAGCCGTCGCTTCATGTGACTCCACGGAGTACCGGGCGCCGT CCAGGCACCTCGATTAGTTCTCGAGCTTTTGGAGTACGTCGTCTTTAGGTTGGG GGGAGGGGTTTTATGCGATGGAGTTTCCCCACACTGAGTGGGTGGAGACTGA AGTTAGGCCAGCTTGGCACTTGATGTAATTCTCCTTGGAATTTGCCCTTTTTGA GTTTGGATCTTGGTTCATTCTCAAGCCTCAGACAGTGGTTCAAAGTTTTTTTCT TCCATTTCAGGTGTCGTGAGGAATTAGCTGGCGCGCCTTCCCGCCACCATGGG CGTGAGAAACTCCGTCTTGTCAGGGAAGAAAGCAGATGAATTAGAAAAAATT AGGCTACGACCCAACGGAAAGAAAAAGTACATGTTGAAGCATGTAGTATGGG CAGCAAATGAATTAGATAGATTTGGATTAGCAGAAAGCCTGTTGGAGAACAA AGAAGGATGTCAAAAAATACTTTCGGTCTTAGCTCCATTAGTGCCAACAGGCT CAGAAAATTTAAAAAGCCTTTATAATACTGTCTGCGTCATCTGGTGCATTCAC GCAGAAGAGAAAGTGAAACACACTGAGGAAGCAAAACAGATAGTGCAGAGA CACCTAGTGGTGGAAACAGGAACCACCGAAACCATGCCGAAGACCTCTCGAC CAACAGCACCATCTAGCGGCAGAGGAGGAAACTACCCAGTACAGCAGATCGG TGGCAACTACGTCCACCTGCCACTGTCCCCGAGAACCCTGAACGCTTGGGTCA AGCTGATCGAGGAGAAGAAGTTCGGAGCAGAAGTAGTGCCAGGATTCCAGGC ACTGTCAGAAGGTTGCACCCCCTACGACATCAACCAGATGCTGAACTGCGTTG GAGACCATCAGGCGGCTATGCAGATCATCCGTGACATCATCAACGAGGAGGC TGCAGATTGGGACTTGCAGCACCCACAACCAGCTCCACAACAAGGACAACTT AGGGAGCCGTCAGGATCAGACATCGCAGGAACCACCTCCTCAGTTGACGAAC AGATCCAGTGGATGTACCGTCAGCAGAACCCGATCCCAGTAGGCAACATCTA CCGTCGATGGATCCAGCTGGGTCTGCAGAAATGCGTCCGTATGTACAACCCGA CCAACATTCTAGATGTAAAACAAGGGCCAAAAGAGCCATTTCAGAGCTATGT AGACAGGTTCTACAAAAGTTTAAGAGCAGAACAGACAGATGCAGCAGTAAAG AATTGGATGACTCAAACACTGCTGATTCAAAATGCTAACCCAGATTGCAAGCT AGTGCTGAAGGGGCTGGGTGTGAATCCCACCCTAGAAGAAATGCTGACGGCT TGTCAAGGAGTAGGGGGGCCGGGACAGAAGGCTAGATTAATGGCAGAAGCCC TGAAAGAGGCCCTCGCACCAGTGCCAATCCCTTTTGCAGCAGCCCAACAGAG GGGACCAAGAAAGCCAATTAAGTGTTGGAATTGTGGGAAAGAGGGACACTCT GCAAGGCAATGCAGAGCCCCAAGAAGACAGGGATGCTGGAAATGTGGAAAA ATGGACCATGTTATGGCCAAATGCCCAGACAGACAGGCGGGTTTTTTAGGCCT TGGTCCATGGGGAAAGAAGCCCCGCAATTTCCCCATGGCTCAAGTGCATCAGG GGCTGATGCCAACTGCTCCCCCAGAGGACCCAGCTGTGGATCTGCTAAAGAAC TACATGCAGTTGGGCAAGCAGCAGAGAGAAAAGCAGAGAGAAAGCAGAGAG AAGCCTTACAAGGAGGTGACAGAGGATTTGCTGCACCTCAATTCTCTCTTTGG AGGAGACCAGGACTACAAAGACGATGACGACAAGTAGTAGCCTGCAGGCCCC TCTCCCTCCCCCCCCCCTAACGTTACTGGCCGAAGCCGCTTGGAATAAGGCCG | Expression cassette nucleic acid sequence (EF-1α promoter: 6-1,184; EF-1α intron A: 237-1,175; codon-optimized SIV Gag: 1,214-2,742; FLAG: 2,744-2,767; EMCV IRES: 2,782-3,355; rhesus macaque IL-10: 3,363-3,902; SV40 polyadenylation signal: 4,029-4,110) |
| | GTGTGCGTTTGTCTATATGTTATTTTCCACCATATTGCCGTCTTTTGGCAATGT GAGGGCCCGGAAACCTGGCCCTGTCTTCTTGACGAGCATTCCTAGGGGTCTTT CCCCTCTCGCCAAAGGAATGCAAGGTCTGTTGAATGTCGTGAAGGAAGCAGTT CCTCTGGAAGCTTCTTGAAGACAAACAACGTCTGTAGCGACCCTTTGCAGGCA GCGGAACCCCCCACCTGGCGACAGGTGCCTCTGCGGCCAAAAGCCACGTGTA TAAGATACACCTGCAAAGGCGGCACAACCCCAGTGCCACGTTGTGAGTTGGA TAGTTGTGGAAAGAGTCAAATGGCTCTCCTCAAGCGTATTCAACAAGGGGCTG AAGGATGCCCAGAAGGTACCCCATTGTATGGGATCTGATCTGGGGCCTCGGTG CACATGCTTTACATGTGTTTAGTCGAGGTTAAAAAAACGTCTAGGCCCCCCGA ACCACGGGGACGTGGTTTTCCTTTGAAAAACACGATGATAAAGCCACCATGCA CAGCTCAGCACTGCTCTGTTGCCTAGTCCTCCTGACTGGGGTGAGGGCCAGCC CAGGCCAGGGCACCCAGTCTGAGAACAGCTGCACCCGCTTCCCAGGCAACCT GCCTCACATGCTTCGAGACCTCCGAGATGCCTTCAGCAGAGTGAAGACTTTCT TTCAAATGAAGGATCAGCTGGACAACATATTGTTAAAGGAGTCCTTGCTGGAG GACTTTAAGGGTTACCTGGGTTGCCAAGCCTTGTCTGAGATGATCCAGTTTTA CCTGGAGGAGGTGATGCCCCAAGCTGAGAACCACGACCCAGACATCAAGGAG CATGTGAACTCCCTGGGGGAGAATCTGAAGACCCTCAGGCTGAGGCTGCGGC GCTGTCATCGATTTCTTCCCTGTGAAAACAAGAGCAAGGCCGTGGAGCAGGTG AAGAATGCCTTTAGTAAGCTCCAAGAGAAAGGCGTCTACAAAGCCATGAGTG AGTTTGACATCTTCATCAACTACATAGAAGCCTACATGACAATGAAGATACAA AACTGAGACCTGCAGGACGCCACAGCTCTGATCATAATCAGCCATACCACATT TGTAGAGGTTTTACTTGCTTTAAAAAACCTCCCACACCTCCCCCTGAACCTGA AACATAAAATGAATGCAATTGTTGTTGTTAACTTGTTTATTGCAGCTTATAATG GTTACAAATAAAGCAATAGCATCACAAATTTCACAAATAAAGCATTTTTTTCA CTGCATTCTAGTTGTGGTTTGTCCAAACTCATCAATGTATCT-3’ | |
| 2 | MGVRNSVLSGKKADELEKIRLRPNGKKKYMLKHVVWAANELDRFGLAESLLEN KEGCQKILSVLAPLVPTGSENLKSLYNTVCVIWCIHAEEKVKHTEEAKQIVQRHL VVETGTTETMPKTSRPTAPSSGRGGNYPVQQIGGNYVHLPLSPRTLNAWVKLIEE KKFGAEVVPGFQALSEGCTPYDINQMLNCVGDHQAAMQIIRDIINEEAADWDLQ HPQPAPQQGQLREPSGSDIAGTTSSVDEQIQWMYRQQNPIPVGNIYRRWIQLGLQ KCVRMYNPTNILDVKQGPKEPFQSYVDRFYKSLRAEQTDAAVKNWMTQTLLIQN ANPDCKLVLKGLGVNPTLEEMLTACQGVGGPGQKARLMAEALKEALAPVPIPFA AAQQRGPRKPIKCWNCGKEGHSARQCRAPRRQGCWKCGKMDHVMAKCPDRQA GFLGLGPWGKKPRNFPMAQVHQGLMPTAPPEDPAVDLLKNYMQLGKQQREKQR ESREKPYKEVTEDLLHLNSLFGGD | Codon-optimized SIV Gag amino acid sequence |
| 3 | DYKDDDDK | FLAG amino acid sequence |
| 4 | MHSSALLCCLVLLTGVRASPGQGTQSENSCTRFPGNLPHMLRDLRDAFSRVKTFF QMKDQLDNILLKESLLEDFKGYLGCQALSEMIQFYLEEVMPQAENHDPDIKEHVN SLGENLKTLRLRLRRCHRFLPCENKSKAVEQVKNAFSKLQEKGVYKAMSEFDIFI NYIEAYMTMKIQN | Rhesus macaque IL-10 amino acid sequence |
| 5 | 5’- ATGCGGAGGAGGAGGAGGTCTTTCGGCATCATCGTCGCCGGCGCTATCGGAA CACTACTCATGATGGCGGTGGTCGTGCTTTCAGCCCATGACCATGAACACAAA GAAGTACCACCGGCCTGTGACCCCGTTCACGGTAACTTGGCAGGCATCTTCAA GGAGTTGCGGGCGACCTACGCTTCCATTAGAGAAGGTTTGGTATGTTAGGCAA CGCAGTTCTCGGATGTCAGTCCGGATCGGAGGAGTCACAGTCTGTCATGTGAT GATATATTGCTTAATTTTTGTTTTGCAGCAAAAGAAGGACACGGTGTACTACA CATCGCTGTTCAATGACCGCGTGCTCCATGAAATGCTGAGTCCTATGGGCTGT CGCGTGACCAATGAACTCATGGAACATTATTTAGATGGTGTTCTGCCTCGAGC AAGTCATTTAGACTACGATAATAGCACTCTGAATGGCTTACATGTGTTTGCTTC ATCCATGCAGGCGCTGTATCAGCACATGTTAAAGTGTGTAAGTGTTTCAGGTT CGATAACCCCGCGATATGACACGTAAATAGCGATATCGTGGCACCAGACGTC AGTCACAGTCTTCCCCGGTCGAGACGCATCTTATATCGCGATATATCGCGGAT TATCGCAGTATGTAGCGATATATCGTGTCAAAGCACTCCGAACGACATTCTGA TGACGGCTATCGCCTTATGTCGCGGTATATCGCGGAATATCGCAGTATATCGC GGTTATGTCGCGACATAACCGTCATGTCGCGACTATCGCCGCATATCGCCACT | Viral IL-10-like sequence from RhCMV strain 68-1 |
| | ATCGCGACTTGGCACCGTGCCAACGATAGTCGACCTTAGGGTGGTCGTGTGGT GGTGGGGGGCTGCTTGCGGTTTGCAAACCGGAGAGGTAGCACACGCTGATTG TCGGTTTGGAAGCGTTGTTTACACATGTCTTTGTCTTGGCAGCCCGCGTTGGCA TGTACTGGCAAAACGCCAGCTTGGATGTACTTCTTGGAGGTGGAACACAAGGT CAGTTAAGGTTGCCAGGTAGGTTAAAACGCAGAAACCATTGTTCTACCGGTTT CCTAAAACGCCGTTCAACGTGTTTTGCAGCTCAACCCCTGGAGGGGCACGGCA AAAGCCGCGGCCGAGGCTGACCTTTTGCTGAACTACTTGGAAACGTTCCTGCT GCAGTTCTGA-3' | |
| 6 | 5’- ATGCGGAGGAGGAGGAGGTCTTTCGGCATCATCGTCGCCGGCGCTATCGGAA CACTACTCATGATGGCGGTGGTCGTGCTTTCAGCCCATGAACACAAAGAAGTA CCACCGGCCTGTGACCCCGTTCACGGTAACTTGGCAGGCATCTTCAAGGAGTT GCGGGCGACCTACGCTTCCATTAGAGAAGGTTTGGTATGTTAGGCAACGCAGT TCTCGGATGTCAGTCCGGATCGGAGGAGTCACAGTCTGTCATGTGATGATATA TTGCTTCATTTTTGTTTTGCAGCAAAAGAAGGACACGGTGTACTACACATCGC TGTTCAATGACCGCGTGCTCCATGAGATGCTGAGTCCTATGGGCTGTCGCGTG ACCAACGAACTCATGGAACATTATTTAGATGGTGTTCTGCCTCGAGCAAGTCA TTTAGACTACGATAATAGCACTCTGAATGGCTTACATGTGTTTGCTTCATCCAT GCAGGCGCTGTATCAGCACATGTTAAAGTGTGTAAGTGTTTCAGGTTCGATAA CCCCGCGATATGACACGTAAATAGCGATATCGTGGCACCAGACGTCAGTCAC AGTCTTCCCCGGTCGAGACGCATCTTATATCGCGATATATCGCGGTTTATCGC AGTATGTCGCGATATATCGCTCCAAAACACTCCGGATGACTTTCTATCGCCGA ATATCACCTCATATCGTCTTATATCGCGGTGTATCGCGGGTTATCGTCATATAT CGCGGTTATGTCGCGACATAACCGTCATGTCGCGACTATCGCCGCATATCGCC ACTATCGCGACTTGGCACGGTGCCAACAATAGTTGCCTCTAGGGTGGTCGTGT GGTGGTAGGGGGCTGCTTACGGTTTGCAAACCGGAGAGGTCGCACACGCTGA TTGTCGGTTTGGAAGCGTTGTTTACACATGTCTTTGTCTTGGCAGCCCGCGTTG GCATGTACTGGCAAAACGCCAGCTTGGATGTACTTCTTGGAGGTGGAACACAA GGTCAGTTAAGGTTGCCAGGTAGGTTAAAACGCAGAAACCATTGTTCTACCGG TTTCCTAAAACGCCGTTCAACGTGTTTTGCAGCTCAACCCCTGGAGGGGCACG GCAAAAGCCGCGGCCGAGGCTGACCTTTTGCTGAACTACTTGGAAACGTTCCT GCTGCAGTTCTGA-3’ | Viral IL-10-like sequence from *Cercopithecine herpesvirus* 8 isolate CMV 180.92 |
| 7 | MHSSALLCCLVLLTGVRASPGQGTQSENSCTHFPGNLPNMLRDLRDAFSRVKTFF QMKDQLDNLLLKESLLEDFKGYLGCQALSEMIQFYLEEVMPQAENQDPDIKAHV NSLGENLKTLRLRLRRCHRFLPCENKSKAVEQVKNAFNKLQEKGIYKAMSEFDIFI NYIEAYMTMKIRN | Human IL-10 |
| 8 | CGTGAGGCTCCGGTGCCCGTCAGTGGGCAGAGCGCACATCGCCCACAGTCCCC GAGAAGTTGGGGGGAGGGGTCGGCAATTGAACCGGTGCCTAGAGAAGGTGGC GCGGGGTAAACTGGGAAAGTGATGTCGTGTACTGGCTCCGCCTTTTTCCCGAG GGTGGGGGAGAACCGTATATAAGTGCAGTAGTCGCCGTGAACGTTCTTTTTCG CAACGGGTTTGCCGCCAGAACACAGGTAAGTGCCGTGTGTGGTTCCCGCGGGC CTGGCCTCTTTACGGGTTATGGCCCTTGCGTGCCTTGAATTACTTCCACCTGGC TGCAGTACGTGATTCTTGATCCCGAGCTTCGGGTTGGAAGTGGGTGGGAGAGT TCGAGGCCTTGCGCTTAAGGAGCCCCTTCGCCTCGTGCTTGAGTTGAGGCCTG GCCTGGGCGCTGGGGCCGCCGCGTGCGAATCTGGTGGCACCTTCGCGCCTGTC TCGCTGCTTTCGATAAGTCTCTAGCCATTTAAAATTTTTGATGACCTGCTGCGA CGCTTTTTTTCTGGCAAGATAGTCTTGTAAATGCGGGCCAAGATCTGCACACT GGTATTTCGGTTTTTGGGGCCGCGGGCGGCGACGGGGCCCGTGCGTCCCAGCG CACATGTTCGGCGAGGCGGGGCCTGCGAGCGCGGCCACCGAGAATCGGACGG GGGTAGTCTCAAGCTGGCCGGCCTGCTCTGGTGCCTGTCCTCGCGCCGCCGTG TATCGCCCCGCCCTGGGCGGCAAGGCTGGCCCGGTCGGCACCAGTTGCGTGAG CGGAAAGATGGCCGCTTCCCGGCCCTGCTGCAGGGAGCTCAAAATGGAGGAC GCGGCGCTCGGGAGAGCGGGCGGGTGAGTCACCCACACAAAGGAAAAGGGC CTTTCCGTCCTCAGCCGTCGCTTCATGTGACTCCACGGAGTACCGGGCGCCGT CCAGGCACCTCGATTAGTTCTCGAGCTTTTGGAGTACGTCGTCTTTAGGTTGGG GGGAGGGGTTTTATGCGATGGAGTTTCCCCACACTGAGTGGGTGGAGACTGA AGTTAGGCCAGCTTGGCACTTGATGTAATTCTCCTTGGAATTTGCCCTTTTTGA GTTTGGATCTTGGTTCATTCTCAAGCCTCAGACAGTGGTTCAAAGTTTTTTTCT | pAdMAGEA4IRE SIL10 from EFla promoter-SV40 |
| | TCCATTTCAGGTGTCGTGAGGAATTAGCTGGCGCGCCTTCCCGCCACCATGTC TTCTGAGCAGAAGAGTCAGCACTGCAAGCCTGAGGAAGGCGTTGAGGCCCAA GAAGAGGCCCTGGGCCTGGTGGGTGCACAGGCTCCTACTACTGAGGAGCAGG AGGCTGCTGTCTCCTCCTCCTCTCCTCTGGTCCCTGGCACCCTGGAGGAAGTGC CTGCTGCTGAGTCAGCAGGTCCTCCCCAGAGTCCTCAGGGAGCCTCTGCCTTA CCCACTACCATCAGCTTCACTTGCTGGAGGCAACCCAATGAGGGTTCCAGCAG CCAAGAAGAGGAGGGGCCAAGCACCTCGCCTGACGCAGAGTCCTTGTTCCGA GAAGCACTCAGTAACAAGGTGGATGAGTTGGCTCATTTTCTGCTCCGCAAGTA TCGAGCCAAGGAGCTGGTCACAAAGGCAGAAATGCTGGAGAGAGTCATCAAA AATTACAAGCGCTGCTTTCCTGTGATCTTCGGCAAAGCCTCCGAGTCCCTGAA GATGATCTTTGGCATTGACGTGAAGGAAGTGGACCCCGCCAGCAACACCTAC ACCCTTGTCACCTGCCTGGGCCTTTCCTATGATGGCCTGCTGGGTAATAATCAG ATCTTTCCCAAGACAGGCCTTCTGATAATCGTCCTGGGCACAATTGCAATGGA GGGCGACAGCGCCTCTGAGGAGGAAATCTGGGAGGAGCTGGGTGTGATGGGG GTGTATGATGGGAGGGAGCACACTGTCTATGGGGAGCCCAGGAAACTGCTCA CCCAAGATTGGGTGCAGGAAAACTACCTGGAGTACCGGCAGGTACCCGGCAG TAATCCTGCGCGCTATGAGTTCCTGTGGGGTCCAAGGGCTCTGGCTGAAACCA GCTATGTGAAAGTCCTGGAGCATGTGGTCAGGGTCAATGCAAGAGTTCGCATT GCCTACCCATCCCTGCGTGAAGCAGCTTTGTTAGAGGAGGAAGAGGGAGTCT GATAGCCTGCAGGCCCCTCTCCCTCCCCCCCCCCTAACGTTACTGGCCGAAGC CGCTTGGAATAAGGCCGGTGTGCGTTTGTCTATATGTTATTTTCCACCATATTG CCGTCTTTTGGCAATGTGAGGGCCCGGAAACCTGGCCCTGTCTTCTTGACGAG CATTCCTAGGGGTCTTTCCCCTCTCGCCAAAGGAATGCAAGGTCTGTTGAATG TCGTGAAGGAAGCAGTTCCTCTGGAAGCTTCTTGAAGACAAACAACGTCTGTA GCGACCCTTTGCAGGCAGCGGAACCCCCCACCTGGCGACAGGTGCCTCTGCGG CCAAAAGCCACGTGTATAAGATACACCTGCAAAGGCGGCACAACCCCAGTGC CACGTTGTGAGTTGGATAGTTGTGGAAAGAGTCAAATGGCTCTCCTCAAGCGT ATTCAACAAGGGGCTGAAGGATGCCCAGAAGGTACCCCATTGTATGGGATCT GATCTGGGGCCTCGGTGCACATGCTTTACATGTGTTTAGTCGAGGTTAAAAAA ACGTCTAGGCCCCCCGAACCACGGGGACGTGGTTTTCCTTTGAAAAACACGAT GATAAAGCCACCATGCACAGCTCAGCACTGCTCTGTTGCCTAGTCCTCCTGAC TGGGGTGAGGGCCAGCCCAGGCCAGGGCACCCAGTCTGAGAACAGCTGCACC CGCTTCCCAGGCAACCTGCCTCACATGCTTCGAGACCTCCGAGATGCCTTCAG CAGAGTGAAGACTTTCTTTCAAATGAAGGATCAGCTGGACAACATATTGTTAA AGGAGTCCTTGCTGGAGGACTTTAAGGGTTACCTGGGTTGCCAAGCCTTGTCT GAGATGATCCAGTTTTACCTGGAGGAGGTGATGCCCCAAGCTGAGAACCACG ACCCAGACATCAAGGAGCATGTGAACTCCCTGGGGGAGAATCTGAAGACCCT CAGGCTGAGGCTGCGGCGCTGTCATCGATTTCTTCCCTGTGAAAACAAGAGCA AGGCCGTGGAGCAGGTGAAGAATGCCTTTAGTAAGCTCCAAGAGAAAGGCGT CTACAAAGCCATGAGTGAGTTTGACATCTTCATCAACTACATAGAAGCCTACA TGACAATGAAGATACAAAACTGAGACCTGCAGGACGCCACAGCTCTGATCAT AATCAGCCATACCACATTTGTAGAGGTTTTACTTGCTTTAAAAAACCTCCCAC ACCTCCCCCTGAACCTGAAACATAAAATGAATGCAATTGTTGTTGTTAACTTG TTTATTGCAGCTTATAATGGTTACAAATAAAGCAATAGCATCACAAATTTCAC AAATAAAGCATTTTTTTCACTGCATTCTAGTTGTGGTTTGTCCAAACTCATCAA TGTATCT | |
| 9 | CGTGAGGCTCCGGTGCCCGTCAGTGGGCAGAGCGCACATCGCCCACAGTCCCC GAGAAGTTGGGGGGAGGGATCGGCAATTGAACCGGTGCCTAGAGAAGGTGGC GCGGGGTAAACTGGGAAAGTGATGTCGTGTACTGGCTCCGCCTTTTTCCCGAG GGTGGGGGAGAACCGTATATAAGTGCAGTAGTCGCCGTGAACGTTCTTTTTCG CAACGGGTTTGCCGCCAGAACACAGGTAAGTGCCGTGTGTGGTTCCCGCGGGC CTGGCCTCTTTACGGGTTATGGCCCTTGCGTGCCTTGAATTACTTCCACCTGGC TGCAGTACGTGATTCTTGATCCCGAGCTTCGGGTTGGAAGTGGGTGGGAGAGT TCGAGGCCTTGCGCTTAAGGAGCCCCTTCGCCTCGTGCTTGAGTTGAGGCCTG GCCTGGGCGCTGGGGCCGCCGCGTGCGAATCTGGTGGCACCTTCGCGCCTGTC TCGCTGCTTTCGATAAGTCTCTAGCCATTTAAAATTTTTGATGACCTGCTGCGA CGCTTTTTTTCTGGCAAGATAGTCTTGTAAATGCGGGCCAAGATCTGCACACT GGTATTTCGGTTTTTGGGGCCGCGGGCGGCGACGGGGCCCGTGCGTCCCAGCG CACATGTTCGGCGAGGCGGGGCCTGCGAGCGCGGCCACCGAGAATCGGACGG GGGTAGTCTCAAGCTGGCCGGCCTGCTCTGGTGCCTGTCCTCGCGCCGCCGTG | pAdMAGEA10IR ESIL10 from EF1a promoter-SV40 |
| | TATCGCCCCGCCCTGGGCGGCAAGGCTGGCCCGGTCGGCACCAGTTGCGTGAG CGGAAAGATGGCCGCTTCCCGGCCCTGCTGCAGGGAGCTCAAAATGGAGGAC GCGGCGCTCGGGAGAGCGGGCGGGTGAGTCACCCACACAAAGGAAAAGGGC CTTTCCGTCCTCAGCCGTCGCTTCATGTGACTCCACGGAGTACCGGGCGCCGT CCAGGCACCTCGATTAGTTCTCGAGCTTTTGGAGTACGTCGTCTTTAGGTTGGG GGGAGGGGTTTTATGCGATGGAGTTTCCCCACACTGAGTGGGTGGAGACTGA AGTTAGGCCAGCTTGGCACTTGATGTAATTCTCCTTGGAATTTGCCCTTTTTGA GTTTGGATCTTGGTTCATTCTCAAGCCTCAGACAGTGGTTCAAAGTTTTTTTCT TCCATTTCAGGTGTCGTGAGGAATTAGCTGGCGCGCCTTCCCGCCACCATGCC TCGAGCTCCAAAGCGTCAGCGCTGCATGCCTGAAGAAGATCTTCAATCCCAAA GTGAGACACAGGGCCTCGAGGGTGCACAGGCTCCCCTGGCTGTGGAGGAGGA TGCTTCATCATCCACTTCCACCAGCTCCTCTTTTCCATCCTCTTTTCCCTCCTCC TCCTCTTCCTCCTCCTCCTCCTGCTATCCTCTAATACCAAGCACCCCAGAGGAG GTTTCTGCTGATGATGAGACACCAAATCCTCCCCAGAGTGCTCAGATAGCCTG CTCCTCCCCCTCGGTCGTTGCTTCCCTTCCATTAGATCAATCTGATGAGGGCTC CAGCAGCCAAAAGGAGGAGAGTCCAAGCACCCTACAGGTCCTGCCAGACAGT GAGTCTTTACCCAGAAGTGAGATAGATGAAAAGGTGACTGATTTGGTGCAGTT TCTGCTCTTCAAGTATCAAATGAAGGAGCCGATCACAAAGGCAGAAATACTG GAGAGTGTCATAAGAAATTATGAAGACCACTTCCCTTTGTTGTTTAGTGAAGC CTCCGAGTGCATGCTGCTGGTCTTTGGCATTGATGTAAAGGAAGTGGATCCCA CTGGCCACTCCTTTGTCCTTGTCACCTCCCTGGGCCTCACCTATGATGGGATGC TGAGTGATGTCCAGAGCATGCCCAAGACTGGCATTCTCATACTTATCCTAAGC ATAGTCTTCATAGAGGGCTACTGCACCCCTGAGGAGGTCATCTGGGAAGCACT GAATATGATGGGGCTGTATGATGGGATGGAGCACCTCATTTATGGGGAGCCC AGGAAGCTGCTCACCCAAGATTGGGTGCAGGAAAACTACCTGGAGTACCGGC AGGTGCCTGGCAGTGATCCTGCACGGTATGAGTTTCTGTGGGGTCCAAGGGCT CATGCTGAAATTAGGAAGATGAGTCTCCTGAAATTTTTGGCCAAGGTAAATGG GAGTGATCCAAGATCCTTCCCACTGTGGTATGAGGAGGCTTTGAAAGATGAGG AAGAGAGAGCCCAGGACAGAATTGCCACCACAGATGATACTACTGCCATGGC CAGTGCAAGTTCTAGCGCTACAGGTAGCTTCTCCTACCCTGAATAATAGCCTG CAGGCCCCTCTCCCTCCCCCCCCCCTAACGTTACTGGCCGAAGCCGCTTGGAA TAAGGCCGGTGTGCGTTTGTCTATATGTTATTTTCCACCATATTGCCGTCTTTT GGCAATGTGAGGGCCCGGAAACCTGGCCCTGTCTTCTTGACGAGCATTCCTAG GGGTCTTTCCCCTCTCGCCAAAGGAATGCAAGGTCTGTTGAATGTCGTGAAGG AAGCAGTTCCTCTGGAAGCTTCTTGAAGACAAACAACGTCTGTAGCGACCCTT TGCAGGCAGCGGAACCCCCCACCTGGCGACAGGTGCCTCTGCGGCCAAAAGC CACGTGTATAAGATACACCTGCAAAGGCGGCACAACCCCAGTGCCACGTTGT GAGTTGGATAGTTGTGGAAAGAGTCAAATGGCTCTCCTCAAGCGTATTCAACA AGGGGCTGAAGGATGCCCAGAAGGTACCCCATTGTATGGGATCTGATCTGGG GCCTCGGTGCACATGCTTTACATGTGTTTAGTCGAGGTTAAAAAAACGTCTAG GCCCCCCGAACCACGGGGACGTGGTTTTCCTTTGAAAAACACGATGATAAAGC CACCATGCACAGCTCAGCACTGCTCTGTTGCCTAGTCCTCCTGACTGGGGTGA GGGCCAGCCCAGGCCAGGGCACCCAGTCTGAGAACAGCTGCACCCGCTTCCC AGGCAACCTGCCTCACATGCTTCGAGACCTCCGAGATGCCTTCAGCAGAGTGA AGACTTTCTTTCAAATGAAGGATCAGCTGGACAACATATTGTTAAAGGAGTCC TTGCTGGAGGACTTTAAGGGTTACCTGGGTTGCCAAGCCTTGTCTGAGATGAT CCAGTTTTACCTGGAGGAGGTGATGCCCCAAGCTGAGAACCACGACCCAGAC ATCAAGGAGCATGTGAACTCCCTGGGGGAGAATCTGAAGACCCTCAGGCTGA GGCTGCGGCGCTGTCATCGATTTCTTCCCTGTGAAAACAAGAGCAAGGCCGTG GAGCAGGTGAAGAATGCCTTTAGTAAGCTCCAAGAGAAAGGCGTCTACAAAG CCATGAGTGAGTTTGACATCTTCATCAACTACATAGAAGCCTACATGACAATG AAGATACAAAACTGAGACCTGCAGGACGCCACAGCTCTGATCATAATCAGCC ATACCACATTTGTAGAGGTTTTACTTGCTTTAAAAAACCTCCCACACCTCCCCC TGAACCTGAAACATAAAATGAATGCAATTGTTGTTGTTAACTTGTTTATTGCA GCTTATAATGGTTACAAATAAAGCAATAGCATCACAAATTTCACAAATAAAGC ATTTTTTTCACTGCATTCTAGTTGTGGTTTGTCCAAACTCATCAATGTATCT | |
| 10 | | Human STAT3 protein sequence; Residues in bold (A and N) indicate |
| | | the amino acids replaced by C to produce STAT3C |
| 11 | MTACPGQAGAGRGLAEGAVIQRFPELRRRRPGGGAGGSDVAAEGTSPNRILDRH PGSALSLPLGSDKPSGLREPRRPPPAHAQPRPLGLCWRNSRMAQWNQLQQLDTR YLEQLHQLYSDSFPMELRQFLAPWIESQDWAYAASKESHATLVFHNLLGEIDQQY SRFLQESNVLYQHNLRRIKQFLQSRYLEKPMEIARIVARCLWEESRLLQTAATAA QQGGQANHPTAAVVTEKQQMLEQHLQDVRKRVQDLEQKMKVVENLQDDFDFN YKTLKSQGGQGVSPSSADMQDLNGNNQSVTRQKMQQLEQMLTALDQMRRSIVS ELAGLLSAMEYVQKTLTDEELADWKRRQQIACIGGPPNICLDRLENWITSLAESQ LQTRQQIKKLEELQQKVSYKGDPIVQHRPMLEERIVELFRNLMKSAFVVERQPCM PMHPDRPLVIKTGVQFTTKVRLLVKFPELNYQLKIKVCIDKDSGDVAALRGSRKF NILGTNTKVMNMEESNNGSLSAEFKHLTLREQRCGNGGRANCDASLIVTEELHLI TFETEVYHQGLKIDLETHSLPVVVISNICQMPNAWASILWYNMLTNNPKNVNFFT KPPIGTWDQVAEVLSWQFSSTTKRGLSIEQLTTLAEKLLGPGVNYSGCQITWAKF CKENMAGKGFSFWVWLDNIIDLVKKYILALWNEGYIMGFISKERERAILSTKPPG TFLLRFSESSKEGGVTFTWVEKDISGKTQIQSVEPYTKQQLNNMSFAEIIMGYKIM DATNILVSPLVYLYPDIPKEEAFGKYCRPESQEHPEADPGSAAPYLKTKFICVTPTT CSNTIDLPMSPRTLDSLMQFGNNGEGAEPSAGGQFESLTFDMELTSECATSPM | Rhesus STAT3 protein sequence; Residues in bold (A and N) indicate the amino acids replaced by C to produce STAT3C |
| 12 | MAQWNQLQQLDTRYLEQLHQLYSDSFPMELRQFLAPWIESQDWAYAASKESHA TLVFHNLLGEIDQQYSRFLQESNVLYQHNLRRIKQFLQSRYLEKPMEIARIVARCL WEESRLLQTAATAAQQGGQANHPTAAVVTEKQQMLEQHLQDVRKRVQDLEQK MKVVENLQDDFDFNYKTLKSQGDMQDLNGNNQSVTRQKMQQLEQMLTALDQ MRRSIVSELAGLLSAMEYVQKTLTDEELADWKRRQQIACIGGPPNICLDRLENWI TSLAESQLQTRQQIKKLEELQQKVSYKGDPIVQHRPMLEERIVELFRNLMKSAFV VERQPCMPMHPDRPLVIKTGVQFTTKVRLLVKFPELNYQLKIKVCIDKDSGDVAA LRGSRKFNILGTNTKVMNMEESNNGSLSAEFKHLTLREQRCGNGGRANCDASLIV TEELHLITFETEVYHQGLKIDLETHSLPVVVISNICQMPNAWASILWYNMLTNNPK NVNFFTKPPIGTWDQVAEVLSWQFSSTTKRGLSIEQLTTLAEKLLGPGVNYSGCQI TWAKFCKENMAGKGFSFWVWLDNIIDLVKKYILALWNEGYIMGFISKERERAILS TKPPGTFLLRFSESSKEGGVTFTWVEKDISGKTQIQSVEPYTKQQLNNMSFAEIIM GYKIMDCTCILVSPLVYLYPDIPKEEAFGKYCRPESQEHPEADPGSAAPYLKTKFI CVTPTTCSNTIDLPMSPRTLDSLMQFGNNGEGAEPSAGGQFESLTFDMELTSECAT SPM | Human STAT3C protein sequence; Residues in bold (C and C) indicate the amino acids that have been replaced in STAT3 to produce STAT3C |
| 13 | MTACPGQAGAGRGLAEGAVIQRFPELRRRRPGGGAGGSDVAAEGTSPNRILDRH PGSALSLPLGSDKPSGLREPRRPPPAHAQPRPLGLCWRNSRMAQWNQLQQLDTR YLEQLHQLYSDSFPMELRQFLAPWIESQDWAYAASKESHATLVFHNLLGEIDQQY SRFLQESNVLYQHNLRRIKQFLQSRYLEKPMEIARIVARCLWEESRLLQTAATAA QQGGQANHPTAAVVTEKQQMLEQHLQDVRKRVQDLEQKMKVVENLQDDFDFN YKTLKSQGGQGVSPSSADMQDLNGNNQSVTRQKMQQLEQMLTALDQMRRSIVS ELAGLLSAMEYVQKTLTDEELADWKRRQQIACIGGPPNICLDRLENWITSLAESQ LQTRQQIKKLEELQQKVSYKGDPIVQHRPMLEERIVELFRNLMKSAFVVERQPCM PMHPDRPLVIKTGVQFTTKVRLLVKFPELNYQLKIKVCIDKDSGDVAALRGSRKF NILGTNTKVMNMEESNNGSLSAEFKHLTLREQRCGNGGRANCDASLIVTEELHLI TFETEVYHQGLKIDLETHSLPVVVISNICQMPNAWASILWYNMLTNNPKNVNFFT KPPIGTWDQVAEVLSWQFSSTTKRGLSIEQLTTLAEKLLGPGVNYSGCQITWAKF CKENMAGKGFSFWVWLDNIIDLVKKYILALWNEGYIMGFISKERERAILSTKPPG TFLLRFSESSKEGGVTFTWVEKDISGKTQIQSVEPYTKQQLNNMSFAEIIMGYKIM DCTCILVSPLVYLYPDIPKEEAFGKYCRPESQEHPEADPGSAAPYLKTKFICVTPTT CSNTIDLPMSPRTLDSLMQFGNNGEGAEPSAGGQFESLTFDMELTSECATSPM | Rhesus STAT3C protein sequence; Residues in bold (C and C) indicate the amino acids that have been replaced in STAT3 to produce STAT3C |

### ASPECTS:

1. A system for expressing an antigen and a protein having interleukin-10 (IL-10)-like activity in a cell, the system comprising a recombinant polynucleotide comprising a nucleic acid sequence encoding the antigen and a recombinant polynucleotide comprising a nucleic acid sequence encoding the protein having IL-10-like activity.
2. The system of aspect 1, wherein the antigen and the protein having IL-10-like activity are expressed in the same cell.
3. The system of aspect 1, wherein the cell is selected from the group consisting of an antigen-presenting cell, a muscle cell, a tumor cell, a fibroblast, an epithelial cell, an endothelial cell, and a combination thereof.
4. The system of aspect 3, wherein the antigen-presenting cell is a dendritic cell.
5. The system of aspect 1, wherein the recombinant polynucleotide comprising the nucleic acid sequence encoding the antigen and/or the recombinant polynucleotide comprising the nucleic acid sequence encoding the protein having IL-10-like activity further comprise one or more nucleic acid sequences that encode a regulatory sequence.
6. The system of aspect 5, wherein the regulatory sequence controls expression of the antigen and/or the protein having IL-10-like activity.
7. The system of aspect 5, wherein the regulatory sequence is selected from the group consisting of a promoter, an intronic sequence, an internal ribosome entry sequence (IRES), a polyadenylation signal, a Kozak consensus sequence, and a combination thereof.
8. The system of aspect 7, wherein the promoter is an EF1α promoter.
9. The system of aspect 8, wherein the EF1α promoter contains an intronic sequence.
10. The system of aspect 7, wherein the polyadenylation signal is an SV40 virus polyadenylation signal.
11. The system of aspect 7, wherein the IRES is selected from the group consisting of an encephalomyocarditis (EMCV) IRES, a human VCIP IRES, a mouse Gtx IRES, a cricket paralysis virus intergenic region, a hepatitis C virus IRES, a poliovirus IRES, a human rhinovirus IRES, a dicistrovirus intergenic IRES, a hepatitis A virus IRES, an enterovirus IRES, an Aichivirus IRES, a cardiovirus IRES, an aphthorivus IRES, a porcine teschovirus IRES, a sapelovirus IRES, a simian virus IRES, an avian encephalomyelitis virus IRES, a foot and mouth disease virus IRES, a cold stress-induced mRNA (Rbm3) IRES, a human NF-kB repressing factor (NRF) IRES, a human apoptotic protease-activating factor-1 (Apaf-1) IRES, an immunoglobulin heavy chain binding protein (BIP) IRES, an aquaporin 4 (AQP-4) IRES, a c-myc33 IRES, and a rat cationic amino-acid transporter-1 (CAT-1) IRES.
12. The system of aspect 1, wherein the recombinant polynucleotide comprising the nucleic acid sequence encoding the antigen and the recombinant polynucleotide comprising the nucleic acid sequence encoding the protein having IL-10-like activity are separate recombinant polynucleotides.
13. The system of aspect 1, wherein the recombinant polynucleotide comprising the nucleic acid sequence encoding the antigen and the recombinant polynucleotide comprising the nucleic acid sequence encoding the protein having IL-10-like activity are the same recombinant polynucleotide.
14. The system of aspect 13, wherein the recombinant polynucleotide comprises:
   (a) the nucleic acid sequence encoding the antigen;
   (b) a nucleic acid sequence encoding an IRES; and
   (c) the nucleic acid sequence encoding the protein having IL-10-like activity.
15. The system of aspect 14, wherein the nucleic acid sequence encoding the antigen is located 5' of the nucleic acid sequence encoding the IRES and the nucleic acid sequence encoding the protein having IL-10-like activity is located 3' of the nucleic acid sequence encoding the IRES.
16. The system of aspect 15, wherein the recombinant polynucleotide further comprises:
   (d) a nucleic acid sequence encoding an EF1α promoter that is located 5' of the nucleic acid sequence encoding the antigen;
   (e) a nucleic acid sequence encoding an SV40 virus polyadenylation signal that is located 3' of the nucleic acid sequence encoding the protein having IL-10-like activity, and
   (f) a nucleic acid sequence encoding a Kozak consensus sequence that is located 3' of the nucleic acid sequence encoding the EF1α promoter and 5' of the nucleic acid sequence encoding the antigen.
17. The system of aspect 13, wherein the antigen and the protein having IL-10-like activity are expressed under the control of two different promoters.
18. The system of aspect 13, wherein the antigen and the protein having IL-10-like activity are expressed as a single protein.
19. The system of aspect 18, wherein the single protein is a self-cleaving protein, and wherein self-cleavage of the single protein yields the antigen and the protein having IL-10-like activity as separate proteins.
20. The system of aspect 1, wherein the nucleic acid sequence encoding the antigen is codon-optimized for expression in a host.
21. The system of aspect 1, wherein the antigen is an infectious disease antigen.
22. The system of aspect 21, wherein the infectious disease antigen is a bacterial, viral, fungal, protozoal, and/or helminthic infectious disease antigen.
23. The system of aspect 22, wherein the infectious disease antigen is a viral infectious disease antigen from simian immunodeficiency virus (SIV), human immunodeficiency virus (HIV), hepatitis C virus, herpes simplex virus 1, herpes simplex virus 2, varicella-zoster virus, human cytomegalovirus, human herpesvirus 6A, human herpesvirus 6B, human herpesvirus 7, human herpesvirus 8, Epstein-Barr virus, a hemorrhagic fever virus, or a combination thereof.
24. The system of aspect 23, wherein the infectious disease antigen comprises an HIV or SIV group-specific antigen (gag) protein.
25. The system of aspect 24, wherein the system comprises a recombinant polynucleotide comprising the nucleic acid sequence set forth in SEQ ID NO:1.
26. The system of aspect 22, wherein the infectious disease antigen is a bacterial infectious disease antigen from Mycobacterium tuberculosis.
27. The system of aspect 1, wherein the antigen is a tumor-associated antigen.
28. The system of aspect 27, wherein the tumor-associated antigen is selected from the group consisting of prostate-specific antigen, melanoma-associated antigen 4 (MAGEA4), melanoma-associated antigen 10 (MAGEA10), NY-ESO-1, and a combination thereof.
29. The system of aspect 28, wherein the system comprises a recombinant polynucleotide comprising the nucleic acid sequence set forth in SEQ ID NO:8 or 9.
30. The system of aspect 27, wherein the tumor-associated antigen is an antigen that newly arises in a subject's tumor.
31. The system of aspect 1, wherein the protein having IL-10-like activity is a heterologous protein having IL-10-like activity and/or a viral protein having IL-10-like activity.
32. The system of aspect 31, wherein the heterologous protein having IL-10-like activity is an IL-10 protein from a host.
33. The system of aspect 32, wherein the IL-10 protein from the host is a human IL-10 protein or a rhesus macaque IL-10 protein.
34. The system of aspect 31, wherein the viral protein having IL-10-like activity is a cytomegalovirus (CMV) protein having IL-10-like activity.
35. The system of aspect 34, wherein the CMV protein having IL-10-like activity is human CMV IL-10 (HCMVIL-10) or rhesus macaque CMV IL-10 (RhCMVIL-10).
36. The system of aspect 31, wherein the heterologous protein having IL-10-like activity is STAT3 or a constitutively active form of STAT3.
37. The system of aspect 36, wherein the constitutively active form of STAT3 is STAT3C.
38. The system of aspect 36, wherein the STAT3 or constitutively active form of STAT3 is a human STAT3, or a rhesus macaque STAT3, or a constitutively active form thereof.
39. The system of aspect 1, wherein the nucleic acid sequence encoding the antigen and/or the nucleic acid sequence encoding the protein having IL-10-like activity do not contain intronic sequences.
40. The system of aspect 1, wherein the nucleic acid sequence encoding the antigen and/or the nucleic acid sequence encoding the protein having IL-10-like activity contain intronic sequences.
41. The system of aspect 1, wherein the recombinant polynucleotide comprising the nucleic acid sequence encoding the antigen and/or the recombinant polynucleotide comprising the nucleic acid sequence encoding the protein having IL-10-like activity further comprise a nucleic acid sequence encoding a tag.
42. The system of aspect 41, wherein the tag is a FLAG tag.
43. The system of aspect 1, 5, or 41, wherein the nucleic acid sequences encoding the antigen, the protein having IL-10-like activity, the one or more regulatory sequences, and/or the tag are located within the same open reading frame.
44. The system of aspect 1, wherein the recombinant polynucleotide comprising the nucleic acid sequence encoding the antigen and/or the recombinant polynucleotide comprising the nucleic acid sequence encoding the protein having IL-10-like activity are present within a viral vector.
45. The system of aspect 44, wherein the viral vector is selected from the group consisting of a CMV vector, an adenoviral vector, an adeno-associated virus vector, a lentiviral vector, a herpes virus vector, an alphavirus vector, a retroviral vector, a poxvirus vector, and a vesicular stomatitis virus vector.
46. The system of aspect 45, wherein the viral vector is selected from the group consisting of an adenoviral vector, an adeno-associated virus vector, a lentiviral vector, an alphavirus vector, a retroviral vector, and a vesicular stomatitis virus vector.
47. The system of aspect 44, wherein the viral vector contains a mutation that increases tropism for the cell.
48. The system of aspect 47, wherein the viral vector is a CMV vector and the mutation that increases tropism comprises a mutation that modifies a protein, or portion thereof, that may be positioned on the outside of the CMV virion.
49. The system of aspect 47, wherein the mutation that increases tropism comprises an insertion of a nucleotide sequence encoding a cellular targeting ligand.
50. The system of aspect 49, wherein the cellular targeting ligand is selected from the group consisting of an antibody fragment that recognizes a target cell antigen, a ligand that is recognized by a target cell cognate receptor, a viral capsid protein that recognizes a target cell, and a combination thereof.
51. The system of aspect 49, wherein the cellular targeting ligand is CD154.
52. The system of aspect 47, wherein the viral vector is a CMV vector, wherein the CMV is a CMV capable of infecting rhesus macaque cells, and wherein the mutation that increases tropism comprises a mutation within a gene selected from the group consisting of Rh13.1, Rh61/Rh60, Rh157.4, Rh157.5, Rh157.6, and a combination thereof.
53. The system of aspect 47, wherein the viral vector is a CMV vector, wherein the CMV is a CMV capable of infecting human cells, and wherein the mutation that increases tropism comprises a mutation within a gene selected from the group consisting of RL13, UL36, UL130, UL128, UL131, and a combination thereof.
54. The system of aspect 1, wherein the protein having IL-10-like activity is one to which a host does not have pre-existing immunity.
55. The system of aspect 20, wherein the host is a human or a non-human primate.
56. The system of aspect 1, wherein the recombinant polynucleotide comprising the nucleic acid sequence encoding the antigen and/or the recombinant polynucleotide comprising the nucleic acid sequence encoding the protein having IL-10-like activity further comprise a nucleic acid sequence encoding a selectable marker.
57. The system of aspect 56, wherein the nucleic acid sequence encoding the selectable marker comprises a nucleic acid sequence encoding an antibiotic resistance gene and/or a fluorescent protein.
58. A viral particle comprising the system of aspect 1.
59. An engineered cell comprising the system of aspect 1 or the viral particle of aspect 58.
60. A pharmaceutical composition comprising:
   (a) the system of aspect 1, the viral particle of aspect 58, or the engineered cell of aspect 59; and
   (b) a pharmaceutically acceptable carrier.
61. A method for inducing an immune response against an antigen in a subject, the method comprising administering to the subject a therapeutically effective amount of the pharmaceutical composition of aspect 60.
62. The method of aspect 61, wherein the antigen is an infectious disease antigen or a tumor-associated antigen.
63. The method of aspect 62, wherein the infectious disease antigen is a bacterial, viral, fungal, protozoal, and/or helminthic infectious disease antigen.
64. The method of aspect 63, wherein the infectious disease antigen is a viral infectious disease antigen from simian immunodeficiency virus (SIV), human immunodeficiency virus (HIV), hepatitis C virus, herpes simplex virus 1, herpes simplex virus 2, varicella-zoster virus, human cytomegalovirus, human herpesvirus 6A, human herpesvirus 6B, human herpesvirus 7, human herpesvirus 8, Epstein-Barr virus, a hemorrhagic fever virus, or a combination thereof.
65. The method of aspect 64, wherein the infectious disease antigen comprises an HIV or SIV group-specific antigen (gag) protein.
66. The method of aspect 63, wherein the infectious disease antigen is a bacterial infectious disease antigen from Mycobacterium tuberculosis.
67. The method of aspect 62, wherein the tumor-associated antigen is selected from the group consisting of prostate-specific antigen, melanoma-associated antigen 4 (MAGEA4), melanoma-associated antigen 10 (MAGEA10), NY-ESO-1, and a combination thereof.
68. The method of aspect 62, wherein the tumor-associated antigen is an antigen that newly arises in the subject's tumor.
69. The method of aspect 61, wherein inducing the immune response comprises generating antibodies that recognize the antigen.
70. The method of aspect 61, wherein the immune response that is induced in the subject comprises a lower CD8⁺ T cell response as compared to the CD8⁺ T cell response that is induced using a pharmaceutical composition that does not express the antigen and the protein having IL-10-like activity in the cell.
71. The method of aspect 61, wherein the immune response that is induced in the subject comprises a higher CD4⁺ T cell response as compared to the CD4⁺ T cell response that is induced using a pharmaceutical composition that does not express the antigen and the protein having IL-10-like activity in the cell.
72. The method of aspect 61, wherein the expression or activity of interleukin-8 (IL-8) is increased in the subject.
73. The method of aspect 61, wherein inducing the immune response comprises increasing the number or activation of MHC class Ib-restricted T cells in the subject.
74. The method of aspect 61, wherein a sample is obtained from the subject.
75. The method of aspect 74, wherein the sample is selected from the group consisting of a blood sample, a tissue sample, a urine sample, a saliva sample, a cerebrospinal fluid (CSF) sample, and a combination thereof.
76. The method of aspect 74, wherein the level of one or more biomarkers is determined in the sample.
77. The method of aspect 76, wherein the one or more biomarkers is selected from the group consisting of IL-8, IL-10, IL-12, interferon-gamma, tumor necrosis factor-alpha, and a combination thereof.
78. The method of aspect 76, wherein the level of the one or more biomarkers is compared to a reference sample.
79. The method of aspect 78, wherein the reference sample is obtained from the subject.
80. The method of aspect 78, wherein the reference sample is obtained from a different subject or a population of subjects.
81. A method for preventing or treating a disease in a subject, the method comprising administering to the subject a therapeutically effective amount of the pharmaceutical composition of aspect 60.
82. The method of aspect 81, wherein the disease is an infectious disease or cancer.
83. The method of aspect 82, wherein the infectious disease is a bacterial, viral, fungal, protozoal, and/or helminthic infectious disease.
84. The method of aspect 83, wherein the viral infectious disease is caused by a simian immunodeficiency virus (SIV), a human immunodeficiency virus (HIV), a hepatitis C virus, a herpes simplex virus 1, a herpes simplex virus 2, a varicella-zoster virus, a human cytomegalovirus, a human herpesvirus 6A, a human herpesvirus 6B, a human herpesvirus 7, a human herpesvirus 8, an Epstein-Barr virus, or a hemorrhagic fever virus.
85. The method of aspect 82, wherein the cancer is melanoma, ovarian cancer, lymphoma, leukemia, or prostate cancer.
86. The method of aspect 81, wherein treating the subject comprises decreasing or eliminating one or more signs or symptoms of the disease.
87. The method of aspect 61 or 81, further comprising administering an exogenous protein having IL-10-like activity to the subject.

## Claims

1. A system for expressing an antigen and an IL-10 protein in a cell, the system comprising a i) recombinant polynucleotide comprising a nucleic acid sequence encoding the antigen, wherein the antigen is a tumor-associated antigen or a viral antigen and ii) a recombinant polynucleotide comprising a nucleic acid sequence encoding the IL-10 protein, wherein the IL-10 protein comprises a human IL-10 protein or a rhesus macaque IL-10 protein,
wherein the recombinant polynucleotide comprising the nucleic acid sequence encoding the antigen and the recombinant polynucleotide comprising the nucleic acid sequence encoding the IL10-protein are present within a vector.

2. The system of claim 1, wherein the recombinant polynucleotide comprising the nucleic acid sequence encoding the antigen or the recombinant polynucleotide comprising the nucleic acid sequence encoding the IL-10 protein further comprises one or more nucleic acid sequences that encode a regulatory sequence.

3. The system of claim 2, wherein the regulatory sequence is selected from the group consisting of a promoter, an intronic sequence, an internal ribosome entry site (IRES), a polyadenylation signal, a Kozak consensus sequence, and a combination thereof.

4. The system of claim 3, wherein the promoter is an EF1α promoter, wherein the EF1α promoter comprises an intronic sequence.

5. The system of claim 3, wherein the IRES is selected from the group consisting of an encephalomyocarditis (EMCV) IRES, a human VCIP IRES, a mouse Gtx IRES, a cricket paralysis virus intergenic region, a hepatitis C virus IRES, a poliovirus IRES, a human rhinovirus IRES, a dicistrovirus intergenic IRES, a hepatitis A virus IRES, an enterovirus IRES, an Aichivirus IRES, a cardiovirus IRES, an aphthorivus IRES, a porcine teschovirus IRES, a sapelovirus IRES, a simian virus IRES, an avian encephalomyelitis virus IRES, a foot and mouth disease virus IRES, a cold stress-induced mRNA (Rbm3) IRES, a human NF-kB repressing factor (NRF) IRES, a human apoptotic protease-activating factor-1 (Apaf-1) IRES, an immunoglobulin heavy chain binding protein (BIP) IRES, an aquaporin 4 (AQP-4) IRES, a c-myc33 IRES, and a rat cationic amino-acid transporter-1 (CAT-1) IRES.

6. The system of claim 3, wherein the polyadenylation signal is an SV40 virus polyadenylation signal.

7. The system of any one of claims 1-6, wherein the viral antigen comprises a viral infectious disease antigen from simian immunodeficiency virus (SIV) or human immunodeficiency virus (HIV).

8. The system of any one of claims 1-7, wherein the human IL-10 protein comprises a sequence set forth in SEQ ID NO: 7, and the rhesus macaque IL-10 protein comprises a sequence set forth in SEQ ID NO: 4.

9. The system of any one of claims 1-8, wherein the system comprises a recombinant polynucleotide comprising the nucleic acid sequence set forth in SEQ ID NO:1.

10. The system of any one of claims 1-9, wherein the tumor-associated antigen comprises a melanoma-associated antigen (MAGE), optionally wherein the MAGE comprises melanoma-associated antigen 4 (MAGEA4) or a melanoma-associated antigen 10 (MAGEA10), further optionally wherein the system comprises a recombinant polynucleotide comprising the nucleic acid sequence set forth in SEQ ID NO: 8 or SEQ ID NO: 9.

11. The system of any one of claims 1-10, wherein the recombinant polynucleotide comprising the nucleic acid sequence encoding the antigen and the recombinant polynucleotide comprising the nucleic acid sequence encoding the IL-10 protein are the same recombinant polynucleotide.

12. A viral particle comprising the system of any one of claims 1-11.

13. An engineered cell comprising the system of any one of claims 1-11 or the viral particle of claim 12.

14. A pharmaceutical composition for use in inducing an immune response against an antigen in a subject, wherein the pharmaceutical composition comprises: (i) the system of any one of claims 1-11, the viral particle of claim 12, or the engineered cell of claim 13; and (ii) a pharmaceutically acceptable carrier.

15. The pharmaceutical composition for use of claim 14, wherein the immune response comprises increasing the number or activation of MHC class Ib-restricted T cells in the subject or increasing expression of interferon in the subject.

16. The pharmaceutical composition for use of claim 14 or 15, wherein the immune response comprises a lower CD8+ T cell response or a higher CD4+ T cell response as compared to a CD8+ T cell response or a CD4+ T cell response that is induced using a pharmaceutical composition that does not comprise the system, the viral particle or the engineered cell.
